# FASCICULE DE BREVET EUROPEEN

(11) **EP 2 393 809 B1**
(45) Date de publication et mention de la délivrance du brevet: **30.04.2014**
(21) Numéro de dépôt: 10708283.6
(22) Date de dépôt: 04.02.2010
(51) Int. Cl.: C07D 413/14, C07D 417/14, A61K 31/438, A61P 37/00, A61P 29/00, A61P 1/00, A61P 25/00, A61P 9/00, A61P 35/00, A61P 31/00

(54) **DERIVES D'AZASPIRANYL-ALKYLCARBAMATES D'HETEROCYCLES A 5 CHAINONS, LEUR PREPARATION ET LEUR APPLICATION EN THERAPEUTIQUE**
DERIVATE VON AZASPIRANYLALKYLCARBAMATEN 5-GLIEDRIGER HETEROCYCLISCHER VERBINDUNGEN, DEREN HERSTELLUNG UND DEREN THERAPEUTISCHE VERWENDUNG
DERIVATIVES OF AZASPIRANYL-ALKYLCARBAMATES OF 5-MEMBER HETEROCYCLIC COMPOUNDS, PREPARATION THEREOF AND THERAPEUTIC USE THEREOF

(30) Priorité: 05.02.2009 FR 0900493
(43) Date de publication de la demande: 14.12.2011
(73) Titulaire: SANOFI, 75008 Paris (FR)
(72) Inventeur: ABOUABDELLAH, Ahmed, F-75013 Paris (FR); CHEREZE, Nathalie, F-75013 Paris (FR); FAYOL, Aude, F-75013 Paris (FR); LOCHEAD, Alistair, F-75013 Paris (FR); SAADY, Mourad, F-75013 Paris (FR); VACHE, Julien, F-75013 Paris (FR); YAICHE, Philippe, F-75013 Paris (FR)
(74) Mandataire: Le Coupanec, Pascale A.M.P.
(86) Numéro de dépôt international: PCT/FR2010/050183
(87) Numéro de publication internationale: WO 2010/089510

(56) Documents cités:
- WO-A-2004/033422
- WO-A-2004/099176

## Description

L'invention a pour objet des dérivés d'azaspiranyl-alkylcarbamates d'hétérocycles à 5 chaînons, leur préparation et leur application en thérapeutique.

Il existe toujours une nécessité de trouver et de développer des produits inhibiteurs de l'enzyme FAAH (Fatty Acid Amide Hydrolase). Les composés de l'invention répondent à ce but.

Ces composés doivent présenter des propriétés métaboliques, pharmacocinétiques et un index de sécurité permettant leur utilisation commme médicaments.

Des dérivés de carbamate comme inhibituers de l'enzyme FAAH sont connus de WO2004/033422 et WO2004/099176.

Les composés de l'invention répondent à la formule générale (I) : 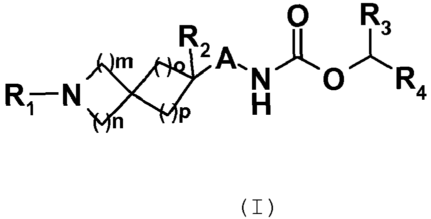 dans laquelle
R₂ représente un atome d'hydrogène, de fluor ou un groupe hydroxyle, cyano, trifluorométhyle, C₁₋₆-alkyle, C₁₋₆-alcoxy, NR₈R₉ ;
m, n, o et p représentent, indépendamment l'un de l'autre, un nombre entier égal à 0, 1, 2 ou 3 ;
A représente une liaison covalente ou un groupe C₁₋₈-alkylène ;
R₁ représente un groupe R₅ éventuellement substitué par un ou plusieurs groupes R₆ et/ou R₇ ;
R₅ représente un groupe choisi parmi un phényle, pyridinyle, pyridazinyle, pyrimidinyle, pyrazinyle, triazinyle, naphtalènyle, quinolinyle, isoquinolinyle, phtalazinyle, quinazolinyle, quinoxalinyle, cinnolinyle, naphthyridinyle ;
R₆ représente un atome d'halogène, un groupe cyano, -CH₂CN, nitro, hydroxyle, C₁₋₆-alkyle, C₁₋₆-alcoxy, C₁₋₆-thioalkyle, C₁₋₆-haloalkyle, C₁₋₆-haloalcoxy, C₁₋₆-halothioalkyle, C₃₋₇-cycloalkyle, C₃₋₇-cycloalkyle-C₁₋₆-alkylène, C₃₋₇-cycloalkyle-C₁₋₃-alkylène-O-, NR₈R₉, NR₈COR₉, NR₈CO₂R₉, NR₈SO₂R₉, NR₈SO₂NR₈R₉, COR₈, CO₂R₈, CONR₈R₉, SO₂R₈, SO₂NR₈R₉ ou -O-(C₁₋₃-alkylène)-O- ;
R₇ représente un groupe choisi parmi un furanyle, pyrrolyle, thiènyle, oxazolyle, isoxazolyle, thiazolyle, isothiazolyle, imidazolyle, pyrazolyle, oxadiazole, thiadiazole, phényle, pyridinyle, pyridazinyle, pyrimidinyle, pyrazinyle, triazine, naphtalènyle, quinolinyle, isoquinolinyle, phtalazinyle, quinazolinyle, quinoxalinyle, cinnolinyle, naphthyridinyle, imidazopyrimidinyle, thiènopyrimidinyle, benzofuranyle, benzothiènyle, benzimidazolyle, benzoxazolyle, benzisoxazolyle, benzothiazolyle, benzisothiazolyle, indolyle, isoindolyle, indazolyle, pyrrolopyridinyle, furopyridinyle, thiènopyridinyle, imidazopyridinyle, pyrazolopyridinyle, oxazolopyridinyle, isoxazolopyridinyle, thiazolopyridinyle, phényloxy, benzyloxy, pyrimidinoxy ; le ou les groupes R₇ pouvant être substitués par un ou plusieurs groupes R₆ identiques ou différents l'un de l'autre ;
R₃ représente un atome d'hydrogène, de fluor, un groupe C₁₋₆-alkyle ou un groupe trifluorométhyle ;
R₄ représente un groupe choisi parmi un furanyle, pyrrolyle, thiènyle, thiazolyle, isothiazolyle, oxazolyle, isoxazolyle, pyrazolyle, oxadiazolyle, thiadiazolyle, imidazolyle, triazolyle, tétrazolyle;
   ce groupe étant éventuellement substitué par un ou plusieurs substituants choisis parmi un atome d'halogène, un groupe C₁₋₆-alkyle, C₁₋₆-haloalkyle, C₃₋₇-cycloalkyle, C₃₋₇-cycloalkyle-C₁₋₃-alkylène, C₁₋₆-haloalcoxy, cyano, NR₈R₉, NR₈COR₉, NR₈CO₂R₉, NR₈SO₂R₉, NR₈SO₂NR₈R₉, COR₈, CO₂R₉, CONR₈R₉, CON(R₈) (C₁₋₃-alkylène-NR₁₀R₁₁), SO₂R₈, SO₂NR₈R₉, -O-(C₁₋₃-alkylène)-O-, phényle, phényloxy, benzyloxy, pyridinyle, pyrazinyle, pyridazinyle, triazinyle ou pyrimidinyle; les groupes phényle, phényloxy, benzyloxy, pyridinyle, pyrazinyle, pyridazinyle, triazinyle et pyrimidinyle pouvant être substitués par un ou plusieurs substituants choisis parmi un atome d'halogène, un groupe cyano, nitro, C₁₋₆-alkyle, C₁₋₆-alcoxy, C₁₋₆-thioalkyle, C₁₋₆-haloalkyle, C₁₋₆-haloalcoxy, C₁₋₆-halothioalkyle, C₃₋₇-cycloalkyle, C₃₋₇-cycloalkyle-C₁₋₃-alkylène ;
R₈ et R₉ représentent indépendamment l'un de l'autre un atome d'hydrogène ou un groupe C₁₋₆-alkyle,
   ou forment avec le ou les atomes qui les portent,
   dans le cas de NR₈R₉, un cycle choisi parmi les cycles azétidine, pyrrolidine, pipéridine, morpholine, thiomorpholine, azépine, oxazépine ou pipérazine, ce cycle étant éventuellement substitué par un groupe C₁₋₆-alkyle ou benzyle ;
   dans le cas de NR₈COR₉, un cycle lactame ; dans le cas de NR₈CO₂R₉, un cycle oxazolidinone, oxazinone ou oxazépinone; dans le cas de NR₈SO₂R₉, un cycle sultame ; dans le cas de NR₈SO₂NR₈R₉, un cycle dioxyde de thiazolidine ou dioxyde de thiadiazinane ;
   R₁₀ et R₁₁ représentent indépendamment l'un de l'autre un atome d'hydrogène ou un groupe C₁₋₆-alkyle.

Parmi les composés de formule générale (I), un premier sous-groupe de composés est constitué des composés pour lesquels R₂ représente un atome d'hydrogène.

Parmi les composés de formule générale (I), un second sous-groupe de composés est constitué des composés pour lesquels le groupe 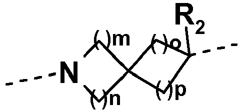 représente 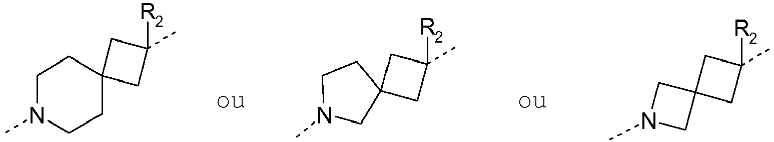 R₂ étant tel que défini dans la formule générale (I).

Parmi les composés de formule générale (I), un troisième sous-groupe de composés est constitué des composés pour lesquels le groupe 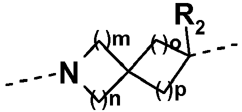 représente

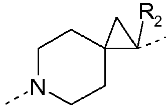

R₂ étant tel que défini dans la formule générale (I).

Parmi les composés de formule générale (I), un quatrième sous-groupe de composés est constitué des composés pour lesquels A représente une liaison covalente ou un groupe C₁₋₈₋alkylène, plus particulièrement un groupe méthylène.

Parmi les composés de formule générale (I), un cinquième sous-groupe de composés est constitué des composés pour lesquels R₁ représente un groupe R₅ éventuellement substitué par un ou plusieurs groupes R₆, et/ou R₇ ;
R₅ représente un groupe pyrimidinyle, pyrazinyle, pyridinyle ou quinolinyle ;
R₆ représente un atome d'halogène, plus particulièrement un atome de brome, de fluor ou de chlore, un groupe C₁₋₆-haloalkyle, plus particulièrement trifluorométhyle ou un groupe C₁₋₆-alkyle plus particulièrement un isobutyle;
R₇ représente un phényle pouvant être substitué par un ou plusieurs groupes R₆ identiques ou différents l'un de l'autre.

Parmi les composés de formule générale (I), un sixième sous-groupe de composés est constitué des composés pour lesquels R₁ représente un groupe R₅ éventuellement substitué par un ou plusieurs groupes R₆ et/ou R₇ ;
R₅ représente un groupe pyridinyle ou quinolinyle ;
R₆ représente un atome d'halogène, plus particulièrement un atome de brome, de fluor ou de chlore, ou un groupe C₁₋₆-haloalkyle, plus particulièrement trifluorométhyle;
R₇ représente un phényle pouvant être substitué par un ou plusieurs groupes R₆ identiques ou différents l'un de l'autre.

Parmi les composés de formule générale (I), un septième sous-groupe de composés est constitué des composés pour lesquels R₃ représente un atome d'hydrogène.

Parmi les composés de formule générale (I), un huitième sous-groupe de composés est constitué des composés pour lesquels R₄ représente un groupe choisi parmi un thiazolyle, un oxazolyle, un oxadiazolyle, un isoxazolyle;
ce groupe étant éventuellement substitué par un ou plusieurs substituants choisis parmi un groupe C₁₋₆-alkyle, CONR₈R₉, CON(R₈)(C₁₋₃-alkylène-NR₁₀R₁₁) ou un phényle ; le groupe phényle étant éventuellement substitué par un ou plusieurs substituants choisis parmi un atome d'halogène ;
R₈, R₉, R₁₀ et R₁₁ représentent indépendamment l'un de l'autre un atome d'hydrogène ou un groupe C₁₋₆-alkyle, plus particulièrement méthyle.

Parmi les composés de formule générale (I), un neuvième sous-groupe de composés est constitué des composés pour lesquels R₄ représente un groupe choisi parmi un thiazolyle, un oxazolyle, un isoxazolyle;
ce groupe étant éventuellement substitué par un ou plusieurs groupes CONR₈R₉ ;
R₈ et R₉ représentent indépendamment l'un de l'autre un atome d'hydrogène ou un groupe C₁₋₆-alkyle, plus particulièrement méthyle.

Parmi les composés de formule générale (I), un dixième sous-groupe de composés est constitué par les composés de formule générale (I) dans laquelle à la fois R₁ et/ou R₂ et/ou R₃ et/ou R₄ et/ou n et/ou m et/ou o et/ou p et/ou A sont tels que définis dans les groupes ci-dessus.

Parmi les composés de formule générale (I), les composés suivants peuvent être cités (nomenclature IUPAC générée par le logiciel AutoNom) :
1. [7-(6-Fluoro-quinolin-2-yl)-7-aza-spiro[3.5]non-2-yl]-carbamate de thiazol-4-ylméthyle
2. [7-(6-Fluoro-quinolin-2-yl)-7-aza-spiro[3.5]non-2-yl]-carbamate de 3-carbamoyl-isoxazol-5-ylméthyle
3. [7-(5-Trifluorométhyl-pyridin-2-yl)-7-aza-spiro[3.5]non-2-yl]-carbamate de 3-carbamoyl-isoxazol-5-ylméthyle
4. [7-(5-Bromo-pyridin-2-yl)-7-aza-spiro[3.5]non-2-yl]-carbamate de 3-carbamoyl-isoxazol-5-ylméthyle
5. {7-[5-(4-Fluoro-phényl)-pyridin-2-yl]-7-aza-spiro[3.5]non-2-yl}-carbamate de 3-carbamoyl-isoxazol-5-ylméthyle
6. [7-(6-Fluoro-quinolin-2-yl)-7-aza-spiro[3.5]non-2-yl]-méthylcarbamate de 3-carbamoyl-isoxazol-5-ylméthyle
7. [7-(6-Fluoro-quinolin-2-yl)-7-aza-spiro[3.5]non-2-yl]-méthylcarbamate de 3-(méthylcarbamoyl)-isoxazol-5-ylméthyle
8. {7-[5-(4-Fluoro-phényl)-pyridin-2-yl]-7-aza-spiro[3.5]non-2-yl}-carbamate de 3-(méthylcarbamoyl)-isoxazol-5-ylméthyle
9. [7-(6-Fluoro-quinolin-2-yl)-7-aza-spiro[3.5]non-2-yl]-carbamate de 3-(méthylcarbamoyl)-isoxazol-5-ylméthyle
10. {6-[5-(4-Fluoro-phényl)-pyridin-2-yl]-6-aza-spiro[3.4]oct-2-yl}-carbamate de 3-carbamoyl-isoxazol-5-ylméthyle
11. {6-[5-(4-Fluoro-phényl)-pyridin-2-yl]-6-aza-spiro[3.4]oct-2-yl}-carbamate de 3-(méthylcarbamoyl)-isoxazol-5-ylméthyle
12. {6-[5-(4-Fluoro-phényl)-pyridin-2-yl]-6-aza-spiro[3.4]oct-2-ylméthyl}carbamate de 3-(méthylcarbamoyl)-isoxazol-5-ylméthyle (isomère I)
13. {6-[5-(4-Fluoro-phényl)-pyridin-2-yl]-6-aza-spiro[3.4]oct-2-ylméthyl}carbamate de 3-(méthylcarbamoyl)-isoxazol-5-ylméthyle (isomère II)
14. [7-(6-Fluoro-quinolin-2-yl)-7-aza-spiro[3.5]non-2-yl]-carbamate de 4-carbamoyl-oxazol-2-ylméthyle
15. [2-(6-Fluoro-quinolin-2-yl)-2-aza-spiro[3.3]hept-6-yl]-carbamate de 3-(méthylcarbamoyl)-isoxazol-5-ylméthyle
16. [6-(6-Fluoro-quinolin-2-yl)-6-aza-spiro[3.4]oct-2-ylméthyl]-carbamate de 3-(méthylcarbamoyl)-isoxazol-5-ylméthyle (un isomère)
17. {6-[5-(4-Fluoro-phényl)-pyridin-2-yl]-6-aza-spiro[3.4]oct-2-yl}-carbamate de 3-(méthylcarbamoyl)-isoxazol-5-ylméthyle (isomère I)
18. {6-[5-(4-Fluoro-phényl)-pyridin-2-yl]-6-aza-spiro[3.4]oct-2-yl}-carbamate de 3-(méthylcarbamoyl)-isoxazol-5-ylméthyle (isomère II)
19. [7-(6-Chloro-quinolin-2-yl)-7-aza-spiro[3.5]non-2-yl]-carbamate de 3-(méthylcarbamoyl)-isoxazol-5-ylméthyle
20. {2-[5-(4-Fluoro-phényl)-pyridin-2-yl]-2-aza-spiro[3.3]hept-6-yl}-carbamate de 3-carbamoyl-isoxazol-5-ylméthyle
21. [6-(5-Bromo-pyridin-2-yl)-6-aza-spiro[3.4]oct-2-yl]-carbamate de 3-méthylcarbamoyl-isoxazol-5-ylméthyle (un isomere)
22. [6-(4-Trifluorométhyl-pyridin-2-yl)-6-aza-spiro[3.4]oct-2-yl]-carbamate de 3-méthylcarbamoyl-isoxazol-5-ylméthyle (un isomere)
23. {2-[5-(4-Fluoro-phényl)-pyridin-2-yl]-2-aza-spiro[3.3]hept-6-yl}-carbamate de 3-méthylcarbamoyl-isoxazol-5-ylméthyle
24. [7-(4-Trifluorométhyl-pyrimidin-2-yl)-7-aza-spiro[3.5]non-2-yl]-carbamate de 3-carbamoyl-isoxazol-5-ylméthyle
25. {7-[6-(4-Fluoro-phényl)-pyrazin-2-yl]-7-aza-spiro[3.5]non-2-yl}-carbamate de 3-carbamoyl-isoxazol-5-ylméthyle
26. [7-(4-trifluorométhyl-pyrimidin-2-yl)-7-aza-spiro[3.5]non-2-yl]-carbamate de 3-(2-diméthylamino-éthylcarbamoyl)-isoxazol-5-ylméthyle et son chlorhydrate ;
27. {7-[4-(4-Fluoro-phényl)-pyridin-2-yl]-7-aza-spiro[3.5]non-2-yl}-carbamate de 3-carbamoyl-isoxazol-5-ylméthyle
28. [7-(4-Chloro-pyridin-2-yl)-7-aza-spiro[3.5]non-2-yl]-carbamate de 3-carbamoyl-isoxazol-5-ylméthyle
29. [7-(4-Trifluorométhyl-pyridin-2-yl)-7-aza-spiro[3.5]non-2-yl]-carbamate de 3-carbamoyl-isoxazol-5-ylméthyle
30. {7-[5-(3-Fluoro-phényl)-pyridin-2-yl]-7-aza-spiro[3.5]non-2-yl}-carbamate de 3-carbamoyl-isoxazol-5-ylméthyle
31. [7-(5-Isobutyl-pyridin-2-yl)-7-aza-spiro[3.5]non-2-yl]-carbamate de 3-carbamoyl-isoxazol-5-ylméthyle
32. [7-(6-Chloro-quinolin-2-yl)-7-aza-spiro[3.5]non-2-yl]-carbamate de 3-carbamoyl-isoxazol-5-ylméthyle
33. [7-(6-Chloro-quinolin-2-yl)-7-aza-spiro[3.5]non-2-ylméthyl]-carbamate de 3-méthylcarbamoyl-isoxazol-5-ylméthyle
34. [7-(4-Trifluorométhyl-pyridin-2-yl)-7-aza-spiro[3.5]non-2-yl]-carbamate de 3-méthylcarbamoyl-isoxazol-5-ylméthyle
35. [7-(4-Trifluorométhyl-pyridin-2-yl)-7-aza-spiro[3.5]non-2-yl]-carbamate de 3-(4-fluoro-phényl)-[1,2,4]oxadiazol-5-ylméthyle
36. [7-(4-Trifluorométhyl-pyridin-2-yl)-7-aza-spiro[3.5]non-2-yl]-carbamate de 4-carbamoyl-oxazol-2-ylméthyle
37. [7-(4-Trifluorométhyl-pyridin-2-yl)-7-aza-spiro[3.5]non-2-yl]-carbamate de 5-méthyl-3-phényl-isoxazol-4-ylméthyle
38. [7-(4-Trifluorométhyl-pyridin-2-yl)-7-aza-spiro[3.5]non-2-yl]-carbamate de 3-éthyl-[1,2,4]oxadiazol-5-ylméthyle
39. [7-(4-Trifluoroméhyl-pyridin-2-yl)-7-aza-spiro[3.5]non-2-yl]-carbamate de 5-méthyl-[1,2,4]oxadiazol-3-ylméthyle
40. [6-(4-Trifluorométhyl-pyrimidin-2-yl)-6-aza-spiro[2.5]oct-1-yl]-carbamate de 3-carbamoyl-isoxazol-5-ylméthyle
41. [6-(4-Trifluorométhyl-pyrimidin-2-yl)-6-aza-spiro[2.5]oct-1-yl]-carbamate de 3-méthylcarbamoyl-isoxazol-5-ylméthyle.

Les composés de formule générale (I) peuvent comporter un ou plusieurs carbones asymétriques. Ils peuvent exister sous forme d'énantiomères ou de diastéréoisomères. Les composés de formule générale (I) peuvent également exister sous forme de stéréoisomères *cis* ou *trans.* Ces stéréoisomères, énantiomères et diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention.

Dans le cadre de l'invention, on entend par :
- C_{t-z} où t et z peuvent prendre les valeurs de 1 à 8, une chaîne carbonée pouvant avoir de t à z atomes de carbone, par exemple C₁₋₃ une chaîne carbonée qui peut avoir de 1 à 3 atomes de carbone ;
- alkyle, un groupe aliphatique saturé, linéaire ou ramifié; par exemple un groupe C₁₋₆-alkyle représente une chaîne carbonée de 1 à 6 atomes de carbone, linéaire ou ramifiée, plus particulièrement un méthyle, éthyle, propyle, isopropyle, butyle, isobutyle, tert-butyle, pentyle, hexyle ;
- alkylène, un groupe alkyle divalent saturé, linéaire ou ramifié, par exemple un groupe C₁₋₃-alkylène représente une chaîne carbonée divalente de 1 à 3 atomes de carbone, linéaire ou ramifiée, plus particulièrement un méthylène, éthylène, 1-méthyléthylène, propylène ;
- cycloalkyle, un groupe alkyle cyclique, par exemple un groupe C₃₋₇-cycloalkyle représente un groupe carboné cyclique de 3 à 7 atomes de carbone, plus particulièrement un cyclopropyle, cyclobutyle, cyclopentyle, cyclohexyle, cycloheptyle ;
- alcoxy, un groupe -O-alkyle à chaîne aliphatique saturée, linéaire ou ramifiée ;
- thioalkyle, un groupe -S-alkyle à chaîne aliphatique saturée, linéaire ou ramifiée ;
- haloalkyle, un groupe alkyle dont un ou plusieurs atomes d'hydrogène ont été substitués par un atome d'halogène ;
- haloalcoxy, un groupe alcoxy dont un ou plusieurs atomes d'hydrogène ont été substitués par un atome d'halogène ;
- halothioalkyle, un groupe thioalkyle dont un ou plusieurs atomes d'hydrogène ont été substitués par un atome d'halogène ;
- atome d'halogène, un fluor, un chlore, un brome ou un iode.

Les composés de l'invention peuvent être préparés selon différentes méthodes, illustrées par les schémas qui suivent.

Ainsi une première méthode (schéma 1) consiste à faire réagir une amine de formule générale (II), dans laquelle A, R₁, R₂, m, n, o et p sont tels que définis dans la formule générale (I) définie ci-dessus, avec un carbonate de formule générale (III) dans laquelle Z représente un atome d'hydrogène ou un groupe nitro, R₃ et R₄ sont tels que définis dans la formule générale (I) définie ci-dessus, en présence d'une base telle que la triéthylamine, la pyridine, la *N,N*-diméthylaminopyridine ou la diisopropyléthylamine, dans un solvant tel que le toluène ou le dichloroéthane, à une température comprise entre la température ambiante et la température de reflux du solvant.

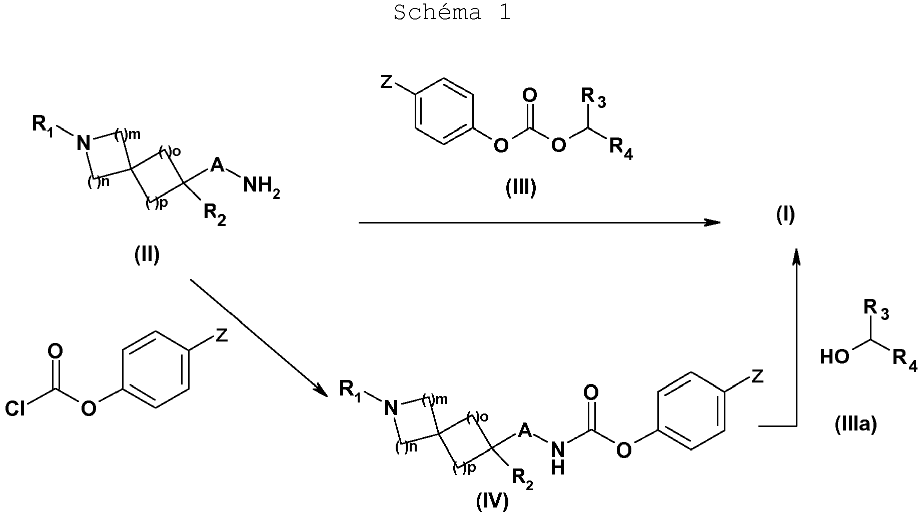

Une variante d'obtention des composés de formule générale (I) (schéma 1) consiste à faire réagir une amine de formule générale (II), telle que définie ci-dessus, avec le chloroformiate de phényle ou de 4-nitro-phényle, en présence d'une base telle que la triéthylamine ou la diisopropyléthylamine, dans un solvant tel que le dichlorométhane ou le tétrahydrofurane, à une température comprise entre 0°C et la température ambiante, pour conduire au dérivé carbamate de formule générale (IV), dans laquelle A, R₁, R₂, m, n, o et p sont tels que définis dans la formule générale (I) définie ci-dessus, et Z représente un atome d'hydrogène ou un groupe nitro. Le dérivé carbamate de formule générale (IV) ainsi obtenu est ensuite transformé en composé de formule générale (I), par action d'un alcool de formule générale HOCHR₃R₄ (IIIa), dans laquelle R₃ et R₄ sont tels que définis dans la formule générale (I) définie ci-dessus, en présence d'une base telle que la triéthylamine, la pyridine, la *N,N*-diméthylaminopyridine ou la diisopropyléthylamine, dans un solvant tel que le toluène ou le dichloroéthane, à une température comprise entre la température ambiante et la température de reflux du solvant.

Une seconde méthode (schéma 2 - voie A) consiste à faire réagir dans un premier temps une amine de formule générale (IIa), dans laquelle A, R₂, m, n, o et p sont tels que définis dans la formule générale (I) définie ci-dessus, et PG représente un groupe protecteur tel qu'un Boc (*tert-*butyloxycarbonyl), un Cbz (benzyloxycarbonyl), un benzyle ou un benzhydrile, avec un carbonate de formule générale (III) telle que définie ci-dessus, dans les conditions décrites ci-dessus lors de la réaction de l'amine de formule générale (II) avec le carbonate de formule générale (III), pour obtenir un composé de formule générale (Ic) correspondant au composé de formule (Ia) où l'atome d'azote spiranique est protégée par un groupe protecteur PG, puis suivie d'une réaction de déprotection, par exemple en présence d'une solution d'acide chlorhydrique (5N) dans l'isopropanol ou le dioxane, pour obtenir l'intermédiaire de formule générale (Ia), dans laquelle A, R₂, R₃, R₄, m, n, o et p sont tels que définis dans la formule générale (I).

Une variante d'obtention (schéma 2 - variante voie A) des intermédiaires de formule générale (Ia) consiste à faire réagir une amine de formule générale (IIa), telle que définie ci-dessus, avec le chloroformiate de phényle ou de 4-nitro-phényle, en présence d'une base telle que la triéthylamine ou la diisopropyléthylamine, dans un solvant tel que le dichlorométhane ou le tétrahydrofurane, à une température comprise entre 0°C et la température ambiante, pour conduire au dérivé carbamate de formule générale (IVa), dans laquelle A, R₂, m, n, o et p sont tels que définis dans la formule générale (I) définie ci-dessus, PG est tel que défini ci-dessus et Z représente un atome d'hydrogène ou un groupe nitro. Le dérivé carbamate de formule générale (IVa) ainsi obtenu est ensuite transformé en composé de formule générale (Ia), par action d'un alcool de formule générale HOCHR₃R₄ (IIIa), telle que définie ci-dessus, en présence d'une base telle que la triéthylamine, la pyridine, la *N,N-*diméthylaminopyridine ou la diisopropyléthylamine, dans un solvant tel que le toluène ou le dichloroéthane, à une température comprise entre la température ambiante et la température de reflux du solvant, suivie par une réaction de déprotection, par exemple en présence d'une solution d'acide chlorhydrique (5N) dans l'isopropanol ou le dioxane.

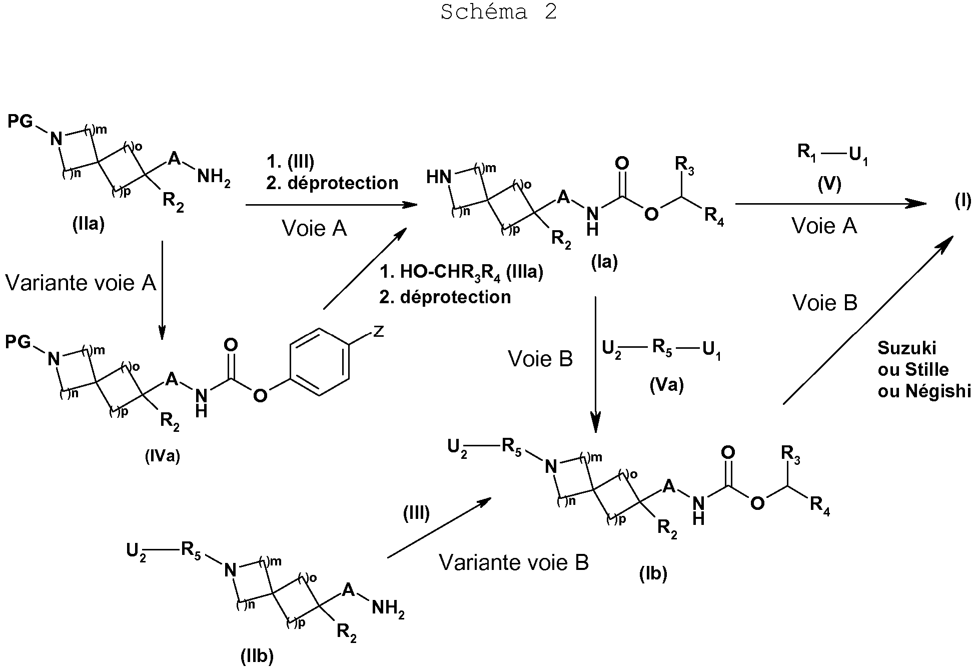

Ensuite, selon le schéma 2 voie A, le composé de formule générale (I) est ensuite obtenu par réaction du composé de formule générale (Ia) avec un dérivé de formule générale R₁-U₁ (V), dans laquelle R₁ est tel que défini dans la formule générale (I) et U₁ représente un atome d'halogène ou un groupe O-triflate, en utilisant les conditions de réactions de substitution nucléophile aromatique ou hétéroaromatique, par exemple au moyen d'une base telle que la triéthylamine, la diisopropyléthylamine, la pyridine ou la *N,N-*diméthylaminopyridine dans un solvant tel que le dichlorométhane, le dichloroéthane, l'acétonitrile, la *N,N-*diméthylformamide, le dioxane ou le tétrahydrofurane, à une température comprise entre 0°C et la température de reflux du solvant. Cette transformation peut être également réalisée en utilisant les conditions de *N*-arylation ou *N*-hétéroarylation de Buchwald, par exemple au moyen d'un catalyseur au Palladium ou au Cuivre.

Selon le schéma 2 voie B, les composés de formule générale (I), dans laquelle R₁ représente un groupe R₅ substitué notamment par un groupe R₆ de type C₁₋₆-alkyle, C₃₋₇-cycloalkyle ou C₃₋₇-cycloalkyle-C₁₋₃-alkylène, ou par un groupe R₇ tel que défini dans la formule générale (I) définie ci-dessus, peuvent être également préparés selon une réaction de couplage, catalysée au moyen d'un métal de transition, par exemple le Palladium (0), réalisée sur le composé de formule générale (Ib), dans laquelle A, R₂, R₃, R₄, R₅, m, n, o et p sont tels que définis dans la formule générale (I) et U₂ représente un atome de chlore, de brome, d'iode ou un groupement triflate, U₂ étant dans la position où l'on souhaite introduire le groupe R₆, ou R₇:
soit par une réaction de type Suzuki, par exemple au moyen d'un acide boronique d'alkyle, de cycloalkyle, d'aryle ou d'hétéroaryle,
soit selon une réaction de type Stille, par exemple en utilisant un dérivé *tri*-alkylstanneux d'aryle ou d'hétéroaryle soit par une réaction de type Négishi, par exemple en utilisant un dérivé zincate d'halogénure d'alkyle, de cycloalkyle, d'aryle ou d'hétéroaryle.

Ensuite, selon le schéma 2 voie B, l'intermédiaire de formule générale (Ib) telle que définie ci-dessus est préalablement obtenu en faisant réagir une amine de formule générale (Ia) telle que définie ci-dessus avec un dérivé de formule générale U₂-R₅-U₁ (Va), dans laquelle R₅, U₁ et U₂ sont tels que définis ci-dessus en utilisant les réactions de substitution nucléophile aromatique, hétéroaromatique ou de *N*-arylation, *N*-hétéroarylation de Buchwald, par exemple au moyen d'un catalyseur au Palladium ou au Cuivre.

Une variante d'obtention des intermédiaires de formule générale (Ib) (schéma 2 - variante voie B) consiste à faire réagir dans un premier temps une amine de formule générale (IIb), dans laquelle A, R₅, R₂, m, n, o et p sont tels que définis dans la formule générale (I) définie ci-dessus, et U₂ est tel que défini ci-dessus, avec un carbonate de formule générale (III) telle que définie ci-dessus, dans les conditions décrites ci-dessus lors de la réaction de l'amine de formule générale (II) avec le carbonate de formule générale (III), pour obtenir l'intermédiaire de formule générale (Ib), dans laquelle A, R₅, R₂, R₃, R₄, m, n, o et p sont tels que définis dans la formule générale (I), et U₂ est tel que défini ci-dessus.

Un autre objet de la présente invention se rapporte à un composé de formule (Ia) tel que décrit ci-dessus.

Un autre objet de la présente invention se rapporte à un composé de formule (Ic) tel que décrit ci-dessus.

Un autre objet de la présente invention se rapporte à un composé de formule (II) tel que décrit ci-dessus.

Un autre objet de la présente invention se rapporte à un composé de formule (IV) tel que décrit ci-dessus.

Les composés de formules générales (IIa), (IIb), (III), (IIIa), (V) et (Va) ainsi que les autres réactifs sont disponibles dans le commerce ou décrits dans la littérature, ou bien peuvent être préparés selon des méthodes qui y sont décrites ou qui sont connues de l'homme du métier. Notamment, le carbonate de formule générale (III) peut être préparé selon toute méthode décrite dans la littérature, par exemple par réaction d'un alcool de formule générale HOCHR₃R₄ (IIIa),dans laquelle R₃ et R₄ sont tels que définis dans la formule générale (I) telle que définie ci-dessus, avec le chloroformiate de phényle ou de 4-nitrophényle, en présence d'une base telle que la triéthylamine, la *N*-méthylmorpholine ou la diisopropyléthylamine, dans un solvant tel que le dichlorométhane ou le tétrahydrofurane, à une température comprise entre 0°C et la température ambiante.

Les exemples qui suivent illustrent la préparation de quelques composés de l'invention. Ces exemples ne sont pas limitatifs et ne font qu'illustrer l'invention. Les microanalyses, les spectres I.R. et R.M.N. et/ou la LC-MS (Liquid Chromatography coupled to Mass Spectroscopy) confirment les structures et les puretés des composés obtenus.
Méthode LC-MS (M+H) :
UPLC / TOF - Gradient 3 min - H₂O / ACN / TFA TO : 98%A - T1, 6 à T2, 1min : 100%B - T2,5 à T3min : 98%A Voie A : H2O + 0,05%TFA ; Voie B : ACN + 0,035%TFA Débit : 1.0mL/min - T°=40°C - Injection 2µL Colonne Acquity BEH C18 (50*2,1mm ; 1,7µm)"; 220 nm
PF(°C) représente le point de fusion en degrés Celsius.
R_{f} indique le temps de rétention obtenu par analyse CCM (Chromatographie sur Couche Mince).

Les numéros indiqués entre parenthèses dans les titres des exemples correspondent à ceux de la 1ère colonne des tableaux ci-après.

La nomenclature UICPA (Union Internationale de Chimie Pure et Appliquée - IUPAC en anglais) a été utilisée pour la dénomination des composés dans les exemples ci-dessous.

### Exemple 1 (Composé N°1)

### [7-(6-Fluoro-quinolin-2-yl)-7-aza-spiro[3.5]non-2-yl]-carbamate de thiazol-4-ylméthyle

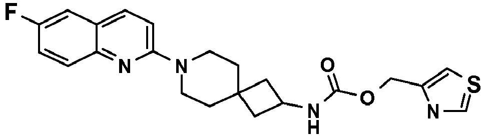

### 1.1. (Thiazol-4-ylméthoxycarbonylamino)-7-aza-spiro[3.5]nonane-7-carboxylate de benzyle

On chauffe à 50°C pendant 2 heures, une solution contenant 0,32 g (1,15 mmole) de 2-amino-7-aza-spiro[3.5]nonane-7-carboxylate de benzyle (WO92/22550), 0,36 g (1,26 mmole) de 4-nitro-phényl-carbonate de thiazol-4-ylméthyle (W02008/013834), 0,45 g (3,44 mmoles) de *N,N*-diisopropyléthylamine et de 0,014 g (0,11 mmole) de *N,N*-diméthylaminopyridine dans 5 mL de dichlorométhane.

On ajoute de l'eau au milieu réactionnel, on sépare la phase aqueuse, on l'extrait plusieurs fois avec du dichlorométhane, on lave les phases organiques réunies avec une solution aqueuse de soude (1N) puis avec une solution aqueuse saturée en chlorure d'ammonium. On les sèche sur sulfate de sodium et on concentre le filtrat sous pression réduite.

Après évaporation du solvant, on purifie le résidu obtenu par chromatographie sur gel de silice en éluant avec un mélange 95/5 de dichlorométhane et de méthanol.

On obtient ainsi 0,345 g de produit pur sous forme de poudre blanche.
LC-MS : M+H = 416
PF(°C) : 91-93°C
RMN ¹H (DMSO) δ (ppm) : 8,80 (s, 1H) ; 7,50-7.30 (m, 6H) ; 5,30 (s, 2H) ; 5,15 (s, 2H) ; 4,90 (large s, 1H) ; 4,15 (m, 1H) ; 3,50 (m, 2H) ; 3,30 (m, 2H) ; 2,30 (t, 2H) ; 1,70-1.40 (m, 6H).

### 1.2. (7-Aza-spiro[3.5]non-2-yl)-carbamate de thiazol-4-ylméthyle

A une solution de 0,29 g (0,70 mmole) de (thiazol-4-ylméthoxycarbonylamino)-7-aza-spiro [3.5]nonane-7-carboxylate de benzyle, obtenu à l'étape 1.1., dans 1 mL d'acide acétique, refroidie par un bain de glace/eau, on ajoute lentement 1,22 mL (6,98 mmoles) d'une solution d'acide bromhydrique 5,7N dans l'acide acétique. On poursuit l'agitation à température ambiante pendant 1 heure.

Après évaporation sous pression réduite, on reprend le résidu dans l'eau que l'on basifie avec une solution aqueuse de soude (30%). On extrait plusieurs fois au dichlorométhane puis on sèche les phases organiques réunies sur sulfate de sodium et on concentre le filtrat sous pression réduite. On obtient 0,138 g de produit sous forme d'une huile incolore utilisé tel quel dans l'étape suivante.

RMN ¹H (CDCl3) δ (ppm) : 8,80 (s, 1H) ; 7,30 (s, 1H) ; 5,20 (s, 2H) ; 4.80 (m, 1H) ; 4.50 (large s, 1H) ; 4,00 (m, 1H) ; 2.90-2.70 (m, 3H) ; 2.40-2.10 (m, 3H) ; 1,70-1.40 (m, 6H).

### 1.3. [7-(6-Fluoro-quinolin-2-yl)-7-aza-spiro[3.5]non-2-yl]-carbamate de thiazol-4-ylméthyle

Dans un tube scellé, on introduit 0,135g (0,48 mmole) de (7-aza-spiro[3.5]non-2-yl)-carbamate de thiazol-4-ylméthyle, obtenu à l'étape 1.2., 0,141g (0,62 mmole) de 2-bromo-6-fluoro-quinoline et 0,186 g (1,44 mmole) de *N,N-*diisopropyléthylamine dans 1,5 mL d'acétonitrile. On chauffe ensuite à 100°C pendant 12 heures. On laisse revenir à température ambiante puis on reprend le milieu réactionnel avec de l'acétate d'éthyle, on sépare la phase aqueuse, on l'extrait deux fois avec de l'acétate d'éthyle, on lave les phases organiques réunies avec une solution aqueuse saturée en chlorure d'ammonium et on les sèche sur sulfate de sodium. Après évaporation du solvant, on purifie le résidu obtenu par chromatographie sur plaques préparatives en éluant avec un mélange 95/5/0,5 de dichlorométhane, de méthanol et d'ammoniaque à 28%.

On obtient ainsi 0,100 g de produit pur sous forme de poudre blanche.
LC-MS : M+H = 427
PF(°C) : 107-109°C
RMN ¹H (DMSO) δ (ppm) : 8,80 (s, 1H) ; 7,80 (d, 1H) ; 7,60 (m, 1H) ; 7,35 (s, 1H) ; 7,30-7,15 (m, 2H); 7,00 (d, 1H) ; 5,30 (s, 2H) ; 4,90 (large s, 1H) ; 4,20 (m, 1H) ; 3,70-3,50 (m, 4H) ; 2,35 (t, 2H) ; 1,80-1,60 (m, 6H).

### Exemple 2 (Composé N°5)

### {7-[5-(4-Fluoro-phényl)-pyridin-2-yl]-7-aza-spiro[3.5]non-2-yl}-carbamate de 3-carbamoyl-isoxazol-5-ylméthyle

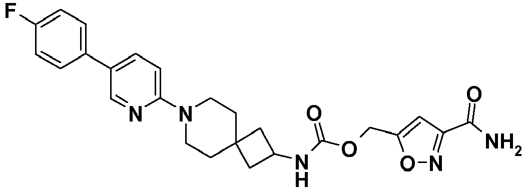

### 2.1. 2-Azido-7-aza-spiro[3.5]nonane-7-carboxylate de tert-butyle

On porte au reflux pendant 12 heures, sous atmosphère inerte, une solution de 5,90 g (18,47 mmoles) de 2-méthanesulfonyloxy-7-aza-spiro[3.5]nonane-7-carboxylate de *tert*-butyle (WO2003084948) et de 3,60 g (55,41 mmoles) d'azoture de sodium dans 27 mL de *N,N*-diméthylformamide.

On laisse revenir à température ambiante puis on reprend le milieu réactionnel par du dichlorométhane et de l'eau, on sépare la phase aqueuse, on l'extrait deux fois avec du dichlorométhane, on lave les phases organiques réunies avec une solution aqueuse saturée en chlorure de sodium et on les sèche sur sulfate de sodium. Après évaporation du solvant, on obtient 4,78 g de produit sous forme d'huile orange utilisé tel quel dans l'étape suivante.
RMN ¹H (CDCl3) δ(ppm) : 3,80 (m, 1H) ; 3,20 (m, 4H) ; 2,30-2,10 (m, 2H); 1,90-1,70 (m, 2H); 1,50 (m, 4H); 1,35 (s, 9H).

### 2.2. 2-Amino-7-aza-spiro[3.5]nonane-7-carboxylate de tert-butyle

A une solution de 4,78 g (17,95 mmoles) de 2-azido-7-aza-spiro[3.5]nonane-7-carboxylate de *tert*-butyle, obtenu à l'étape 2.1., dans 70 mL d'éthanol, on ajoute 1,85g (8,97 mmoles) de catalyseur de Lindlar (PdCaCO₃). Le milieu réactionnel est placé dans un appareil de Parr sous atmosphère d'hydrogène (20 Psi) à température ambiante pendant 5 heures. On filtre sur célite puis on concentre le filtrat sous pression réduite. On ajoute de l'eau et du dichlorométhane. On sépare la phase aqueuse puis on l'extrait trois fois avec du dichlorométhane, on lave les phases organiques réunies avec une solution aqueuse saturée en chlorure de sodium, on les sèche sur sulfate de sodium. Après évaporation du solvant, on obtient 3,62 g de produit sous forme d'huile utilisé tel quel dans l'étape suivante.
RMN ¹H (DMSO) δ(ppm) : 3,40 (m, 1H) ; 3,30-3,10 (m, 4H) ; 2,25-2,15 (m, 2H); 1.70 (large s, 2H) ; 1,50-1,35 (m, 6H); 1,30 (m, 9H) .

### 2.3. 2-(4-Nitro-phénoxycarbonylamino)-7-aza-spiro[3.5]nonane-7-carboxylate de tert-butyle

A une solution de 1,00 g (4,16 mmoles) de 2-amino-7-aza-spiro[3.5]nonane-7-carboxylate de *tert*-butyle, préparé à l'étape 2.2., de 1,34 g (10,40 mmoles) de *N,N-*diisopropyléthylamine et de 0,05 g (0,42 mmole) de *N,N-*diméthylaminopyridine dans 40 mL de dichlorométhane, refroidie à environ 0°C, on ajoute par petites portions 0,922 g (4,58 mmoles) de chloroformiate de 4-nitrophényle. On poursuit l'agitation à 0°C pendant 3 heures puis à température ambiante pendant 3 heures.On ajoute de l'eau au milieu réactionnel, on sépare la phase aqueuse, on l'extrait plusieurs fois avec du dichlorométhane, on lave les phases organiques réunies avec une solution aqueuse saturée en chlorure d'ammonium puis avec une solution aqueuse saturée en chlorure de sodium , on les sèche sur sulfate de sodium et on concentre le filtrat sous pression réduite. On obtient ainsi 1,8 g de produit sous forme de solide beige amorphe utilisé tel quel dans l'étape suivante.
RMN ¹H (CDCl3) δ(ppm) : 8,20(d,2H) ; 7,30(d,2H) ; 5.30 (large S, 1H) ; 4,10 (m, 1H) ; 3,25 (m, 4H) ; 2,30-2,10 (m, 2H); 1,70 (m, 2H); 1,50 (m, 4H); 1,40 (s, 9H).

### 2.4. 2-(3-Carbamoyl-isoxazol-5-ylméthoxycarbonylamino)-7-aza-spiro[3.5]nonane-7-carboxylate de tert-butyle

On chauffe dans un tube scellé à 90°C pendant 12 heures, une solution de 1,70 g (4,19 mmoles) de 2-(4-nitro-phénoxycarbonylamino)-7-aza-spiro[3.5]nonane-7-carboxylate de *tert*-butyle, préparé à l'étape 2.3., de 1,08 g (8,39 mmoles) de *N,N*-diisopropyléthylamine, de 0,033 g (0,27 mmole) de *N,N-*diméthylaminopyridine et de 0,05 g (0,42 mmole) de 3-carbamoyl-isoxazol-5-ylméthanol dans 20 mL de 1,2-dichloroéthane. On laisse revenir à température ambiante, on ajoute de l'eau au milieu réactionnel, on sépare la phase aqueuse, on l'extrait plusieurs fois avec du dichlorométhane, on lave les phases organiques réunies avec une solution aqueuse de soude (1N) puis avec une solution aqueuse saturée en chlorure d'ammonium, on les sèche sur sulfate de sodium et on concentre le filtrat sous pression réduite. L'huile obtenue est cristallisée dans l'éther et le solide ainsi obtenu est filtré puis rincé abondamment à l'éther. Après séchage sous vide à environ 40°C, on obtient 0,910 g de produit pur sous forme de poudre blanche.
LC-MS : M+H = 409
PF(°C) : 123-125°C
RMN ¹H (DMSO) δ (ppm) : 8,15 (large s, 1H) ; 7,80 (large s, 1H) ; 7,70 (d, 1H); 6,80 (s, 1H); 5,20 (s, 2H) ; 4,00 (m, 1H) ; 3,30-3,10 (m, 4H) ; 2,10 (m, 2H) ; 1,85 (m, 2H) ; 1,50 (m, 2H) ; 1, 40 (s, 9H) ; 1, 30 (m, 2H).

### 2.5. Chlorhydrate de (7-Aza-spiro[3.5]non-2-yl)-carbamate de 3-carbamoyl-isoxazol-5-ylméthyle

A une solution de 0,87 g (2,13 mmoles) de 2-(3-carbamoyl-isoxazol-5-ylméthoxycarbonylamino)-7-aza-spiro[3.5]nonane-7-carboxylate de *tert*-butyle, obtenu à l'étape 2.4., dans 2 mL de dioxane, refroidie par un bain de glace/eau, on ajoute lentement 8 mL (32 mmoles) d'une solution d'acide chlorhydrique 4N dans le dioxane. On poursuit l'agitation à température ambiante pendant 12 heures.

Après évaporation sous pression réduite, on obtient 0,77 g de produit sous forme de chlorhydrate utilisé tel quel dans l'étape suivante.
RMN ¹H (D₂O) δ(ppm) : 6,80 (s, 1H) ; 5,20 (s, 2H) ; 4,00 (m, 1H); 3,10 (m, 4H) ; 2,20 (m, 2H) ; 1,80 (m, 6H).

### 2.6. [7-(5-Bromo-pyridin-2-yl)-7-aza-spiro[3.5]non-2-yl]-carbamate de 3-carbamoyl-isoxazol-5-ylméthyle

Dans un tube scellé, on introduit 0,265 g (1,51 mmole) de 5-bromo-2-fluoro-pyridine, 0,40 g (1,16 mmole) de chlorhydrate de (7-aza-spiro[3.5]non-2-yl)-carbamate de 3-carbamoyl-isoxazol-5-ylméthyle, préparé à l'étape 2.5., et 0,60 g (4,64 mmoles) de *N,N*-diisopropyléthylamine dans 3,5 mL d'acétonitrile. On ajoute 2 mL de DMF puis on chauffe à 100°C pendant 12 heures. On laisse revenir à température ambiante puis on reprend le mélange réactionnel par du dichlorométhane et de l'eau. On sépare la phase aqueuse, on l'extrait deux fois avec du dichlorométhane, on lave les phases organiques réunies avec une solution aqueuse saturée en chlorure d'ammonium puis avec une solution aqueuse saturée en chlorure de sodium, on les sèche sur sulfate de sodium et on concentre le filtrat sous pression réduite. L'huile obtenue est triturée dans le diisopropyléther. Le solide ainsi obtenu est filtré puis rincé abondamment à l'éther. Après séchage sous vide à environ 40°C, on obtient 0,195 g de produit pur sous forme de poudre beige.
LC-MS : M+H = 465
PF(°C) : 165-167°C
RMN ¹H (DMSO) δ (ppm) : 8,10 (s, 2H) ; 7,80 (s, 1H) ; 7,70 (d, 1H); 7,60 (d, 1H) ; 6,85 (d, 1H); 6,75 (s, 1H); 5,20 (s, 2H) ; 4,00 (m, 1H) ; 3,55 (t, 2H) ; 3,45 (t, 2H) ; 2,20 (m, 2H) ; 1,75 (m; 2H); 1,65-1,45 (m, 4H).

### 2.7. {7-[5-(4-Fluoro-phényl)-pyridin-2-yl]-7-aza-spiro[3.5]non-2-yl}-carbamate de 3-carbamoyl-isoxazol-5-ylméthyle

Sous atmosphère inerte, on introduit 0,160 g (0,34 mmole) de [7-(5-bromo-pyridin-2-yl)-7-aza-spiro[3.5]non-2-yl]-carbamate de 3-carbamoyl-isoxazol-5-ylméthyle, obtenu à l'étape 2.6., 0,058 g (0,41 mmole) d'acide 4-fluorophénylboronique, 0,349 g (1,03 mmole) de carbonate de cesium en suspension dans 3 mL d'un mélange 9/1 de tétrahydrofurane et d'eau. On ajoute ensuite 0,028 g (0,03 mmole) de PdCl₂dppf.CH₂Cl₂. On chauffe ensuite à environ 75°C pendant 12 heures. On laisse revenir à température ambiante, on sépare les sels par filtration sur célite, puis on reprend le filtrat avec du dichlorométhane et de l'eau. On sépare la phase aqueuse, on l'extrait deux fois avec du dichlorométhane, on lave les phases organiques réunies avec une solution aqueuse saturée en chlorure de sodium et on les sèche sur sulfate de sodium. Après évaporation du solvant, on purifie le résidu obtenu par chromatographie sur plaques préparatives en éluant avec un mélange 90/10/1 de dichlorométhane, de méthanol et d'ammoniaque à 28%.

On obtient ainsi 0,084 g de produit pur sous forme de poudre blanche.
LC-MS : M+H = 480
PF(°C) : 216-218°C
RMN ¹H (DMSO) δ (ppm) : 8,40 (s, 1H) ; 8,20 (large s, 1H) ; 7,90-7,70 (m, 3H); 7,60 (m, 2H); 7,25 (m, 2H); 6,90 (d, 1H) ; 6,75(s, 1H); 5,20 (s, 2H) ; 4,00 (m, 1H) ; 3,55 (t, 2H) ; 3,45 (t, 2H) ; 2,20 (m, 2H) ; 1,75 (m; 2H); 1,65-1,45 (m, 4H) .

### Exemple 3 (Composé N°2)

### [7-(6-Fluoro-quinolin-2-yl)-7-aza-spiro[3.5]non-2-yl]-carbamate de 3-carbamoyl-isoxazol-5-ylméthyle

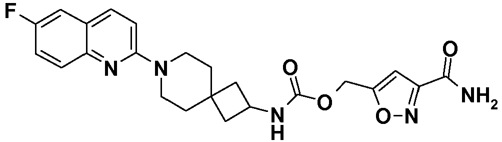

On procède suivant le mode opératoire décrit dans l'exemple 2 (étape 2.6.). A partir de 0,20 g (0,58 mmole) de chlorhydrate de (7-aza-spiro[3.5]non-2-yl)-carbamate de 3-carbamoyl-isoxazol-5-ylméthyle, décrit dans l'exemple 2 (étape 2.5.), de 0,170g (0,75 mmole) de 2-bromo-6-fluoro-quinoline et de 0,30g (2,32 mmoles) de *N,N*-diisopropyléthylamine et après chromatographie sur plaques préparatives en éluant avec un mélange 90/10/1 de dichlorométhane, de méthanol et d'ammoniaque à 28%, on obtient 0,05 g de produit pur sous forme de poudre blanche.
LC-MS : M+H = 455
PF(°C) : 226-228°C
RMN ¹H (DMSO) δ (ppm) : 8,15 (large s, 1H) ; 8,00 (d, 1H) ; 7,85 (large s, 1H); 7,70 (d, 1H); 7,60 (m, 1H); 7,50 (m, 1H); 7,40 (m, 1H) ; 7,30 (d, 1H); 6,70 (s, 1H); 5,20 (s, 2H) ; 4,10 (m, 1H) ; 3,70-3,50 (m, 4H) ; 2,35 (t, 2H) ; 1,80 (t, 2H) ; 1,70-1,40 (m, 4H).

### Exemple 4 (Composé N°9)

### [7-(6-Fluoro-quinolin-2-yl)-7-aza-spiro[3.5]non-2-yl]-carbamate de 3-(méthylcarbamoyl)-isoxazol-5-ylméthyle

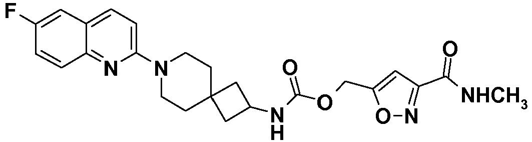

### 4.1. 4-Nitro-phényl-carbonate de 3-(méthylcarbamoyl)-isoxazol-5-ylméthyle

A une solution de 2,00 g (12,81 mmoles) de 3-(méthylcarbamoyl)-isoxazol-5-ylméthanol, de 1,52 g (19,21 mmoles) de pyridine et de 0,157 g (1,28 mmole) de *N,N-*diméthylaminopyridine, dans 15 mL de dichlorométhane, refroidie à environ 0°C, on ajoute par petites portions 2,58 g (12,81 mmoles) de chloroformiate de 4-nitrophényle. On poursuit l'agitation à 0°C pendant 1 heure puis à température ambiante pendant 1 heure. Le précipité ainsi formé filtré puis rincé abondamment avec le diisopropyléther. Après séchage sous vide à environ 60°C, on est obtient 2.60 g de produit sous forme de poudre blanche utilisée tel quel dans l'étape suivante.
RMN ¹H (CDCl₃) δ (ppm) : 8, 40 (d, 2H) ; 7,50 (d, 2H) ; 7,0 (s, 1H) ; 6,90 (large s, 1H) ; 5,50 (s, 2H) ; 3,10 (d, 3H).

### 4.2. 2-[3-(Méthylcarbamoyl)-isoxazol-5-ylméthoxycarbonylamino]-7-aza-spiro[3.5]nonane-7-carboxylate de tert-butyle

On procède suivant le mode opératoire décrit dans l'exemple 1 à l'étape 1.1. A partir de 0,3 g (1,25 mmole) de 2-amino-7-aza-spiro[3.5]nonane-7-carboxylate de *tert*-butyle, décrit dans l'exemple 2 (étape 2.2.), de 0,481 g (1,50 mmole) de 4-nitro-phényl-carbonate de 3-(méthylcarbamoyl)-isoxazol-5-ylméthyle, obtenu à l'étape 4.1., de 0,403 g (3,12 mmoles) de *N,N-*diisopropyléthylamine et de 0,076 g (0,62 mmole) de *N,N-*diméthylaminopyridine, et après précipitation à l'éther et filtration, on obtient 0,364 g de produit sous forme de solide beige amorphe utilisé tel quel dans l'étape suivante.
LC-MS : M+H = 423
RMN ¹H (DMSO) δ (ppm) : 8,80 (large s, 1H) ; 7,80 (d, 1H) ; 6,80 (d, 1H); 5,20 (s, 2H); 4,00 (m, 1H) ; 3,30-3,10 (m, 4H) ; 2,80 (s, 3H) ; 2,10 (m, 2H) ; 1,70 (m, 2H) ; 1,50 (m, 2H) ; 1,40 (s, 9H) ; 1,30 (m, 2H).

### 4.3. Chlorhydrate de (7-aza-spiro[3.5]non-2-yl)-carbamate de 3-(méthylcarbamoyl)-isoxazol-5-ylméthyle

On procède suivant le mode opératoire décrit dans l'exemple 2 à l'étape 2.5. A partir de 0,364 g (0,86 mmole) de 2-[3-(méthylcarbamoyl)-isoxazol-5-ylméthoxycarbonylamino]-7-aza-spiro[3.5]nonane-7-carboxylate de *tert*-butyle, obtenu à l'étape 4.2., et de 3,25mL (12,92 mmoles) d'une solution d'acide chlorhydrique 4N dans le dioxane, on obtient 0,32 g de produit sous forme de chlorhydrate utilisé tel quel dans l'étape suivante.
LC-MS : M+H = 359
RMN ¹H (DMSO) δ (ppm) : 8,80 (large s, 2H) ; 7,80 (d, 1H) ; 6,80 (s, 1H); 5,20 (s, 2H); 4,0 (m, 1H) ; 3,00-2,85 (m, 4H) ; 2,75 (s, 3H) ; 2,20 (m, 2H) ; 1,70 (m, 4H) ; 1,60 (m, 2H).

### 4.4. [7-(6-Fluoro-quinolin-2-yl)-7-aza-spiro[3.5]non-2-yl]-carbamate de 3-(méthylcarbamoyl)-isoxazol-5-ylméthyle

On procède suivant le mode opératoire décrit dans l'exemple 1 (étape 1.3.). A partir de 0,163 g (0,45 mmole) de chlorhydrate de (7-aza-spiro[3.5]non-2-yl)-carbamate de 3-(méthylcarbamoyl)-isoxazol-5-ylméthyle, obtenu à l'étape 4.3., de 0,133g (0,59 mmole) de 2-bromo-6-fluoro-quinoline et de 0,234g (1,82 mmole) de *N,N*-diisopropyléthylamine et après purification par chromatographie sur plaques préparatives en éluant avec un mélange 92,5/7,5/0,75 de dichlorométhane , de méthanol et d'ammoniaque à 28%, on obtient 0,068g de produit pur sous forme de poudre blanche.
LC-MS : M+H = 468
PF(°C) : 193-195°C
RMN ¹H (DMSO) δ (ppm) : 8,70 (large s, 1H) ; 8,00 (d, 1H) ; 7,85 (d, 1H); 7,70 (m, 1H); 7,60 (m, 1H); 7,50 (m, 1H); 7,40 (m, 1H); 6,80 (s, 1H); 5,20 (s, 2H) ; 4,10 (m, 1H) ; 3,70-3,50 (m, 4H) ; 2, 80 (s, 3H) ; 2,20 (t, 2H) ; 1,70 (t, 2H) ; 1,70-1,50 (m, 4H).

### Exemple 5 (Composé N°6)

### [7-(6-Fluoro-quinolin-2-yl)-7-aza-spiro[3.5]non-2-yl]-méthylcarbamate de 3-carbamoyl-isoxazol-5-ylméthyle

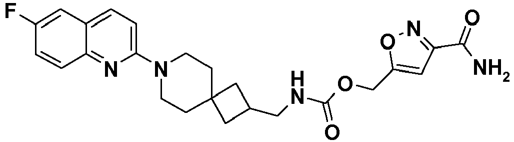

### 5.1. 2-(Aminométhyl)-7-aza-spiro[3.5]nonane-7-carboxylate de tert-butyle

A une solution de 1,40 g (5,59 mmoles) de 2-cyano-7-aza-spiro[3.5]nonane-7-carboxylate de tert-butyle (Chem. Pharm. Bull.; 52(6),675-687, 2004), dans 10 mL d'une solution de soude 1N dans l'éthanol. On ajoute ensuite 0,164g (2,80 mmoles) de Nickel de Raney. Le milieu réactionnel est placé dans un appareil de Parr sous atmosphère d'hydrogène (60 psi) à température ambiante pendant 2 heures. On filtre sur büchner puis on concentre le filtrat sous pression réduite. On ajoute du dichlorométhane, on sépare la phase aqueuse, on l'extrait trois fois avec du dichlorométhane, on lave les phases organiques réunies avec une solution aqueuse saturée en chlorure de sodium, on les sèche sur sulfate de sodium. Après évaporation du solvant, on obtient 1,212 g de produit sous forme d'une huile incolore utilisé tel quel dans l'étape suivante.
LC-MS : M+H = 255
RMN ¹H (DMSO) δ (ppm) : 3,30-3,10 (m, 4H) ; 2,50 (d, 2H) ; 2,10 (m, 1H) ; 1,80 (m, 2H) ; 1,40 (m, 2H) ; 1,30 (m, 13H).

### 5.2. 2-[(4-Nitro-phénoxycarbonylamino)-méthyl]-7-aza-spiro[3.5]nonane-7-carboxylate de tert-butyle

On procède suivant le mode opératoire décrit dans l'exemple 2 (étape 2.3.) . A partir de 1,10 g (4,32 mmoles) de 2-aminométhyl-7-aza-spiro[3.5]nonane-7-carboxylate de *tert-*butyle, préparé à l'étape 5.1., de 1,40g (10,81 mmoles) de *N*,*N*-diisopropyléthylamine, de 0,053 g (0,43 mmole) de *N,N-*diméthylaminopyridine et de 0,959 g (4,76 mmoles) de chloroformiate de 4-nitrophényle, on obtient 1,8g de produit sous forme d'une huile de couleur jaune utilisé tel quel dans l'étape suivante.

### 5.3. 2-[(3-Carbamoyl-isoxazol-5-ylméthoxycarbonylamino)-méthyl]-7-aza-spiro[3.5]nonane-7-carboxylate de tert-butyle

On procède suivant le mode opératoire décrit dans l'exemple 2 (étape 2.4.). A partir de 0,50 g (1,19 mmole) de 2-[(4-nitro-phénoxycarbonylamino)-méthyl]-7-aza-spiro[3.5]nonane-7-carboxylate de *tert*-butyle, préparé à l'étape 5.2., de 0,337g (2,38 mmoles) de *N,N*-diisopropyléthylamine, de 0,073 g (0,60 mmole) de *N,N*-diméthylaminopyridine et de 0,169 g (1,19 mmole) de 3-carbamoyl-isoxazol-5-ylméthanol, on obtient 0,50g de produit sous forme d'une huile utilisé tel quel dans l'étape suivante.

### 5.4. Chlorhydrate de (7-aza-spiro[3.5]non-2-ylméthyl)-carbamate de 3-carbamoyl-isoxazol-5-ylméthyle

On procède suivant le mode opératoire décrit dans l'exemple 2 à l'étape 2.5. A partir de 0,50g (1,18 mmole) de 2-[(3-carbamoyl-isoxazol-5-ylméthoxycarbonylamino)-méthyl]-7-aza-spiro[3.5]nonane-7-carboxylate de tert-butyle, obtenu à l'étape 5.3., et de 2,96mL (11,83 mmoles) d'une solution d'acide chlorhydrique 4N dans le dioxane, on obtient 0,309g de produit sous forme de chlorhydrate utilisé tel quel dans l'étape suivante.
LC-MS : M+H = 359
PF(°C) : 120-122
RMN ¹H (DMSO) δ (ppm) : 8,10 (large s, 1H); 7,80 (large s, 1H); 7,50 (large s, 1H) ; 6,80 (s, 1H); 5,20 (s, 2H); 3,10 (m, 2H) ; 2,90 (m, 2H) ; 2,80 (m, 2H) ; 2,40 (m, 1H) ; 1,90 (t, 2H) ; 1,75 (m, 2H) ; 1,60 (m, 2H) ; 1,50 (t, 3H).

### 5.5. [7-(6-Fluoro-quinolin-2-yl)-7-aza-spiro[3.5]non-2-yl]-méthylcarbamate de 3-carbamoyl-isoxazol-5-ylméthyle

On procède suivant le mode opératoire décrit dans l'exemple 1 (étape 1.3.). A partir de 0,167g (0,47 mmole) de chlorhydrate de (7-aza-spiro[3.5]non-2-ylméthyl)-carbamate de 3-carbamoyl-isoxazol-5-ylméthyle, décrit dans l'étape 5.4., de 0,137g (0,60 mmole) de 2-bromo-6-fluoro-quinoline et de 0,180g (1,40 mmole) de *N,N*-diisopropyléthylamine et après purification par chromatographie sur plaques préparatives en éluant avec un mélange 90/10/1 de dichlorométhane , de méthanol et d'ammoniaque à 28%, on obtient 0,05g de produit pur sous forme de poudre blanche.
LC-MS : M+H = 468
PF(°C) : 190-192°C
RMN ¹H (DMSO) δ (ppm) : 8,15 (large s, 1H) ; 8,0 (d, 1H) ; 7,85 (large s, 1H); 7,60-7,35 (m, 4H); 7,25 (d, 1H); 6,75 (s, 1H); 5,20 (s, 2H) ; 3,70 (t, 2H) ; 3,60 (t, 2H) ; 3,10 (t, 2H) ; 2,40 (m, 1H) ; 1,90 (t, 2H) ; 1,70 (m, 2H) ; 1,60 (m, 4H) .

### Exemple 6 (Composé N°10)

### {6-[5-(4-Fluoro-phényl)-pyridin-2-yl]-6-aza-spiro[3.4]oct-2-yl}-carbamate de 3-carbamoyl-isoxazol-5-ylméthyle (Mélange d'isomères)

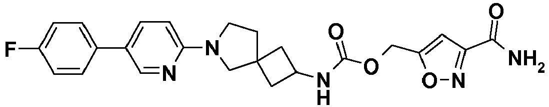

### 6.1. 2-Hydroxy-6-aza-spiro[3.4]octane-6-carboxylate de tert-butyle

A une solution de 3,54g (15,71 mmoles) de 2-oxo-6-aza-spiro[3.4]octane-6-carboxylate de *tert*-butyle (WO98/06720) dilué dans 40 mL de méthanol, est ajouté 0,89g (23,57 mmoles) de borohydrure de sodium par portion à 0 °C. Le mélange réactionnel est agité à température ambiante pendant 1 heure et 30 minutes. Après évaporation du solvant, on ajoute de l'eau au milieu réactionnel, on sépare la phase aqueuse, on l'extrait plusieurs fois avec de l'éther diéthylique, on lave les phases organiques réunies avec une solution aqueuse saturée en chlorure de sodium, on les sèche sur sulfate de sodium et on concentre le filtrat sous pression réduite. Après évaporation du solvant, on obtient 3,10g de produit sous forme d'une huile marron utilisé tel quel dans l'étape suivante.
RMN ¹H (DMSO) δ (ppm) : 4,7 (t, 1H) ; 4,1 (m, 1H) ; 3,2 (m, 4H), ; 2,2 (m, 2H) ; 1,8 (m, 4H); 1,4 (s, 9H).

### 6.2. 2-Méthanesulfonyloxy-6-aza-spiro[3.4]octane-6-carboxylate de tert-butyle

A une solution de 1,52g (4,99 mmoles) de 2-hydroxy-6-aza-spiro[3.4]octane-6-carboxylate de *tert*-butyle, obtenu à l'étape 6.1., dans 45mL de dichlorométhane, sont ajoutés 0,76mL (5,49 mmoles) de triéthylamine puis 0,43mL (5,49 mmoles) de chlorure de mésyle. Le milieu réactionnel est agité à température ambiante pendant 1 heure et 30 minutes. Après évaporation du solvant, on ajoute de l'eau au milieu réactionnel, on sépare la phase aqueuse, on l'extrait plusieurs fois avec du dichlorométhane, on lave les phases organiques réunies avec une solution aqueuse saturée en chlorure de sodium, on les sèche sur sulfate de sodium et on concentre le filtrat sous pression réduite. Après évaporation du solvant, on obtient 1,90 g de produit sous forme d'une huile marron utilisé tel quel dans l'étape suivante.

### 6.3. 2-Azido-6-aza-spiro[3.4]octane-6-carboxylate de tert-butyle

On porte au reflux pendant 12 heures, sous atmosphère inerte, une solution de 0,46 g (1,51 mmole) de 2-méthanesulfonyloxy-6-aza-spiro[3.4]octane-6-carboxylate de tert-butyle ,préparé à l'étape 6.2., et de 0,19 g (3,01 mmoles) d'azoture de sodium dans 5 mL de *N,N*-diméthylformamide. On laisse revenir à température ambiante puis on reprend le milieu réactionnel par de l'acétate d'éthyle et de l'eau. On sépare la phase aqueuse, on l'extrait deux fois avec de l'acétate d'éthyle, on lave les phases organiques réunies avec une solution aqueuse saturée en chlorure d'ammonium et on les sèche sur sulfate de sodium. Après évaporation du solvant, on obtient 0,380 g de produit sous forme d'huile orange utilisé tel quel dans l'étape suivante.
RMN ¹H (DMSO) δ(ppm) : 4,0 (m, 1H) ; 3,4 (m, 4H) ; 2,4 (m, 2H) ; 2,2 (m, 2H) ; 1,9 (m, 2H) ; 1,5 (s, 9H).

### 6.4. 2-Amino-6-aza-spiro[3.4]octane-6-carboxylate de tert-butyle

A une solution de 3,67 g (14,54 mmoles) de 2-azido-6-aza-spiro[3.4]octane-6-carboxylate de *tert*-butyle, obtenu à l'étape 6.3., dans 60 mL d'éthanol, on ajoute 1,50 g (7,27 mmoles) de catalyseur de Lindlar (PdCaCO₃). Le milieu réactionnel est placé dans un appareil de Parr sous atmosphère d'hydrogène à 20 Psi, à température ambiante, pendant 5 heures. On filtre sur célite puis on concentre le filtrat sous pression réduite. Après évaporation du solvant, on purifie le résidu obtenu par chromatographie sur gel de silice en éluant avec un mélange 95/5/0,5 de dichlorométhane, de méthanol et d'ammoniaque à 28%.

On obtient ainsi 1,57 g de produit pur sous forme d'une huile de couleur marron.
RMN ¹H (DMSO) δ(ppm): 3,35 (m, 1H) ; 3,25-3,10 (m, 4H) ; 2,2 (m, 2H) ; 1,80 (t, 2H) ; 1,6 (m, 2H) ; 1,4 (s, 9H).

### 6.5. 2-(3-Carbamoyl-isoxazol-5-ylméthoxycarbonylamino)-6-aza-spiro[3.4]octane-6-carboxylate de tert-butyle

A une solution contenant 0,284 g (1,66 mmole) de 5-hydroxyméthyl-isoxazole-3-carboxylate d'éthyle et 0,39 g (3,02 mmoles) de *N,N*-diisopropyléthylamine dans 10 mL de 1,2-dichloroéthane, refroidie à environ 0°C, on ajoute au goutte à goutte une solution de 0,304 g (1,51 mmole) de chloroformiate de 4-nitrophényle dissout dans 5 mL de 1,2-dichloroéthane. On poursuit l'agitation à 0°C pendant 1 heure puis à température ambiante pendant 1 heure. On ajoute ensuite 0,39 g (3,02 mmoles) de *N,N*-diisopropyléthylamine puis 0,34 g (1,51 mmole) de 2-amino-6-aza-spiro[3.4]octane-6-carboxylate de *tert-*butyle, préparé à l'étape 6.4. Le milieu réactionnel est agité à 70°C pendant 4 heures. On laisse revenir à température ambiante. On ajoute de l'eau au milieu réactionnel, on sépare la phase aqueuse, on l'extrait plusieurs fois avec du dichlorométhane, on lave les phases organiques réunies avec une solution aqueuse de soude (1N) puis avec une solution aqueuse saturée en chlorure d'ammonium, on les sèche sur sulfate de sodium et on concentre le filtrat sous pression réduite. On obtient ainsi 0,44 g de produit pur sous forme d'une huile de couleur orange utilisé tel quel dans l'étape suivante.
LC-MS : M+H = 424
RMN ¹H (DMSO) δ (ppm) : 7,80 (large s, 1H) ; 6,90 (s, 1H) ; 5,20 (s, 2H) ; 4,40 (q, 2H) 4,00 (m, 1H) ; 3,40 -3,10 (m, 4H) ; 2,30 (m, 2H) ; 2,00-1,70 ( m, 4H) ; 1,40 (s, 9H) ; 1,30 (t, 3H).

### 6.6. Trifluoroacétate de 5-(6-aza-spiro[3.4]oct-2-ylcarbamoyloxyméthyl)-isoxazole-3-carboxylate d'éthyle

A une solution de 0,44 g (1,04 mmole) de 2-(3-carbamoyl-isoxazol-5-ylméthoxycarbonylamino)-6-aza-spiro[3.4]octane-6-carboxylate de *tert*-butyle, obtenu à l'étape 6.5., dans 10 mL de dichlorométhane, refroidie par un bain de glace/eau, on ajoute lentement 0,88 mL (10,39 mmoles) d'une solution d'acide trifluoroacétique. On poursuit l'agitation à température ambiante pendant 4 heures.

Après évaporation sous pression réduite, on obtient 0,45 g de produit sous forme de trifluoroacétate utilisé tel quel dans l'étape 6.8. ci-dessous.

### 6.7. 2-Fluoro-5-(4-fluoro-phényl)-pyridine

A une solution de 2,0 g (14,29 mmoles) de 5-bromo-2-fluoro-pyridine dans 140 mL d'un mélange 4 /1 de toluène et d'éthanol, est ajoutée 2,51 g (14,29 mmoles) d'acide 4-fluorophénylboronique, 0,825 g (0,71 mmole) de Pd(PPh₃)₄ et 50 mL d'une solution 1M de carbonate de sodium. Le mélange est agité à 90 °C pendant 2 heures.

On laisse revenir à température ambiante. On extrait plusieurs fois avec de l'acétate d'éthyle. Les phases organiques sont ensuite séchées sur sulfate de sodium et évaporées à sec. On obtient 2,3 g de produit pur sous forme d'une poudre blanche.
PF(°C) : 98-100
RMN ¹H (DMSO) δ (ppm) : 8,55 (m, 1H), 8,28 (dd, 1H), 7,78 (m, 2H), 7,54 (m, 2H), 7,28 (dd, 1H).

### 6.8. 5-{6-[5-(4-Fluoro-phényl)-pyridin-2-yl]-6-aza-spiro[3.4]oct-2-ylcarbamoyloxyméthyl}-isoxazole-3-carboxylate d'éthyle

On procède suivant le mode opératoire décrit dans l'exemple 1 (étape 1.3.). A partir de 0,45 g (1,04 mmole) de trifluoroacétate de 5-(6-aza-spiro[3.4]oct-2-ylcarbamoyloxyméthyl)-isoxazole-3-carboxylate d'éthyle, décrit dans l'étape 6.6., de 0,198 g (1,04 mmole) de 2-fluoro-5-(4-fluoro-phényl)-pyridine, préparé à l'étape 6.7. et 0,40 g (3,12 mmoles) de *N,N*-diisopropyléthylamine, et après purification par chromatographie sur plaques préparatives en éluant avec un mélange 96/4 de dichlorométhane et de méthanol, on obtient 0,10 g de produit pur sous forme de cire utilisé tel que dans l'étape suivante.

### 6.9. {6-[5-(4-Fluoro-phényl)-pyridin-2-yl]-6-aza-spiro[3.4]oct-2-yl}-carbamate de 3-carbamoyl-isoxazol-5-ylméthyle

Dans un tube scellé, une solution de 0,045 g (0,09 mmole) de 5-{6-[5-(4-fluoro-phényl)-pyridin-2-yl]-6-aza-spiro[3.4]oct-2-ylcarbamoyloxyméthyl}-isoxazole-3-carboxylate d'éthyle, préparé à l'étape 6.8., dans 1,3 mL (9,10 mmoles) d'une solution d'ammoniaque (7M) dans le méthanol, est agitée à 70°C pendant 2 heures.

On laisse revenir à température ambiante puis on évapore à sec. Le résidu obtenu cristallise dans le méthanol à chaud. Le précipité ainsi formé est filtré puis rincé abondamment à l'éther. Après séchage sous vide à environ 40°C, on obtient 0,01 g de produit pur sous forme de poudre blanche.
PF(°C) : 216-218°C
RMN ¹H (DMSO) δ (ppm) : 8,40 (s, 1H) ; 8,20 (large s, 1H) ; 7,80 (large s, 3H) ; 7,60 (s, 2H) ; 7,30 (s, 2H) ; 6,80 (s, 1H) ; 6,50 (t, 1H) ; 5,20 (s, 2H) ; 4,10 (m, 1H) ; 3,50 -3,30 (m, 4H) ; 2,30 (m, 2H) ; 2,00 ( m, 4H).

### Exemple 7 (Composé N°11)

### {6-[5-(4-Fluoro-phényl)-pyridin-2-yl]-6-aza-spiro[3.4]oct-2-yl}-carbamate de 3-(méthylcarbamoyl)-isoxazol-5-ylméthyle (Mélange d'isomères)

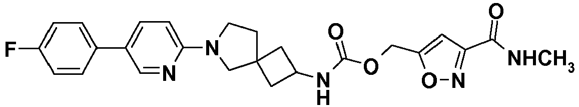

On procède suivant le mode opératoire décrit dans l'exemple 6 (étape 6.9.). A partir de 0,045 g (0,09 mmole) de 5-{6-[5-(4-fluoro-phényl)-pyridin-2-yl]-6-aza-spiro[3.4]oct-2-ylcarbamoyloxyméthyl}-isoxazole-3-carboxylate d'éthyle, décrit dans l'exemple 6 (étape 6.8.) et de 1,14 mL d'une solution de méthylamine (8M) dans l'éthanol, et après purification par chromatographie sur plaques préparatives en éluant avec de l'acétate d'éthyle, on obtient 0,009 g de produit pur sous forme de poudre blanche.
PF(°C) : 184-186°C
RMN ¹H (DMSO) δ (ppm) : 8,70 (s, 1H) ; 8,40 (s, 1H) ; 7,80 (m, 2H) ; 7,60 (m, 2H) ; 7,30 (m, 2H) ; 6,80 (s, 1H) ; 6,50 (t, 1H) ; 5,20 (s, 2H) ; 4,10 (m, 1H) ; 3,50-3,30 (m, 4H) ; 2,80 (s, 3H) ; 2,30 (m, 2H) ; 2,00 ( m, 4H).

### Exemple 8 (Composé N°12)

### 6-[5-(4-Fluoro-phényl)-pyridin-2-yl]-6-aza-spiro[3.4]oct-2-ylméthylcarbamate de 3-(méthylcarbamoyl)-isoxazol-5-ylméthyle (isomère I)

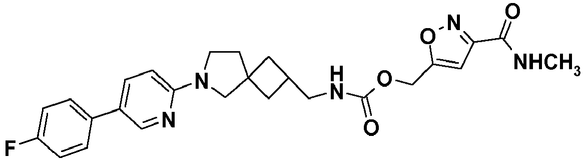

### 8.1. 2-Cyano-6-aza-spiro[3.4]octane-6-carboxylate de tert-butyle : isomères 1a et 1b

A une solution de 1,90 g (6,22 mmoles) de 2-méthanesulfonyloxy-6-aza-spiro[3.4]octane-6-carboxylate de *tert*-butyle, préparé dans l'exemple 6 (étape 6.2.), dans 20 mL de diméthylsulfoxyde anhydre, sont ajoutés 3,05 g (62,21 mmoles) de cyanure de sodium. Le mélange réactionnel est ensuite agité à 130 °C pendant 12 heures.

On laisse revenir à température ambiante puis on reprend le milieu réactionnel avec de l'éther, on sépare la phase aqueuse, on l'extrait deux fois avec de l'éther puis on sèche les phases organiques réunies sur sulfate de sodium. Après évaporation du solvant, on purifie le résidu obtenu par chromatographie sur gel de silice en éluant avec un mélange 5/95 d'acétate d'éthyle et de cyclohexane. On obtient ainsi 0,27 g de l'isomère 1a sous forme de cristaux blancs et 0,20 g de l'isomère 1b sous forme d'une huile jaune.
Isomère 1a
   PF (°C) : 62-64°C
   R_{f} = 0,49 (Acétate d'éthyle / cyclohexane : 50 / 50)
   RMN ¹H (DMSO) δ (ppm) : 3,4 (m, 1H) ; 3,1 (m, 4H) ; 2,5 (m, 4H) ; 2,3 (t, 2H) ; 1,4 (s, 9H).
Isomère 1b
   R_{f} = 0,42 (Acétate d'éthyle / cyclohexane : 50 / 50)
   RMN ¹H (DMSO) δ (ppm) : 3,4 (m, 1H) ; 3,3 (m, 2H) ; 3,2 (m, 2H) ; 2,5 (s, 4H) ; 1,9(m, 2H) ; 1,4 (s, 9H).

### 8.2. 2-Aminométhyl-6-aza-spiro[3.4]octane-6-carboxylate de tert-butyle : isomère 2a

A une solution de 0,27 g (1,14 mmole) de 2-cyano-6-aza-spiro[3.4]octane-6-carboxylate de *tert*-butyle (isomère 1a), obtenu à l'étape 8.1., dans 10 mL d'une solution de soude 1N dans l'éthanol, on ajoute le Nickel de Raney en quantité catalytique. Le milieu réactionnel est placé dans un appareil de Parr sous atmosphère d'hydrogène (4 bars), à température ambiante, pendant 5 heures.

On filtre sur célite puis on concentre le filtrat sous pression réduite. On ajoute du dichlorométhane, on sépare la phase aqueuse, on l'extrait trois fois avec du dichlorométhane, on lave les phases organiques réunies avec une solution aqueuse saturée en chlorure de sodium, on les sèche sur sulfate de sodium. Après évaporation du solvant, on obtient 0,24 g de produit sous forme d'une huile jaune utilisé tel quel dans l'étape suivante.
Isomère 2a
   LC-MS : M+H = 241
   RMN ¹H (DMSO) δ (ppm) : 3,2 (m, 2H) ; 3,1 (m, 2H) ; 2,5 (m, 2H) ; 2,2 (m, 1H) ; 1,9 (m, 2H) ; 1,7 (m, 2H) ; 1,6 (m, 2H) ; 1,4 (s, 9H).

### 8.3. 2-{[3-(Méthylcarbamoyl)-isoxazol-5-ylméthoxycarbonylamino]-méthyl}-6-aza-spiro[3.4]octane-6-carboxylate de tert-butyle

On procède suivant le mode opératoire décrit dans l'exemple 1 à l'étape 1.1. A partir de 0,22 g (0,92 mmole) de 2-aminométhyl-6-aza-spiro[3.4]octane-6-carboxylate de *tert-*butyle (isomère 2a), décrit dans l'exemple 8 (étape 8.2.), de 0,294 g (0,92 mmole) de 4-nitro-phényl-carbonate de 3-(méthylcarbamoyl)-isoxazol-5-ylméthyle, obtenu à l'étape 4.1., de 0,236 g (1,83 mmole) de *N,N*-diisopropyléthylamine et de 0,011g (0,09 mmole) de *N,N*-diméthylaminopyridine, et après purification par chromatographie sur gel de silice en éluant avec un mélange 98/2 de dichlorométhane et de méthanol, on obtient ainsi 0,310 g de produit pur sous forme d'un solide amorphe.
LC-MS : M+H = 423
RMN ¹H (DMSO) δ (ppm) : 8,80 (large s, 1H) ; 7,50 (large s, 1H) ; 6,80 (s, 1H); 5,20 (s, 2H); 3,25 (m, 2H) ; 3,15 (m, 2H) ; 3,10 (m, 2H); 2,80 (s, 3H) ; 2,40 (m, 1H) ; 2,00-1,80 (m, 4H) ; 1,70 (m, 2H) ; 1,40 (s, 9H) 3,15 (m, 2H).

### 8.4. Trifluoroacétate de(6-aza-spiro[3.4]oct-2-ylméthyl)-carbamate 3-(méthylcarbamoyl)-isoxazol-5-ylméthyle

On procède suivant le mode opératoire décrit dans l'exemple 6 à l'étape 6.6. A partir de 0,31g (0,73 mmole) de 2-{[3-(méthylcarbamoyl)-isoxazol-5-ylmethoxycarbonylamino]-méthyl}-6-aza-spiro[3.4]octane-6-carboxylate de tert-butyle, obtenu à l'étape 8.3. et de 0,62 mL (7,34 mmoles) d'une solution d'acide trifluoroacétique, on obtient 0,32 g de produit sous forme de trifluoroacétate utilisé tel quel dans l'étape 8.5. ci-dessous.

### 8.5. 6-[5-(4-Fluoro-phényl)-pyridin-2-yl]-6-aza-spiro[3.4]oct-2-ylméthylcarbamate de 3-(méthylcarbamoyl)-isoxazol-5-ylméthyle (isomère I)

On procède suivant le mode opératoire décrit dans l'exemple 1 (étape 1.3.). A partir de 0,32 g (0,73 mmole) de trifluoroacétate de (6-aza-spiro[3.4]oct-2-ylméthyl)-carbamate de 3-(méthylcarbamoyl)-isoxazol-5-ylméthyle, décrit dans l'étape 8.4., de 0,16 g (0,88 mmole) de 2-fluoro-5-(4-fluoro-phényl)-pyridine, préparé à l'étape 6.7. et 0,38 g (2,92 mmoles) de *N,N*-diisopropyléthylamine, et après purification par chromatographie sur gel de silice en éluant avec un mélange 98/2/0,2 de dichlorométhane et de méthanol et d'ammoniaque, on obtient ainsi 0,07 g de produit pur sous forme d'un solide blanc.
Pf(°C) : 171-173°C
LC-MS : M+H = 494
RMN ¹H (DMSO) δ (ppm) : 8,70 (large s, 1H) ; 8,40 (s, 1H) ; 7,80 (m, 1H) ; 7,60 (m, 2H) ; 7,50 (m, 1H) ; 7,30 (m, 2H) ; 6,80 (s, 1H) ; 6,50 (d, 1H) ; 5,20 (s, 2H) ; 3,50 (m, 2H) ; 3,35 (m, 2H) ; 3,10 (t, 2H) 2,80 (s, 3H) ; 2,40 (m, 1H) ; 2, 00 ( m, 4H) ; 1, 80 (m, 2H).

### Exemple 9 (Composé N°13)

### 6-[5-(4-Fluoro-phényl)-pyridin-2-yl]-6-aza-spiro[3.4]oct-2-ylméthylcarbamate de 3-(méthylcarbamoyl)-isoxazol-5-ylméthyle (isomère II)

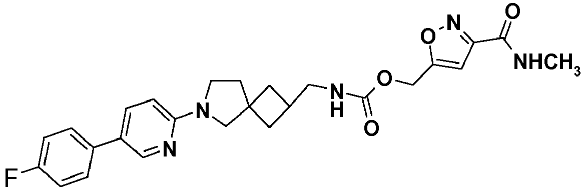

### 9.1. 2-Aminométhyl-6-aza-spiro[3.4]octane-6-carboxylate de tert-butyle : isomère 2b

On procède suivant le mode opératoire décrit dans l'exemple 8 (étape 8.2.). A partir de 0,20 g (0,85 mmole) de 2-cyano-6-aza-spiro[3.4]octane-6-carboxylate de *tert*-butyle, (isomère 1b), décrit dans l'exemple 8 (étape 8.1.) et de Nickel de Raney en quantité catalytique, on obtient 0,22 g de produit sous forme d'une huile jaune utilisé tel quel dans l'étape suivante.
Isomère 2b
LC-MS : M+H = 241
RMN ¹H (DMSO) δ (ppm) : 3,2 (m, 2H) ; 3,1 (m, 2H) ; 2,5 (m, 2H) ; 2,2 (m, 1H) ; 1,9 (m, 4H) ; 1,7 (m, 2H) ; 1,4 (s, 9H).

### 9.2. 2-[(3-(Méthylcarbamoyl)-isoxazol-5-ylméthoxycarbonylamino)-méthyl]-6-aza-spiro[3.4]octane-6-carboxylate de tert-butyle

On procède suivant le mode opératoire décrit dans l'exemple 1 à l'étape 1.1. A partir de 0,24 g (1,00 mmole) de 2-aminométhyl-6-aza-spiro[3.4]octane-6-carboxylate de *tert-*butyle ( isomère 2b), décrit dans l'exemple 9 (étape 9.1.), de 0,321 g (1,00 mmole) de 4-nitro-phényl-carbonate de 3-(méthylcarbamoyl)-isoxazol-5-ylméthyle, obtenu à l'étape 4.1., de 0,258 g (2,00 mmoles) de *N,N*-diisopropyléthylamine et de 0,012g (0,10 mmole) de *N,N*-diméthylaminopyridine, et après purification par chromatographie sur gel de silice en éluant avec un mélange 98/2 de dichlorométhane et de méthanol, on obtient ainsi 0,320 g de produit pur sous forme d'un solide amorphe.
LC-MS : M+H = 423
RMN ¹H (DMSO) δ (ppm) : 8,80 (large s, 1H) ; 7,50 (large s, 1H) ; 6,80 (s, 1H); 5,20 (s, 2H); 3,25 (m, 2H) ; 3,15 (m, 2H) ; 3,10 (m, 2H); 2,80 (s, 3H) ; 2,40 (m, 1H) ; 2,00-1,80 (m, 4H) ; 1,70 (m, 2H) ; 1,40 (s, 9H) 3,15 (m, 2H).

### 9.3. Trifluoroacétate de(6-Aza-spiro[3.4]oct-2-ylméthyl)-carbamate de 3-(méthylcarbamoyl)-isoxazol-5-ylméthyle

On procède suivant le mode opératoire décrit dans l'exemple 6 à l'étape 6.6. A partir de 0,32g (0,76 mmole) de 2-[(3-(méthylcarbamoyl)-isoxazol-5-ylméthoxycarbonylamino)-méthyl]-6-aza-spiro[3.4]octane-6-carboxylate de tert-butyle, obtenu à l'étape 9.2. et de 0,64 mL (7,57 mmoles) d'une solution d'acide trifluoroacétique, on obtient 0,33 g de produit sous forme de trifluoroacétate utilisé tel quel dans l'étape 9.4. ci-dessous.

### 9.4. 6-[5-(4-Fluoro-phényl)-pyridin-2-yl]-6-aza-spiro[3.4]oct-2-ylméthylcarbamate de 3-(méthylcarbamoyl)-isoxazol-5-ylméthyle (isomère II)

On procède suivant le mode opératoire décrit dans l'exemple 1 (étape 1.3.). A partir de 0,33 g (0,76 mmole) de trifluoroacétate de(6-Aza-spiro[3.4]oct-2-ylmethyl)-carbamate de 3-(méthylcarbamoyl)-isoxazol-5-ylméthyle, décrit dans l'étape 9.3., de 0,174 g (0,91 mmole) de 2-fluoro-5-(4-fluoro-phényl)-pyridine, préparé à l'étape 6.7. et 0,393 g (3,04 mmoles) de *N,N*-diisopropyléthylamine, et après purification par chromatographie sur gel de silice en éluant avec un mélange 98/2 de dichlorométhane et de méthanol, on obtient ainsi 0,10 g de produit pur sous forme d'un solide blanc.
Pf(°C) : 180-182°C
LC-MS : M+H = 494
RMN ¹H (DMSO) δ (ppm) : 8,70 (large s, 1H) ; 8,40 (s, 1H) ; 7,80 (m, 1H) ; 7,60 (m, 2H) ; 7,50 (m, 1H) ; 7,30 (m, 2H) ; 6,80 (s, 1H) ; 6,50 (d, 1H) ; 5,20 (s, 2H) ; 3,50 (s, 2H) ; 3,40 (m, 2H) ; 3,10 (t, 2H) ; 2,80 (s, 3H) ; 2,40 (m, 1H) ; 2,10 (t, 2H) ; 1,90 (t, 2H) 1,80 (t, 2H) .

### Exemple 10 (Composé N°8)

### 7-[5-(4-Fluoro-phényl)-pyridin-2-yl]-7-aza-spiro[3.5]non-2-ylcarbamate de 3-(méthylcarbamoyl)-isoxazol-5-ylméthyle

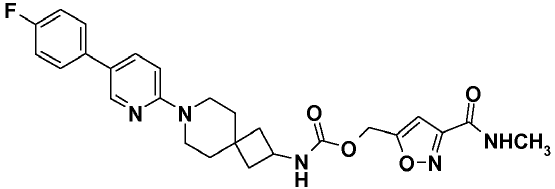

### 10.1. 7-(5-Bromo-pyridin-2-yl)-7-aza-spiro[3.5]nonan-2-ol

Dans un tube scellé, on introduit 0,24 g (1,35 mmole) de 5-bromo-2-fluoro-pyridine, 0,20 g (1,13 mmole) de chlorhydrate de 7-aza-spiro[3.5]nonan-2-ol (JP 2003246780) et 0,51 g (3,94 mmoles) de *N,N*-diisopropyléthylamine dans 3 mL d'acétonitrile. On ajoute 1 mL de DMF puis on chauffe à 95°C pendant 12 heures. On laisse revenir à température ambiante puis on reprend le mélange réactionnel par de l'acétate d'éthyle et de l'eau. On sépare la phase aqueuse puis on lave les phases organiques réunies avec une solution aqueuse saturée en chlorure d'ammonium, on les sèche sur sulfate de sodium et on concentre le filtrat sous pression réduite. Après purification par chromatographie sur gel de silice en éluant avec un mélange 95/5/0,5 de dichlorométhane et de méthanol et d'ammoniaque à 28%, on obtient ainsi 0,138 g de produit pur sous forme d'une gomme incolore.
LC-MS : M+H = 298
RMN ¹H (CDCl3) δ (ppm) : 8,10 (s, 1H) ; 7,50 (d, 1H) ; 6,50 (d, 1H); 4,30 (m, 1H) ; 3,30 (m, 4H); 2,25 (m, 2H); 1,65 (m, 2H) ; 1,50 (m, 4H).

### 10.2. 7-[5-(4-Fluoro-phényl)-pyridin-2-yl]-7-aza-spiro[3.5]nonan-2-ol

On procède suivant le mode opératoire décrit dans l'exemple 2 (étape 2.7.). A partir de 0,138 g (0,46 mmole) de 7-(5-bromo-pyridin-2-yl)-7-aza-spiro[3.5]nonan-2-ol, obtenu à l'étape 10.1., 0,078 g (0,56 mmole) d'acide 4-fluorophénylboronique, 0,454 g (1,39 mmole) de carbonate de cesium en suspension dans 3 mL d'un mélange 9/1 de tétrahydrofurane et d'eau. On ajoute ensuite 0,038 g (0,05 mmole) de PdCl₂dppf.CH₂Cl₂. On obtient ainsi 0,101g de produit pur sous forme de poudre grise.
Pf(°C) : 139-141°C
LC-MS : M+H = 313
RMN ¹H (CDCl3) δ (ppm) : 8,30 (s, 1H) ; 7,60 (d, 1H) ; 7,40 (m, 2H); 7,10 (m, 2H); 6,60 (d, 1H); 4,30 (m, 1H) ; 3,30 (m, 4H) ; 2,30 (m, 2H); 1,65 (m, 2H) ; 1,50 (m, 4H).

### 10.3. Méthanesulfonate de 7-[5-(4-fluoro-phényl)-pyridin-2-yl]-7-aza-spiro[3.5]non-2-yle

On procède suivant le mode opératoire décrit dans l'exemple 6 (étape 6.2.). A une solution de 2,43 g (7,78 mmoles) de 7-[5-(4-fluorophényl)-pyridin-2-yl]-7-aza-spiro[3.5]nonan-2-ol, obtenu à l'étape 10.2., dans 25 mL de dichlorométhane, sont ajoutés 3.25 mL (23,34 mmoles) de triéthylamine puis 0,91 mL (11,67 mmoles) de chlorure de mésyle. On obtient 3,03 g de produit sous forme d'une huile jaune utilisée tel quel dans l'étape suivante.

### 10.4. 2-Azido-7-[5-(4-fluorophényl)-pyridin-2-yl]-7-aza-spiro[3.5]nonane

On procède suivant le mode opératoire décrit dans l'exemple 6 (étape 6.3.). A partir de 7,66 g (19,62 mmoles) de méthanesulfonate de 7-[5-(4-fluoro-phényl)-pyridin-2-yl]-7-aza-spiro[3.5]non-2-yle, préparé à l'étape 10.3., et de 3,83 g (58,85 mmoles) d'azoture de sodium dans 28 mL de *N,N-*diméthylformamide. Après évaporation du solvant, on obtient 6,60 g de produit sous forme d'une huile marron.

### 10.5. 7-[5-(4-Fluorophényl)-pyridin-2-yl]-7-aza-spiro[3.5]non-2-ylamine

On procède suivant le mode opératoire décrit dans l'exemple 6 (étape 6.4.). A partir de 6,60 g (19,56 mmoles) de 2-azido-7-[5-(4-fluorophényl)-pyridin-2-yl]-7-aza-spiro[3.5]nonane, obtenu à l'étape 10.4., dans 28 mL d'éthanol, on ajoute 0,81 g (3,91 mmoles) de catalyseur de Lindlar (PdCaCO₃). On obtient ainsi 3,89 g de produit pur sous forme d'une poudre de couleur jaune.
PF(°C) : 120-122°C
LC-MS : M+H = 312
RMN ¹H (CDCl3) δ(ppm): 8,45 (s, 1H) ; 7,70 (m, 1H) ; 7,50 (m, 2H); 7,20 (m, 2H); 6,80 (d, 1H); 3,70-3,40 (m, 5H) ; 2,35 (m, 4H); 1,80-1,50 (m, 6H).

### 10.6. 7-[5-(4-Fluorophényl)-pyridin-2-yl]-7-aza-spiro[3.5]non-2-ylcarbamate de 3-(méthylcarbamoyl)-isoxazol-5-ylméthyle

On procède suivant le mode opératoire décrit dans l'exemple 1 à l'étape 1.1. A partir de 1,08 g (3,47 mmole) de 7-[5-(4-fluorophényl)-pyridin-2-yl]-7-aza-spiro[3.5]non-2-ylamine, décrit dans l'étape précédente (étape 10.5.), de 1,34 g (4,16 mmole) de 4-nitro-phényl-carbonate de 3-(méthylcarbamoyl)-isoxazol-5-ylméthyle, obtenu à l'étape 4.1., de 1,12 g (8,67 mmoles) de *N,N*-diisopropyléthylamine et de 0,212g (1,73 mmole) de *N,N*-diméthylaminopyridine, et après purification par chromatographie sur gel de silice en éluant avec un mélange 98/2 de dichlorométhane et de méthanol, on obtient ainsi 0,847g de produit pur sous forme d'un solide blanc.
PF(°C) : 219-221°C
LC-MS : M+H = 493
RMN ¹H (DMSO) δ (ppm) : 8,70 (large s, 1H) ; 8,40 (s, 1H) ; 7,90-7,70 (m, 2H); 7,65 (m, 2H); 7,25 (m, 2H); 6,90 (d, 1H); 6, 80 (s, 1H) ; 5,20 0 (s, 2H) ; 4,00 (m, 1H) ; 3,55 (t, 2H) ; 3,45 (t, 2H) ; 2,80 (s, 3H) ; 2,20 (m, 2H) ; 1,75 (m; 2H); 1,65-1,45 (m, 4H).

### Exemple 11 (Composé N°15)

### 2-(6-Fluoro-quinolin-2-yl)-2-aza-spiro[3.3]hept-6-ylcarbamate de 3-(méthylcarbamoyl)-isoxazol-5-ylméthyle

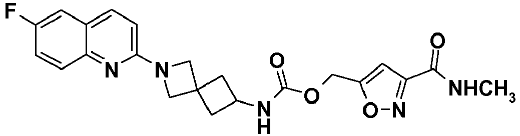

### 11.1. 5,5-Dichloro-6-oxo-2-aza-spiro[3.3]heptane-2-carboxylate de tert-butyle

A un mélange contenant 7,98 g (47,16 moles) de 3-méthylène-azétidine-1-carboxylate de tert-butyle (W02008124085) et 36,48 g (282,94 mmoles) d'amalgame Zinc-Cuivre en suspension dans 200 mL d'éther, sont ajoutés, à 0°C et au goutte à goutte, 28,42 mL (254,65 mmoles) de chlorure de trichloro-acétyle dissout dans 70 mL de diméthoxyéthane. Le milieu réactionnel est agité à température ambiante pendant 12 heures. On verse ensuite le mélange, par petite portion, dans une solution de carbonate de sodium à 0°C. La solution obtenue est filtrée sur célite et rincée abondamment à l'eau et à l'éther. On sépare ensuite la phase aqueuse, on l'extrait plusieurs fois avec de l'éther. Les phases organiques réunies sont séchées sur sulfate de sodium et le filtrat est concentré sous pression réduite. Après évaporation du solvant, le produit obtenu sous forme d'une huile marron est utilisé tel quel dans l'étape suivante.

### 11.2. 6-Oxo-2-aza-spiro[3.3]heptane-2-carboxylate de tert-butyle

A une solution contenant 13,21 g (47,15 mmoles) de 5,5-dichloro-6-oxo-2-aza-spiro[3.3]heptane-2-carboxylate de tert-butyle, obtenue à l'étape précédente, dans 250 mL de méthanol, on ajoute 15,13 g (0,283 mmoles) de chlorure d'ammonium. Le milieu réactionnel est refroidi par un bain de glace/eau et on ajoute 30,83 g (0,471 mmoles) de Zinc. Après agitation à température ambiante pendant 12 heures, on filtre sur célite et on rince au méthanol. On évapore à sec le filtrat. Le résidu obtenu est repris à l'eau et on extrait plusieurs fois avec de l'acétate d'éthyle. Les phases organiques réunies sont séchées sur sulfate de sodium et le filtrat est concentré sous pression réduite. Après évaporation du solvant, on purifie le résidu obtenu par chromatographie sur gel de silice en éluant avec un mélange 90/10 de cyclohexane et d'acétate d'éthyle. On obtient ainsi 1,78 g de produit pur sous forme d'une poudre blanche.
PF(°C) : 117-119°C
LC-MS : M+H = 212
RMN ¹H (DMSO) δ(ppm): 4,05 (s, 4H) ; 3,30 (s, 4H) ; 1,40 (s, 9H) .

### 11.3. 6-Hydroxy-2-aza-spiro[3.3]heptane-2-carboxylate de tert-butyle

On procède suivant le mode opératoire décrit dans l'exemple 6 à l'étape 6.1. A partir de 1,40 g (6,63 mmoles) de 6-oxo-2-aza-spiro[3.3]heptane-2-carboxylate de tert-butyle, obtenu à l'étape 11.2., dissout dans 17 mL de méthanol et de 0,376 g (9,94 mmoles) de borohydrure de sodium. Après évaporation du solvant et cristallisation dans un mélange 70/30 d'éther de pétrole et d'éther diisopropyllique, on obtient 1,18 g du produit attendu sous forme de poudre blanche.
PF(°C) : 131-133°C
LC-MS : M+H = 214
RMN ¹H (DMSO) δ(ppm): 5,00 (s, 1H) ; 3,95 (m, 1H) ; 3,75 (d, 4H) ; 2,40 (m, 2H) ; 1,90 (m, 2H) ; 1,40 (s, 9H).

### 11.4. 6-Méthanesulfonate de 2-aza-spiro[3.3]heptane-2-carboxylate de tert-butyle

On procède suivant le mode opératoire décrit dans l'exemple 6 à l'étape 6.2. A partir de 0,97 g (4,55 mmoles) de 6-hydroxy-2-aza-spiro[3.3]heptane-2-carboxylate de tert-butyle, obtenu à l'étape 11.3. dissout dans 40 mL de dichlorométhane, de 0,70 mL (5,00 mmoles) de triéthylamine et de 0,39 mL (5,00 mmoles) de chlorure de mésyle et après purification sur colonne de gel de silice en éluant avec un gradient de mélange de 90/10 à 70/30 de cyclohexane et d'acétate d'éthyle, on obtient 0,790 g de produit utilisé tel quel dans l'étape suivante.

### 11.5. 6-Azido-2-aza-spiro[3.3]heptane-2-carboxylate de tert-butyle

On procède suivant le mode opératoire décrit dans l'exemple 2 à l'étape 2.1. A partir de 0,780 g (2,68 mmoles) de 6-méthanesulfonate de 2-aza-spiro[3.3]heptane-2-carboxylate de tert-butyle obtenu à l'étape 11.4. et de 0,350 g (5,35 mmoles) de d'azoture de sodium dans 8 mL de *N,N*-diméthylformamide, on obtient 0,63 g de produit utilisé tel quel dans l'étape suivante.

### 11.6. 6-Amino-2-aza-spiro[3.3]heptane-2-carboxylate de tert-butyle

On procède suivant le mode opératoire décrit dans l'exemple 6 (étape 6.4.). A partir de 0,638 g (2,68 mmoles) de 6-azido-2-aza-spiro[3.3]heptane-2-carboxylate de tert-butyle, obtenu à l'étape 11.5., dans 11 mL d'éthanol, on ajoute 0,276 g (1,34 mmole) de catalyseur de Lindlar (PdCaCO₃). Après purification sur colonne de gel de silice en éluant avec un mélange 98/2/0,2 de dichlorométhane , de méthanol et d'ammoniaque à 28%, on obtient 0,330g de produit pur sous forme de poudre blanche.
PF(°C) : 50-53°C
LC-MS : M+H = 213
RMN ¹H (DMSO+ D₂O) δ(ppm): 3,80 (s, 2H) ; 3,70 (s, 2H) ; 3,10 (m, 1H) ; 2,30 (t, 2H) ; 1,75 (t, 2H) ; 1,40 (s, 9H).
11.7. 6-(3-Ethoxycarbonyl-isoxazol-5-ylméthoxycarbonylamino)-2-aza-spiro[3.3]heptane-2-carboxylate de tert-butyle

A une solution de 0,226 g (1,32 mmole) de 5-hydroxyméthyl-isoxazole-3-carboxylate d'éthyle dans 10 mL de dichloroéthane, on ajoute 0,341 g (2,64 mmoles) de N,N-diisopropyléthylamine et 0,265 g (1,32 mmole) de chloroformiate de 4-nitrophényle. On poursuit l'agitation à température ambiante pendant 2 heures puis on ajoute 0,280 g (1,32 mmole) de 6-amino-2-aza-spiro[3.3]heptane-2-carboxylate de tert-butyle, obtenu à l'étape 11.6. en solution dans 4 mL de dichloroéthane. On poursuit l'agitation à température ambiante pendant 4 heures. On ajoute de l'eau au milieu réactionnel, on sépare la phase aqueuse, on l'extrait plusieurs fois avec du dichlorométhane, on lave les phases organiques réunies avec une solution aqueuse de soude (1N) puis avec une solution aqueuse saturée en chlorure d'ammonium. On les sèche sur sulfate de sodium et on concentre le filtrat sous pression réduite. Après évaporation du solvant, on purifie le résidu obtenu par chromatographie sur gel de silice en éluant avec un mélange 98/2 de dichlorométhane et de méthanol.

On obtient ainsi 0,42 g de produit pur sous forme de solide amorphe.
LC-MS : M+H = 410
RMN ¹H (DMSO) δ (ppm) : 7,70 (d, 1H) ; 6.90 (s, 1H) ; 5,20 (s, 2H) ; 4,40 (m, 3H) ; 3,85 (m, 2H) ; 3,75 (s, 2H) ; 2,40 (t, 2H) ; 2,10 (t, 2H) ; 1,40-1.20 (m, 12H).

### 11.8. Trifluoroacétate de 5-(2-aza-spiro[3.3]hept-6-ylcarbamoyloxyméthyl)-isoxazole-3-carboxylate d'éthyle

On procède suivant le mode opératoire décrit dans l'exemple 6 à l'étape 6.6. A partir de 0,42 g (1,03 mmole) de 6-(3-éthoxycarbonyl-isoxazol-5-ylméthoxycarbonylamino)-2-aza-spiro[3.3]heptane-2-carboxylate de tert-butyle, obtenu à l'étape 11.7. et de 0,86 mL (10,26 mmoles) d'une solution d'acide trifluoroacétique, on obtient 0,43 g de produit sous forme de trifluoroacétate utilisé tel quel dans l'étape 11.9. ci-dessous.

### 11.9. 5-[2-(6-Fluoro-quinolin-2-yl)-2-aza-spiro[3.3]hept-6-ylcarbamoyloxyméthyl]-isoxazole-3-carboxylate d'éthyle

On procède suivant le mode opératoire décrit dans l'exemple 2 (étape 2.6.). A partir de 0,43 g (1,03 mmole) de trifluoroacétate de 5-(2-aza-spiro[3.3]hept-6-ylcarbamoyloxyméthyl)-isoxazole-3-carboxylate d'éthyle, obtenu à l'étape 11.8., de 0,187 g (1,03 mmole) de 2-chloro-6-fluoro-quinoline et de 0,399 g (3,09 mmoles) de *N,N-*diisopropyléthylamine et après chromatographie sur gel de silice en éluant avec un mélange 98/2 de dichlorométhane et de méthanol. On obtient ainsi 0,15 g de produit pur sous forme de solide beige.
LC-MS : M+H = 455
PF(°C) : 107-109°C
RMN ¹H (DMSO) δ (ppm) : 8,05 (d, 1H) ; 7,80 (d, 1H) ; 7,60 (m, 1H); 7,50 (m, 1H); 7,40 (m, 1H); 6,90 (s, 1H); 6,70 (d, 1H) ; 5,25 (s, 2H); 4,40 (q, 2H); 4,10 (s, 2H) ; 4,00 (s, 2H) ; 3,90 (m, 1H) ; 2,50 (t, 2H) ; 2,20 (t, 2H) ; 1,35 (t, 3H).

### 11.10. 2-(6-Fluoro-quinolin-2-yl)-2-aza-spiro[3.3]hept-6-ylcarbamate de 3-(méthylcarbamoyl)-isoxazol-5-ylméthyle

Dans un tube scellé, une solution de 0,130 g (0,28 mmole) de 5-[2-(6-fluoro-quinolin-2-yl)-2-aza-spiro[3.3]hept-6-ylcarbamoyloxyméthyl]-isoxazole-3-carboxylate d'éthyle, préparé à l'étape 11.9., dans 4,13 mL (28,01 mmoles) d'une solution de méthylamine (8M) dans l'éthanol, est agitée à température ambiante pendant 5 heures. On évapore à sec. Le résidu obtenu cristallisé dans l'éther à chaud. Le précipité ainsi formé est filtré puis rincé abondamment à l'éther. Après séchage sous vide à environ 60°C, on obtient 0,05 g de produit pur sous forme de poudre blanche.
PF(°C) : 167-169°C
RMN ¹H (DMSO) δ (ppm) : 8,70 (large s, 1H) ; 8,05 (d, 1H) ; 7,80 (d, 1H) ; 7,60 (m, 1H); 7,50 (m, 1H); 7,40 (m, 1H); 6,80 (s, 1H); 6,75 (d, 1H) ; 5,25 (s, 2H); 4,15 (s, 2H) ; 4,05 (s, 2H) ; 3,95 (m, 1H) ; 2,80 (s, 3H) ; 2,50 (t, 2H) ; 2,20 (t, 2H).

### Exemple 12 (Composé N°17)

### {6-[5-(4-Fluoro-phényl)-pyridin-2-yl]-6-aza-spiro[3.4]oct-2-yl}-carbamate de 3-(méthylcarbamoyl)-isoxazol-5-ylméthyle (isomère I)

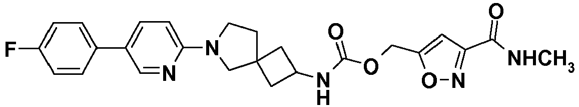

### 12.1 2-méthanesulfonyloxy-6-aza-spiro[3.4]octane-6-carboxylate de tert-butyle (isomère 1 et 1').

Les isomères 1 et 1' du 2-méthanesulfonyloxy-6-aza-spiro[3.4]octane-6-carboxylate de *tert*-butyle, préparé selon la méthode décrite dans l'exemple 6 (étape 6.2.), sont séparés par colonne chromatographie sur gel de silice en éluant avec un mélange cyclohexane/ acétatate d'éthyle de 100/0 à 80/20. On obtient ainsi 1,69 g de l'isomère 1 sous forme de solide blanc et 1,62 g de l'isomère 1' sous forme de solide blanc.
Isomère 1
   PF (°C) : 76-78°C
   LC-MS : M+H = 306
   Rf = 0.35 (Acétate d'éthyle / cyclohexane : 50 / 50)
   RMN ¹H (DMSO) δ (ppm) : 5,08 (m, 1H); 3,28 (m, 2H); 3,20 (m, 2H); 3,15 (s, 3H); 2,42 (m, 2H); 2,22 (m, 2H); 1,85 (m, 2H); 1,40 (s, 9H).
Isomère 1'
   PF (°C) : 79-82°C
   LC-MS : M+H = 306
   Rf = 0.29 (Acétate d'éthyle / cyclohexane : 50 / 50)
   RMN ¹H (DMSO) δ (ppm) : 5,00 (m, 1H); 3,22 (m, 4H), 3.12 (s, 3H), 2,36 (m, 2H); 2,22 (m, 2H), 1,80 (m, 2H); 1,35 (s, 9H).

### 12.2 2-Azido-6-aza-spiro[3.4]octane-6-carboxylate de tert-butyle (isomère 2')

On procède suivant le mode opératoire décrit dans l'exemple 6 (étape 6.3.). A partir de 1,62 g (5,30 mmoles) de 2-méthanesulfonyloxy-6-aza-spiro[3.4]octane-6-carboxylate (isomère 1'), décrit dans l'exemple 12 (étape 12.1.) et de 0,68 g (10,61 mmoles) d'azide de sodium, on obtient le produit sous forme d'une huile jaune utilisé tel quel dans l'étape suivante.

### 12.3 2-Amino-6-aza-spiro[3.4]octane-6-carboxylate de tert-butyle (isomère 3')

On procède suivant le mode opératoire décrit dans l'exemple 6 (étape 6.4.). A partir de 1,33 g (5,30 mmoles) de 2-azido-6-aza-spiro[3.4]octane-6-carboxylate de tert-butyle (isomère 2') et de 0,54 g (2,65 mmoles) de catalyseur de Lindlar (PdCaCO₃), on obtient 0,70 g de produit sous forme d'une huile.
LC-MS : M+H = 227
RMN ¹H (DMSO +D₂O) δ (ppm) : 3,24 (m, 1H) ; 3,15 (m, 4H); 2,12 (m, 2H); 1,74 (m, 2H); 1,60 (m, 2H); 1,36 (s, 9H).

### 12.4 2-(3-Carbamoyl-isoxazol-5-ylméthoxycarbonylamino)-6-aza-spiro[3.4]octane-6-carboxylate de tert-butyle (isomère 4')

On procède suivant le mode opératoire décrit dans l'exemple 4 (étape 4.2). A partir de 0,70 g (3,09 mmoles) de 2-amino-6-aza-spiro[3.4]octane-6-carboxylate de *tert*-butyle (isomère 3') et 1,04 g (3,09 mmoles) de 5-(4-nitro-phénoxycarbonyloxyméthyl)-isoxazole-3-carboxylate d'éthyle, on obtient 1,10 g de produit sous forme de gomme.
LC-MS : M+H = 424
RMN ¹H (DMSO) δ (ppm) : 7,80 (dl, 1H); 6,92 (s, 1H); 5,21 (s, 2H); 4,35 (q, 2H); 3,98 (m, 1H); 3,21 (m, 2H); 3,15 (m, 2H); 2,21 (m, 2H); 1,90 (m, 2H); 1,75 (m, 2H); 1,40 (s, 9H); 1,30 (t, 3H).

### 12.5 Trifluoroacétate de 5-(6-aza-spiro[3.4]oct-2-ylcarbamoyloxyméthyl)-isoxazole-3-carboxylate d'éthyle (isomère 5')

On procède suivant le mode opératoire décrit dans l'exemple 6 (étape 6.6.). A partir de 1,10 g (2,60 mmoles) de 2-(3-carbamoyl-isoxazol-5-ylméthoxycarbonylamino)-6-aza-spiro[3.4]octane-6-carboxylate de tert-butyle (isomère 4') et 2,19 mL (25,98 mmoles) d'acide trifluoroacétique, on obtient le produit utilisé tel quel dans l'étape suivante.

### 12.6 5-[6-(5-Bromo-pyridin-2-yl)-6-aza-spiro[3.4]oct-2-ylcarbamoyloxyméthyl]-isoxazole-3-carboxylate d'éthyle (isomère 6')

On procède suivant le mode opératoire décrit dans l'exemple 2 (étape 2.6.). A partir de 0,43 g (1,03 mmole) de trifluoroacétate de 5-(6-aza-spiro[3.4]oct-2-ylcarbamoyloxyméthyl)-isoxazole-3-carboxylate d'éthyle (isomère 5'), obtenu à l'étape 12.5., de 0,45 g (2,60 mmoles) de 2-fluoro-5-bromo-pyridine et après chromatographie sur gel de silice en éluant avec un mélange 97/3 de dichlorométhane et de méthanol, on obtient 0,68 g de produit pur sous forme d'huile.

### 12.7 5-{6-[5-(4-Fluoro-phényl)-pyridin-2-yl]-6-aza-spiro[3.4]oct-2-ylcarbamoyloxyméthyl}-isoxazole-3-carboxylate d'éthyle (isomère 7')

On procède suivant le mode opératoire décrit dans l'exemple 2 (étape 2.7.). A partir de 0,68 g (1,42 mmole) de 5-[6-(5-bromo-pyridin-2-yl)-6-aza-spiro[3.4]oct-2-ylcarbamoyloxy-méthyl]-isoxazole-3-carboxylate d'éthyle(isomère 6') et 0,23 g (1,70 mmole) d'acide 4-fluorophénylboronique, 1,38 g (4,26 mmoles) de carbonate de cesium et 0,11 g (0,14 mmole) de PdCl₂dppf.CH₂Cl₂, on obtient 0,32 g d'un solide blanc.
PF (°C) = 164-166
LC-MS : M+H = 495
RMN ¹H (DMSO) δ (ppm) : 8,40 (s, 1H) ; 7,80 (m, 2H) ; 7,62 (m, 2H) ; 7,22 (t, 2H); 6,89 (s, 1H) ; 6,50 (d, 1H) ; 5,21 (s, 2H) ; 4,35 (q, 2H); 4,05 (m, 1H) ; 3,50 (s, 2H); 3,40 (m, 2H) ; 2,30 (m, 2H) ; 2,00 (m, 4H); 1.30 (t, 3H).

### 12.8 {6-[5-(4-Fluoro-phényl)-pyridin-2-yl]-6-aza-spiro[3.4]oct-2-yl}-carbamate de 3-(méthylcarbamoyl)-isoxazol-5-ylméthyle (isomère I)

On procède suivant le mode opératoire décrit dans l'exemple 6 (étape 6.9.). A partir de 0,3g (0,61 mmole) de 5-{6-[5-(4-fluoro-phényl)-pyridin-2-yl]-6-aza-spiro[3.4]oct-2-ylcarbamoyloxyméthyl}-isoxazole-3-carboxylate d'éthyle (isomère 7') et 15 mL d'une solution de méthyle amine dans le tétrahydrofuranne (1M) à température ambiante. On obtient 0,21 g de produit sous forme d'une solide blanc.
LC-MS : M+H = 480
PF(°C) : 203-205
RMN ¹H (DMSO) δ (ppm) : 8,70 (large s, 1H), 8,40 (s, 1H) ; 7,81 (m, 2H) ; 7,62 (m, 2H) ; 7,25 (t, 2H) ; 6,78 (s, 1H) ; 6,50 (d, 1H) ; 5,20 (s, 2H) ; 4,08 (m, 1H) ; 3,50 (s, 2H), 3,40 (m, 2H) ; 2.78 (s, 3H), 2,30 (m, 2H) ; 1.99 (m, 4H).

### Exemple 13 (Composé N°18)

### {6-[5-(4-Fluoro-phényl)-pyridin-2-yl]-6-aza-spiro[3.4]oct-2-yl}-carbamate de 3-(méthylcarbamoyl)-isoxazol-5-ylméthyle (isomère II)

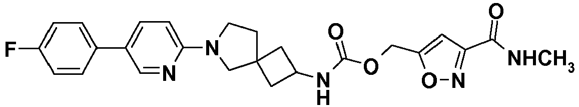

### 13.1 2-Azido-6-aza-spiro[3.4]octane-6-carboxylate de tert-butyle (isomère 2)

On procède suivant le mode opératoire décrit dans l'exemple 6 (étape 6.3.). A partir de 2,49 g (8,15 mmoles) de 2-méthanesulfonyloxy-6-aza-spiro[3.4]octane-6-carboxylate (isomère 1), décrit dans l'exemple 12 (étape 12.1.) et de 1,07 g (16,31 mmoles) d'azide de sodium, on obtient le produit utilisé tel quel dans l'étape suivante.

### 13.2 2-Amino-6-aza-spiro[3.4]octane-6-carboxylate de tert-butyle (isomère 3)

On procède suivant le mode opératoire décrit dans l'exemple 6 (étape 6.4.). A partir de 2,05 g (8,15 mmoles) de 2-azido-6-aza-spiro[3.4]octane-6-carboxylate de *tert*-butyle (isomère 2) et de 0,84 g (4,08 mmoles) de catalyseur de Lindlar (PdCaCO₃), on obtient 1,11 g de produit sous forme d'une huile jaune.
LC-MS : M+H = 227
RMN ¹H (DMSO +D₂O) δ (ppm) : 3,25 (m, 1H) ; 3,20 (m, 2H); 3.10 (m, 2H); 2,10 (m, 2H); 1,75 (m, 2H); 1,65 (m, 2H); 1,40 (s, 9H).

### 13.3 2-(3-Carbamoyl-isoxazol-5-ylméthoxycarbonylamino)-6-aza-spiro[3.4]octane-6-carboxylate de tert-butyle (isomère 4)

On procède suivant le mode opératoire décrit dans l'exemple 4 (étape 4.2). A partir de 1,11 g (4,90 mmoles) de 2-amino-6-aza-spiro[3.4]octane-6-carboxylate de *tert*-butyle (isomère 3) et 1,64 g (4,90 mmoles) de 5-(4-nitro-phénoxycarbonyloxyméthyl)-isoxazole-3-carboxylate d'éthyle, on obtient 1,65 g de produit sous forme de gomme.
LC-MS : M+H = 424
RMN ¹H (DMSO) δ (ppm) : 7,75 (large t, 1H) ; 6,90 (s, 1H) ; 5,20 (s, 2H); 4,35 (q, 2H); 3,99 (m, 1H); 3,21 (m, 2H); 3,11 (m, 2H); 2,19 (m, 2H); 1,92 (m, 2H); 1,81 (m, 2H); 1,41 (s, 9H); 1,32 (t, 3H).

### 13.4 Trifluoroacétate de 5-(6-aza-spiro[3.4]oct-2-ylcarbamoyloxyméthyl)-isoxazole-3-carboxylate d'éthyle

### (isomère 5)

On procède suivant le mode opératoire décrit dans l'exemple 6 (étape 6.6.). A partir de 1,65 g (3,90 mmoles) de 2-(3-carbamoyl-isoxazol-5-ylméthoxycarbonylamino)-6-aza-spiro[3.4]octane-6-carboxylate de *tert*-butyle (isomère 4) et 3,28 mL (38,96 mmoles) d'acide trifluoroacétique, on obtient le produit utilisé tel quel dans l'étape suivante.

### 13.5 5-[6-(5-Bromo-pyridin-2-yl)-6-aza-spiro[3.4]oct-2-ylcarbamoyloxyméthyl]-isoxazole-3-carboxylate d'éthyle (isomère 6)

On procède suivant le mode opératoire décrit dans l'exemple 2 (étape 2.6.). A partir de 1,7 g (3,90 mmole) de trifluoroacétate de 5-(6-aza-spiro[3.4]oct-2-ylcarbamoyloxyméthyl)-isoxazole-3-carboxylate d'éthyle (isomère 5), de 0,68 g (3,90 mmole) de 2-fluoro-5-bromo-pyridine et après chromatographie sur gel de silice en éluant avec un mélange 98/2 de dichlorométhane et de méthanol. On obtient ainsi 1,00 g de produit pur sous forme de gomme.
LC-MS : M+H = 479
RMN ¹H (DMSO) δ (ppm) : 8,11 (s, 1H); 7,80 (large d, 1H); 7,60 (d, 1H); 6,90 (s, 1H); 6,40 (d, 1H), 5,21 (s, 2H); 4,39 (q, 2H); 4.01 (m, 1H); 3,39 (m, 2H); 3,31 (m, 2H); 2,25 (m, 2H); 2,01 (m, 4H); 1,30 (t, 3H).

### 13.6 5-{6-[5-(4-Fluoro-phényl)-pyridin-2-yl]-6-aza-spiro[3.4]oct-2-ylcarbamoyloxyméthyl}-isoxazole-3-carboxylate d'éthyle (isomère 7)

On procède suivant le mode opératoire décrit dans l'exemple 2 (étape 2.7.). A partir de 1,00 g (2,09 mmoles) de 5-[6-(5-bromo-pyridin-2-yl)-6-aza-spiro[3.4]oct-2-ylcarbamoyloxy-méthyl]-isoxazole-3-carboxylate d'éthyle(isomère 6) et 0,29 g (2,09 mmoles) d'acide 4-fluorophénylboronique, 2,03 g (6,26 mmoles) de carbonate de cesium et 0,17 g (0,21 mmole) de PdCl₂dppf.CH₂Cl₂, on obtient 0,50 g d'une gomme après chromatographie sur gel de silice en éluant avec un mélange 90/10 de cyclohexane et d'acétate d'éthyle.
LC-MS : M+H = 495
RMN ¹H (DMSO) δ (ppm) : 8,39 (s, 1H) ; 7,80 (m, 2H) ; 7,60 (m, 2H) ; 7,22 (t, 2H) ; 6,90 (s, 1H) ; 6,50 (d, 1H) ; 5,20 (s, 2H) ; 4,38 (q, 2H); 4,06 (m, 1H) ; 3,45 (m, 2H); 3,38 (m, 2H) ; 2,28 (m, 2H) ; 2,00 (m, 4H); 1,32 (t, 3H).

### 13.7 {6-[5-(4-Fluoro-phényl)-pyridin-2-yl]-6-aza-spiro[3.4]oct-2-yl}-carbamate de 3-(méthylcarbamoyl)-isoxazol-5-ylméthyle (isomère II)

On procède suivant le mode opératoire décrit dans l'exemple 6 (étape 6.9.). A partir de 0,50g (1,01 mmole) de 5-{6-[5-(4-fluoro-phényl)-pyridin-2-yl]-6-aza-spiro[3.4]oct-2-ylcarbamoyloxyméthyl}-isoxazole-3-carboxylate d'éthyle (isomère 7) et 25 mL d'une solution de méthyle amine dans le tétrahydrofuranne (1M) à température ambiante. On obtient 0,32 g de produit sous forme d'un solide blanc.
PF(°C) : 194-196
LC-MS : M+H = 480
**RMN ¹H(DMSO)δ(ppm)**:8,70 (large s, 1H), 8,38 (s, 1H) ; 7,80 (m, 2H) ; 7,60 (m, 2H) ; 7,23 (t, 2H) ; 6,78 (s, 1H) ; 6,48 (d, 1H) ; 5,18 (s, 2H) ; 4,08 (m, 1H) ; 3,45 (m, 2H), 3,40 (m, 2H) ; 2.78 (d, 3H), 2,25 (m, 2H) ; 2.00 (m, 4H).

### Exemple 14 (Composé N°19)

### [7-(6-Chloro-quinolin-2-yl)-7-aza-spiro[3.5]non-2-yl]-carbamate de 3-(méthylcarbamoyl)-isoxazol-5-ylméthyle

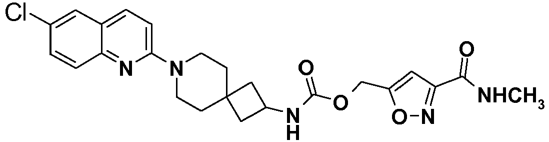

### 14.1 2-Hydroxyimino-7-aza-spiro[3.5]nonane-7-carboxylate de tert-butyle

A une solution de 1,00 g (4,18 mmoles) de 2-Oxo-7-aza-spiro[3.5]nonane-7-carboxylate de tert-butyle (US6,498,159) dans 60 mL d'éthanol, on ajoute 0,58 g (8,36 mmoles) de chlorhydrate d'hydroxylamine et 1,15 g (8,36 mmoles) de carbonate de potassium puis on laisse agiter à température ambiante pendant 12 heures. Après évaporation du solvant sous pression réduite, on reprend le résidu par du dichlorométhane et de l'eau, on sépare la phase aqueuse, on l'extrait deux fois avec du dichlorométhane et on sèche les phases organiques réunies sur sulfate de sodium. Après évaporation du solvant, on purifie le résidu obtenu par chromatographie sur gel de silice en éluant avec un mélange 99/1/0,1 de dichlorométhane, de méthanol et d'ammoniaque à 28%. On obtient ainsi 0,831 g de produit pur sous forme de poudre blanche.
LC-MS : M+H = 255
PF(°C) : 117-119
RMN ¹H (CDCl3) δ (ppm) : 3,30 (m, 4H) ; 2,60 (d, 4H) ; 1,55 (m, 4H); 1,40 (s, 9H).

### 14.2 2-Amino-7-aza-spiro[3.5]nonane-7-carboxylate de tert-butyle

A une solution de 0,50 g (1,97 mmole) de 2-Hydroxyimino-7-aza-spiro[3.5]nonane-7-carboxylate de tert-butyle obtenu à l'étape précédente, dans 32 mL d'une solution d'ammoniaque 7N dans le méthanol. On ajoute ensuite 0,11 g (1,97 mmole) de Nickel de Raney. Le milieu réactionnel est placé dans un appareil de Parr sous atmosphère d'hydrogène (20 psi) à température ambiante pendant 2 heures 30 minutes. On filtre sur büchner puis on concentre le filtrat sous pression réduite. On reprend le résidu par du dichlorométhane et de l'eau, on sépare la phase aqueuse, on l'extrait deux fois avec du dichlorométhane, on lave les phases organiques réunies avec une solution aqueuse saturée en chlorure d'ammonium puis on les sèche sur sulfate de sodium. Après évaporation du solvant, on purifie le résidu obtenu par chromatographie sur gel de silice en éluant avec un mélange 90/10/1 de dichlorométhane, de méthanol et d'ammoniaque à 28%. On obtient ainsi 0,39 g de produit pur sous forme d'une huile incolore.
LC-MS : M+H = 241
RMN ¹H (DMSO) δ(ppm) : 3,40 (m, 1H) ; 3,30-3,10 (m, 4H) ; 2,25-2,15 (m, 2H); 1.70 (large s, 2H) ; 1,50-1,35 (m, 6H); 1,30 (m, 9H).

### 14.3 2-Ethoxycarbonylamino-7-aza-spiro[3.5]nonane-7-carboxylate de tert-butyle

A une solution contenant 10,00 g (41,61 mmoles) de 2-Amino-7-aza-spiro[3.5]nonane-7-carboxylate de tert-butyle obtenu à l'étape précédente, 13,44 g (104,02 mmoles) de *N,N-*diisopropyléthylamine et 0,51 g (4,16 mmoles) de *N,N-*diméthylaminopyridine dans 300 mL de 1,2-dichloroéthane, refroidie à environ 0°C, on ajoute au goutte à goutte 4,96 g (45,77 mmoles) de chloroformiate d'éthyle. On poursuit l'agitation à 0°C pendant 1 heure puis à température ambiante pendant 12 heures. On ajoute de l'eau au milieu réactionnel, on sépare la phase aqueuse, on l'extrait plusieurs fois avec du dichlorométhane, on lave les phases organiques réunies avec une solution aqueuse saturée en chlorure d'ammonium, on les sèche sur sulfate de sodium et on concentre le filtrat sous pression réduite. Après évaporation du solvant, on purifie le résidu obtenu par chromatographie sur gel de silice en éluant avec un mélange 99/1/0,1 de dichlorométhane, de méthanol et d'ammoniaque à 28%. On obtient ainsi 8,728 g de produit pur sous forme d'une huile de couleur marron.
LC-MS : M+H = 313
RMN ¹H (CDC13) δ (ppm) : 4,80 (large s, 1H) ; 4,10 (m, 3H) ; 3,30 (m, 4H); 2,30 (m, 2H) ; 1,60 (m, 6H) ; 1,50 (s, 9H) ; 1,25 (t, 3H).

### 14.4 Chlorhydrate de (7-Aza-spiro[3.5]non-2-yl)-carbamate d'éthyle

On procède suivant le mode opératoire décrit dans l'exemple 2 (étape 2.5.). A partir de 8,63g (27,62 mmoles) de 2-éthoxycarbonylamino-7-aza-spiro[3.5]nonane-7-carboxylate de tert-butyle obtenu à l'étape précédente et de 27,62 mL (110,50 mmoles) d'une solution d'acide chlorhydrique 4N dans le dioxane et après filtration sur fritté et lavage à l'éther , on obtient 5,18g de produit sous forme de chlorhydrate.
LC-MS : M+H = 249
PF(°C) : 238-240
RMN ¹H (DMSO) δ (ppm) : 8,80 (large s, 1H) ; 7,40 (d, 1H) ; 3,90 (m, 3H) ; 2,80 (m, 4H); 2,10 (m, 2H) ; 1,60 (m, 6H) ; 1,25 (t, 3H).

### 14.5 [7-(6-Chloro-quinolin-2-yl)-7-aza-spiro[3.5]non-2-yl]-carbamate d'éthyle

Dans un tube scellé, on introduit 0,166 g (0,78 mmole) de (7-Aza-spiro[3.5]non-2-yl)-carbamate d'éthyle obtenu à l'étape précédente et utilisé sous forme de base, 0,155 g (0,78 mmole) de 2-chloro-6-chloro-quinoline et 0,113 g (0,82 mmole) de carbonate de potassium dans 2 mL de DMSO. On chauffe ensuite à 130°C pendant 12 heures. On laisse revenir à température ambiante puis on reprend le mélange réactionnel par du dichlorométhane et de l'eau. On sépare la phase aqueuse, on l'extrait deux fois avec du dichlorométhane, on lave les phases organiques réunies avec une solution aqueuse saturée en chlorure d'ammonium, on les sèche sur sulfate de sodium et on concentre le filtrat sous pression réduite. Après évaporation du solvant, on purifie le résidu obtenu par chromatographie sur gel de silice en éluant avec un mélange 98/2/0,2 de dichlorométhane, de méthanol et d'ammoniaque à 28%. On obtient ainsi 0,151 g de produit pur sous forme de poudre.
LC-MS : M+H = 374
PF(°C) : 137-139
RMN ¹H (CDC13) δ (ppm) : 7,80 (d, 1H) ; 7,70 (m, 1H) ; 7,60 (m, 1H) ; 7,50 (m, 1H) ; 7,10 (d, 1H) ; 4,80 (large s, 1H) ; 4,20 (m, 3H) ; 3,70 (m, 4H); 2,50 (m, 2H) ; 1,90-1,60 (m, 6H) ; 1,30 (t, 3H).

### 14.6 7-(6-Chloro-quinolin-2-yl)-7-aza-spiro[3.5]non-2-ylamine

A une solution de 0,242 g (0,65 mmole) de [7-(6-Chloro-quinolin-2-yl)-7-aza-spiro[3.5]non-2-yl]-carbamate d'éthyle, obtenu à l'étape 12.5. dans 3,25 mL de Ethanol/Eau (1/1), on ajoute, à température ambiante, 0,726 g (12,95 mmoles) d'hydroxyde de potassium. On chauffe ensuite à 110°C pendant 12 heures. On ajoute 0,363 g (6,47 mmoles) d'hydroxyde de potassium et on laisse agiter 3 heures. On laisse revenir à température ambiante puis on concentre sous pression réduite. On reprend le résidu par du dichlorométhane et une solution d'acide chlorhydrique 1N. On lave la phase aqueuse acide avec du dichlorométhane puis on basifie avec une solution aqueuse d'hydroxyde de sodium 1N que l'on extrait plusieurs fois avec du dichlorométhane. On sèche ensuite les phases organiques réunies sur sulfate de sodium et on concentre le filtrat sous pression réduite. On obtient ainsi 0,188g de produit attendu sous forme d'huile.
LC-MS : M+H = 302
RMN ¹H (DMSO) δ (ppm) : 8,00 (d, 1H) ; 7,80 (d, 1H) ; 7,50 (m, 2H) ; 7,30 (d, 1H) ; 3,60 (m, 4H) ; 3,30 (m, 2H) ; 2,10 (m, 2H); 1,50-1,30 (m, 6H).

### 14.7 [7-(6-Chloro-quinolin-2-yl)-7-aza-spiro[3.5]non-2-yl]-carbamate de 3-(méthylcarbamoyl)-isoxazol-5-ylméthyle

On procède suivant le mode opératoire décrit dans l'exemple 1 à l'étape 1.1. A partir de 0,181 g (0,60 mmole) de 7-(6-chloro-quinolin-2-yl)-7-aza-spiro[3.5]non-2-ylamine, décrit dans l'étape précédente (étape 12.6.), de 0,231 g (0,72 mmole) de 4-nitro-phényl-carbonate de 3-méthylcarbamoyl-isoxazol-5-ylméthyle, obtenu à l'étape 4.1., de 0,194 g (1,50 mmoles) de N,N-diisopropyléthylamine et de 0,037g (0,30 mmole) de *N,N-*diméthylaminopyridine dans 6mL de 1-2 dichloroéthane et après cristallisation dans l'éther, le produit est filtré sur fritté, rincé à l'éther et séché sous vide à environ 70°C. On obtient ainsi 0,220g de produit pur sous forme de poudre blanche.
LC-MS : M+H = 484
PF(°C) : 194-196
RMN ¹H (DMSO) δ (ppm) : 8,70 (large s, 1H) ; 8,00 (d, 1H) ; 7,80 (m, 2H) ; 7,50 (m, 2H); 7,30 (d, 1H) ; 6,80 (s, 1H); 5,20 (s, 2H) ; 4,10 (m, 1H) ; 3,75-3,55 (m, 4H) ; 2,80 (s, 3H) ; 2,20 (m, 2H) ; 1,70 (m, 2H) ; 1,70-1,50 (m, 4H).

### Exemple 15 (Composé N°31)

### [7-(5-Isobutyl-pyridin-2-yl)-7-aza-spiro[3.5]non-2-yl]-carbamate de 3-carbamoyl-isoxazol-5-ylméthyle

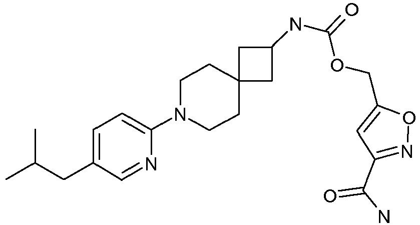

### 15.1. [7-(5-Bromo-pyridin-2-yl)-7-aza-spiro[3.5]non-2-yl]-carbamate d'éthyle

Dans un tube scellé, on introduit 0,60 g (2,41 mmoles) de chlorhydrate de (7-aza-spiro[3.5]non-2-yl)-carbamate d'éthyle, obtenu à l'étape 14.4. (exemple 14), 0,57 g (2,41 mmoles) 2,5-dibromo-pyridine et 0,70 g (5,07 mmoles) de carbonate de potassium dans 2 mL de diméthylsulfoxyde. On chauffe ensuite à 130°C pendant 15 heures. On laisse revenir à température ambiante puis on reprend le milieu réactionnel avec une solution saturée de chlorure de sodium puis on l'extrait avec du dichlorométhane, on sèche les phases organiques réunies sur sulfate de sodium. Après évaporation du solvant, on purifie le résidu obtenu par chromatographie sur plaques préparatives en éluant avec un mélange de 100/0/0 à 99/1/0,1 de dichlorométhane, de méthanol et d'ammoniaque à 28%.
On obtient ainsi 0,75 g de produit pur sous forme de poudre. PF(°C) : 113-115°C

### 15.2 {7-[5-(2-Méthyl-propényl)-pyridin-2-yl]-7-aza-spiro[3.5]non-2-yl}-carbamate d'éthyle

On procède suivant la méthode décrite dans l'exemple 2 (étape 2.7.). A partir de 0,50 g (1,36 mmoles) de [7-(5-bromo-pyridin-2-yl)-7-aza-spiro[3.5]non-2-yl]-carbamate d'éthyle, préparé à l'étape précédente, de 0,33 mL (1,63 mmoles) de 2-méthyl-1-propénylboronate de pinacol (commercial), de 1,33 g (4,07 mmoles) de carbonate de césium, en suspension dans 9 mL d'un mélange 9/1 de tétrahydrofurane et d'eau, de 0,11 g (0,14 mmole) de PdCl₂dppf.CH₂Cl₂ et après purification sur colonne de gel de silice en éluant avec un mélange de 100/0/0 à 99/1/0,1 de dichlorométhane, de méthanol et d'ammoniaque 28%, on obtient 0,39 g de produit attendu sous forme de cire.

### 15.3 [7-(5-Isobutyl-pyridin-2-yl)-7-aza-spiro[3.5]non-2-yl]-carbamate d'éthyle

A une solution de 0,37 g (1,08 mmole) de {7-[5-(2-méthyl-propényl)-pyridin-2-yl]-7-aza-spiro[3.5]non-2-yl}-carbamate d'éthyle obtenu à l'étape précédente, dans 15 mL de méthanol, on ajoute 0,05 g (0,45 mmole) de Palladium sur charbon. Le milieu réactionnel est placé dans un appareil de Parr sous atmosphère d'hydrogène (10 psi) à température ambiante pendant 2 heures. On filtre sur büchner puis on concentre le filtrat sous pression réduite. On obtient ainsi 0,37 g de produit attendu sous forme d'une cire jaune.

### 15.4 7-(5-Isobutyl-pyridin-2-yl)-7-aza-spiro[3.5]non-2-ylamine

A une solution de 0,37 g (1,08 mmole) de [7-(5-isobutyl-pyridin-2-yl)-7-aza-spiro[3.5]non-2-yl]-carbamate d'éthyle, dans 5 mL de éthanol/eau (1/1), on ajoute, à température ambiante, 1,21 g (21,65 mmoles) d'hydroxyde de potassium. On chauffe ensuite à 110°C pendant 15 heures. On laisse revenir à température ambiante puis on concentre sous pression réduite. On reprend le milieu réactionnel avec une solution saturée de chlorure de sodium et du dichlorométhane, on sépare la phase aqueuse, on l'extrait au dichlorométhane, on sèche les phases organiques réunies sur sulfate de sodium. Après évaporation sous pression réduite et purification sur colonne de gel de silice en éluant avec un mélange de 100/0/0 à 96/4/0,4 de dichlorométhane, de méthanol et d'ammoniaque 28%, on obtient 0,27 g de produit attendu sous forme de cire.
LC-MS : M+H = 274
RMN ¹H (CDCl₃) δ (ppm): 8,00 (s, 1H); 7,30 (d, 1H); 6,60 (d, 1H); 3,50 (m, 3H); 3,40 (m, 2H); 2,30 (m, 4H); 1,80 (m, 1H); 1,70 (m, 4H); 1,50 (m, 4H); 0.90 (d, 6H).

### 15.5 4-Nitro-phényl-carbonate de 3-carbamoyl-isoxazol-5-ylméthyle

A une solution de 2,00 g (14,07 mmoles) de 3-carbamoyl-isoxazol-5-ylméthanol, de 1,71 mL (21,11 mmoles) de pyridine et de 0,17 g (1,41 mmoles) de *N,N*-diméthylaminopyridine dans 15 mL de dichlorométhane, refroidie à environ 0°C, sont ajoutés par petites portions 2,84 g (14,07 mmoles) de chloroformiate de 4-nitrophényle. Le milieu est maintenu sous agitation à 0°C pendant 1 heure puis à température ambiante pendant 1 heure. Le précipité formé est filtré puis rincé abondamment avec le diisopropyléther. Après séchage sous vide à environ 60°C, on obtient 3,12 g de produit attendu sous la forme d'un solide blanc utilisé tel quel dans l'étape suivante.
PF(°C) : 143-145
RMN ¹H (DMSO, 400 MHz) δ (ppm) : 8,40(d, 2H) ; 8,25 (large s, 1H) ; 7,90 (large s, 1H) ; 7,65 (d, 2H) ; 7,0 (s, 1H) ; 5,50 (s, 2H).

### 15.6 [7-(5-Isobutyl-pyridin-2-yl)-7-aza-spiro[3.5]non-2-yl]-carbamate de 3-carbamoyl-isoxazol-5-ylméthyle

On procède suivant le mode opératoire décrit dans l'exemple 1 à l'étape 1.1. A partir de 0,26 g (0,95 mmole) de 7-(5-isobutyl-pyridin-2-yl)-7-aza-spiro[3.5]non-2-ylamine, obtenu à l'étape 15.4, de 0,35 g (1,15 mmole) de 4-nitro-phényl-carbonate de 3-carbamoyl-isoxazol-5-ylméthyle, obtenu à l'étape 15.5, de 0,42 mL (2,39 mmoles) de *N,N-*diisopropyléthylamine et de 0,06 g (0,48 mmole) de *N,N-*diméthylaminopyridine, dans 9 mL de 1,2-dichloroéthane, on obtient 0,35 g de produit pur sous forme de poudre blanche.
PF(°C) : 178-180°C
LC-MS : M+H = 442
RMN ¹H (DMSO) δ (ppm) : 8,15 (m, 1H); 7,90 (m, 1H); 7.85 (m, 1H); 7,75 (m, 1H); 7,30 (m, 1H); 6,75 (m, 2H); 5,20 (s, 2H); 4,00 (m, 1H); 3,40 (m, 2H); 3,35 (m, 2H); 2,30 (m, 2H); 2,20 (m, 2H); 1,75 (m, 3H); 1,55 (m, 4H); 0,85 (d, 6H).

### Exemple 16 (Composé N°40)

### [6-(4-Trifluorométhyl-pyrimidin-2-yl)-6-aza-spiro[2.5]oct-1-yl]-carbamate de 3-carbamoyl-isoxazol-5-ylméthyle

### 16.1 1-Benzyloxycarbonylamino-6-aza-spiro[2.5]octane-6-carboxylate de tert-butyle

5,00 g (19,58 mmoles) de 6-aza-spiro[2.5]octane-1,6-dicarboxylate de 6-*tert*-butyle (commerciale) est mis en solution dans 11 mL de toluène puis à 0°C sous argon, 2,99 mL (21,54 mmoles) de triéthylamine et 4,66 mL (21,54 mmoles) de diphenylphosphonique azide sont ajoutés. Laisser revenir à température ambiante et agiter pendant 1 heure 30 à 110°C. L'alcool benzylique (2,23 mL; 21,54 mmoles) est ensuite ajouté puis le mélange agité à 110°C pendant 15 heures.

Après retour à température ambiante, une solution saturée d'hydrogénocarbonate de sodium est ajoutée, le mélange est extrait à l'éther diéthylique puis les phases organiques réunies sont lavées succesivement par une solution saturée d'hydrogénocarbonate de sodium puis une solution saturée de chlorure de sodium. On sèche la phase organique sur sulfate de sodium. Après évaporation sous pression réduite et purification sur colonne de gel de silice en éluant avec un mélange de 100/0/0 à 98/2/0,2 de dichlorométhane, de méthanol et d'ammoniaque 28%, on obtient 5,50 g de produit attendu sous forme d'huile jaune.
LC-MS : M+H = 361
RMN ¹H (DMSO) δ (ppm): 7,40 (m, 5H); 5,10 (s, 2H); 3,45-3,20 (m, 4H); 2,40 (m, 1H); 1,40 (s, 9H); 1,30 (m, 3H), 1,20 (m, 1H); 1,75 (m, 1H); 1,45 (m, 1H).

### 16.2 (6-Aza-spiro[2.5]oct-1-yl)-carbamate de benzyle

On procède suivant le mode opératoire décrit dans l'exemple 2 (étape 2.5.). A partir de 5,50 g (15,28 mmoles) de 1-benzyloxycarbonylamino-6-aza-spiro[2.5]octane-6-carboxylate de *tert*-butyle obtenu à l'étape précédente et de 15,28 mL (61,10 mmoles) d'une solution d'acide chlorhydrique 4N dans le dioxane et après extraction basique, on obtient 3,56 g de produit sous forme poudre jaune claire.
LC-MS : M+H = 261
PF(°C) : 223-225
RMN ¹H (DMSO) δ (ppm) : 7,40 (m, 6H); 5,10 (s, 2H); 3,00 (m, 3H); 2,40 (m, 2H); 1,60 (m, 1H); 1,40 (m, 3H); 1,70-1,40 (m, 2H).

### 16.3 [6-(4-Trifluorométhyl-pyrimidin-2-yl)-6-aza-spiro[2.5]oct-1-yl]-carbamate de benzyle

Dans un ballon, on introduit 0,60 g (2,30 mmoles) de (6-aza-spiro[2.5]oct-1-yl)-carbamate de benzyle obtenu à l'étape précédente, 0,36 mL (3,00 mmole) de 2-chloro-4-trifluorométhylpyrimidine et 0,80 mL (4,61 mmoles) de diisopropyléthylmaine dans 17 mL d'acétoninitrile et 3 mL de diméthylformamide. On chauffe ensuite à 95°C pendant 15 heures. On laisse revenir à température ambiante puis on reprend le mélange réactionnel par du dichlorométhane et de l'eau. On sépare la phase aqueuse, on l'extrait deux fois avec du dichlorométhane, on lave les phases organiques réunies avec une solution aqueuse saturée en chlorure d'ammonium, on les sèche sur sulfate de sodium et on concentre le filtrat sous pression réduite. Après évaporation du solvant, on purifie le résidu obtenu par chromatographie sur gel de silice en éluant avec un mélange de 100/0/0 à 99/1/0,1 de dichlorométhane, de méthanol et d'ammoniaque à 28%. On obtient ainsi 0,88 g de produit sous forme d'huile.
LC-MS : M+H = 407
RMN ¹H (CDCl₃) δ (ppm) : 8,70 (d, 1H); 7,50 (m, 1H); 7,40 (m, 4H); 7,20 (m, 1H); 7,00 (d, 1H); 5,10 (s, 2H); 3,95 (m, 2H); 3,70 (m, 2H); 2,50 (m, 1H); 1,50 (m, 2H); 1,30 (m, 2H); 1,75 (m, 1H); 1,50 (m, 1H)

### 16.4 6-(4-Trifluorométhyl-pyrimidin-2-yl)-6-aza-spiro[2.5]oct-1-ylamine

On procède suivant le mode opératoire décrit dans l'exemple 2 (étape 2.5.). A partir de 0,86 g (2,13 mmoles) de [6-(4-trifluorométhyl-pyrimidin-2-yl)-6-aza-spiro[2.5]oct-1-yl]-carbamate de benzyle obtenu à l'étape précédente et de 3,74 mL (21,33 mmoles) d'une solution d'acide bromydrique à 35 % dans l'acide acétique et après extraction basique et organisation dans l'éther diéthylique, on obtient 0,32 g de produit sous forme poudre blanche.
PF(°C) : 243-245°C
LC-MS : M+H = 273
RMN ¹H (DMSO) δ (ppm) : 8,70 (d, 1H) ; 8,15 (**s large,** 2H); 7,00 (d, 1H); 4,10 (m, 1H) ; 4,00 (m, 1H); 3,70 (m, 2H); 2,50 (m, 1H); 1,70 (m, 2H); 1,50 (m, 1H); 1,35 (m, 1H); 1,90 (m, 1H); 1,80 (m, 1H).

### 16.5 [6-(4-Trifluorométhyl-pyrimidin-2-yl)-6-aza-spiro[2.5]oct-1-yl]-carbamate de 3-carbamoyl-isoxazol-5-ylméthyle

On procède suivant le mode opératoire décrit dans l'exemple 1 à l'étape 1.1. A partir de 0,15 g (0,55 mmole) de 6-(4-trifluorométhyl-pyrimidin-2-yl)-6-aza-spiro[2.5]oct-1-ylamine, obtenu à l'étape 16.4, de 0,20 g (0,66 mmole) de 4-nitro-phényl-carbonate de 3-carbamoyl-isoxazol-5-ylméthyle, obtenu à l'étape 15.5, de 0,24 mL (1,38 mmole) de *N,N-*diisopropyléthylamine et de 0,03 g (0,28 mmole) de *N,N-*diméthylaminopyridine, dans 5 mL de 1,2-dichloroéthane, on obtient 0,15 g de produit pur sous forme de poudre blanche après purification par chromatographie sur gel de silice en éluant avec un mélange de 100/0/0 à 98/2/0,2 de dichlorométhane, de méthanol et d'ammoniaque à 28%.
PF(°C) : 183-185°C
LC-MS : M+H = 441
RMN ¹H (DMSO) δ (ppm) : 8,70 (d, 1H) ; 8,15 (s large, 1H) ; 7,85 (s large, 1H); 7,70 (s large, 1H); 7,00 (d, 1H); 6,80 (s, 1H); 5,20 (s, 2H); 3,90 (m, 2H); 3,75 (m, 2H); 2,50 (m, 1H); 1,40 (m, 3H); 1,30 (m, 1H); 1,70 (m, 1H); 1,50 (m, 1H)

Le tableau 1 qui suit illustre les structures chimiques et les propriétés physiques de quelques composés selon l'invention.

Dans ce tableau :
- tous les composés sont sous forme de base libre ;
- les composés N° 10 et 11 sont des mélanges d'isomères. Le composé N° 12 est sous forme d'isomère I tandis que le composé N° 13 est sous forme d'isomère II. Le composé N° 17 est sous forme d'isomère I tandis que le composé N° 18 est sous forme d'isomère II. Les composées 16, 21 et 22 sont sous la forme d'un isomère. Ces isomères correspondent aux isomères de position de la chaîne -A-NH- par rapport à la chaîne -(CH₂)ₙN-.
- la colonne « PF (°C) » renseigne les points de fusion des produits en degrés Celsius (°C).
- Le composé 26 est sous forme de sel.

**Tableau 1**

| | | | | | | | | | | |
|---|---|---|---|---|---|---|---|---|---|---|
| 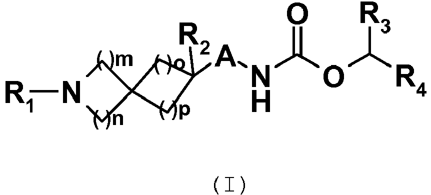 | | | | | | | | | | |

| **N°** | **R₁** | **m** | **n** | **o** | **p** | **A** | **R₂** | **R₃** | **R₄** | **PF (°C)** |
|---|---|---|---|---|---|---|---|---|---|---|
| 1. | 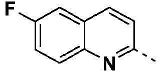 | 2 | 2 | 1 | 1 | liaison | H | H | 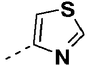 | 107-109°C |
| 2. | 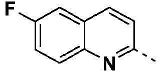 | 2 | 2 | 1 | 1 | liaison | H | H | 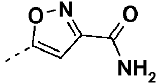 | 226-228°C |
| 3. | 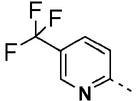 | 2 | 2 | 1 | 1 | liaison | H | H | 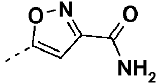 | 167-169°C |
| 4. | 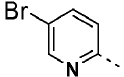 | 2 | 2 | 1 | 1 | liaison | H | H | 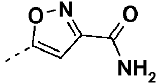 | 165-167°C |
| 5. | 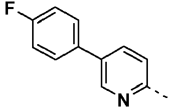 | 2 | 2 | 1 | 1 | liaison | H | H | 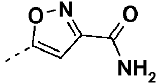 | 216-218°C |
| 6. | 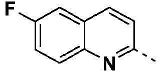 | 2 | 2 | 1 | 1 | CH₂ | H | H | 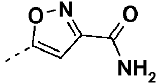 | 190-192°C |
| 7. | 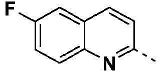 | 2 | 2 | 1 | 1 | CH₂ | H | H | 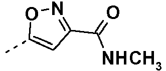 | 134-136°C |
| 8. | 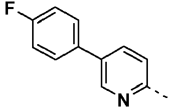 | 2 | 2 | 1 | 1 | liaison | H | H | 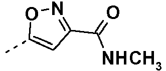 | 219-221°C |
| 9. | 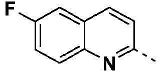 | 2 | 2 | 1 | 1 | liaison | H | H | 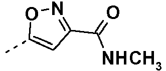 | 193-195°C |
| 10. | 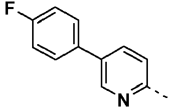 | 2 | 1 | 1 | 1 | liaison | H | H | 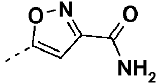 | 216-218°C |
| 11. | 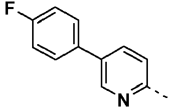 | 2 | 1 | 1 | 1 | liaison | H | H | 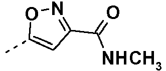 | 184-186°C |
| 12. | 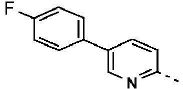 | 2 | 1 | 1 | 1 | CH₂ | H | H | 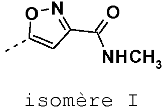 | 171-173°C |
| 13. | 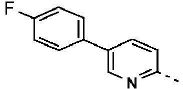 | 2 | 1 | 1 | 1 | CH₂ | H | H | 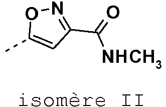 | 180-182°C |
| 14. | 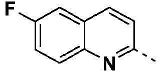 | 2 | 2 | 1 | 1 | liaison | H | H | 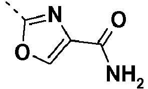 | 188-190°C |
| 15. | 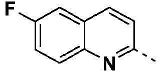 | 1 | 1 | 1 | 1 | liaison | H | H | 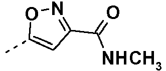 | 167-169°C |
| 16. | 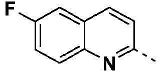 | 2 | 1 | 1 | 1 | CH₂ | H | H | 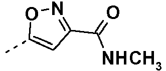 | 102-104°C |
| 17. | 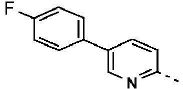 | 2 | 1 | 1 | 1 | liaison | H | H | 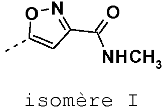 | 203-205°C |
| 18. | 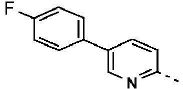 | 2 | 1 | 1 | 1 | liaison | H | H | 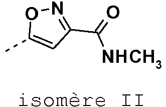 | 194-196°C |
| 19. | 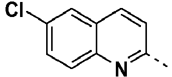 | 2 | 2 | 1 | 1 | liaison | H | H | 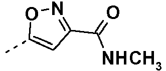 | 194-196°C |
| 20. | 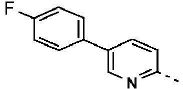 | 1 | 1 | 1 | 1 | liaison | H | H | 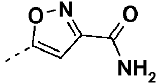 | 239-241°C |
| 21. | 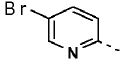 | 2 | 1 | 1 | 1 | liaison | H | H | 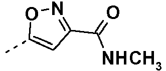 | 175-177°C |
| 22. | 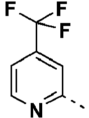 | 2 | 1 | 1 | 1 | liaison | H | H | 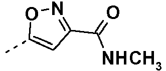 | 167-170°C |
| 23. | 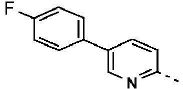 | 1 | 1 | 1 | 1 | liaison | H | H | 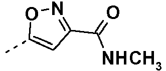 | 218-220°C |
| 24. | 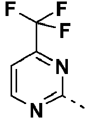 | 2 | 2 | 1 | 1 | liaison | H | H | 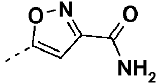 | 153-155°C |
| 25. | 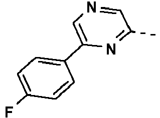 | 2 | 2 | 1 | 1 | liaison | H | H | 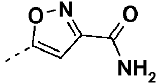 | 145-147°C |
| 26. | 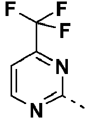 | 2 | 2 | 1 | 1 | liaison | H | H | 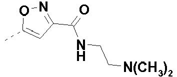 | 147-150°C |
| 27. | 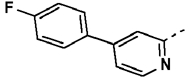 | 2 | 2 | 1 | 1 | liaison | H | H | 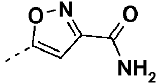 | 162-164°C |
| 28. | 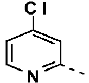 | 2 | 2 | 1 | 1 | liaison | H | H | 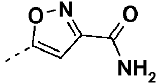 | 164-166°C |
| 29. | 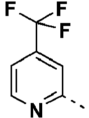 | 2 | 2 | 1 | 1 | liaison | H | H | 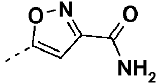 | 162-164°C |
| 30. | 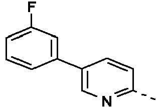 | 2 | 2 | 1 | 1 | liaison | H | H | 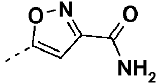 | 214-216°C |
| 31. | 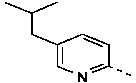 | 2 | 2 | 1 | 1 | liaison | H | H | 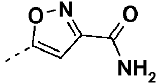 | 178-180°C |
| 32. | 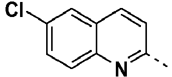 | 2 | 2 | 1 | 1 | liaison | H | H | 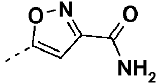 | 216-218°C |
| 33. | 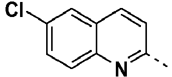 | 2 | 2 | 1 | 1 | CH₂ | H | H | 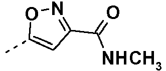 | 179-181°C |
| 34. | 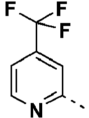 | 2 | 2 | 1 | 1 | liaison | H | H | 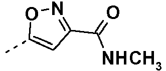 | 116-118°C |
| 35. | 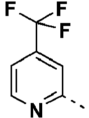 | 2 | 2 | 1 | 1 | liaison | H | H | 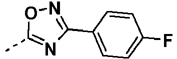 | 103,5-105,5°C |
| 36. | 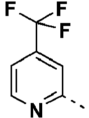 | 2 | 2 | 1 | 1 | liaison | H | H | 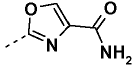 | 153,5-155,5°C |
| 37. | 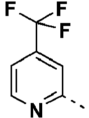 | 2 | 2 | 1 | 1 | liaison | H | H | 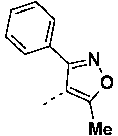 | 115, 6-117,6°C |
| 38. | 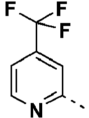 | 2 | 2 | 1 | 1 | liaison | H | H | 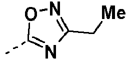 | 87, 6-89,6°C |
| 39. | 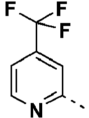 | 2 | 2 | 1 | 1 | liaison | H | H | 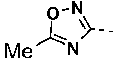 | 66-68°C |
| 40. | 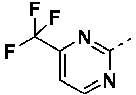 | 2 | 2 | 1 | 0 | liaison | H | H | 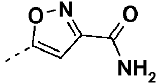 | 183-185 |
| 41. | 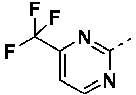 | 2 | 2 | 1 | 0 | liaison | H | H | 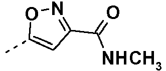 | 165-167 |

Le tableau 2 qui suit donne les résultats des analyses RMN ¹H pour les composés du tableau 1.

**Tableau 2**

| **N°** | **RMN ¹H (DMSO ou CDCl₃ 400MHZ) δ (ppm)** |
|---|---|
| **1** | 8,80 (s, 1H) ; 7,80 (d, 1H) ; 7,60 (m, 1H) ; 7,35 (s, 1H) ; 7,30-7,15 (m, 2H) ; 7,00 (d, 1H) ; 5,30 (s, 2H) ; 4,90 (large s, 1H) ; 4,20 (m, 1H) ; 3,70-3,50 (m, 4H) ; 2,35 (t, 2H); 1,80-1,60 (m, 6H). |
| **2** | 8,15 (large s, 1H) ; 8,00 (d, 1H) ; 7,85 (large s, 1H) ; 7,70 (d, 1H) ; 7,60 (m, 1H) ; 7,50 (m, 1H) ; 7,40 (m, 1H) ; 7,30 (d, 1H) ; 6,70 (s, 1H) ; 5,20 (s, 2H) ; 4,10 (m, 1H) ; 3,70-3,50 (m, 4H) ; 2,35 (t, 2H) ; 1,80 (t, 2H) ; 1,70-1,40 (m, 4H). |
| **3** | 8,40 (s, 1H) ; 8,20 (large s, 1H) ; 7,90 (large s, 1H) ; 7,80 (m, 2H) ; 7,00 (d, 1H) ; 6,80 (s, 1H) ; 5,25 (s, 2H) ; 4,10 (m, 1H) ; 3,70-3,50 (m, 4H) ; 2,35 (t, 2H) ; 1,80 (t, 2H) ; 1,70-1,40 (m, 4H). |
| **4** | 8,10 (s, 2H) ; 7,80 (s, 1H) ; 7,70 (d, 1H) ; 7,60 (d, 1H) ; 6,85 (d, 1H) ; 6,75 (s, 1H) ; 5,20 (s, 2H) ; 4,00 (m, 1H) ; 3,55 (t, 2H) ; 3,45 (t, 2H) ; 2,20 (m, 2H) ; 1,75 (m; 2H) ; 1,65-1,45 (m, 4H). |
| **5** | 8,40 (s, 1H) ; 8,20 (large s, 1H) ; 7,90-7,70 (m, 3H) ; 7,60 (m, 2H) ; 7,25 (m, 2H) ; 6,90 (d, 1H) ; 6,75 (s, 1H) ; 5,20 (s, 2H) ; 4,00 (m, 1H) ; 3,55 (t, 2H) ; 3,45 (t,2H) ; 2,20 (m, 2H) ; 1,75 (m; 2H) ; 1,65-1,45 (m, 4H). |
| **6** | 8,15 (large s, 1H) ; 8,0 (d, 1H) ; 7,85 (large s, 1H) ; 7,60-7,35 (m, 4H) ; 7,25 (d, 1H) ; 6,75 (s, 1H) ; 5,20 (s, 2H) ; 3,70 (t, 2H) ; 3,60 (t, 2H) ; 3,10 (t, 2H) ; 2,40 (m, 1H) ; 1,90 (t, 2H) ; 1,70 (m, 2H) ; 1,60 (m, 4H). |
| **7** | 8,70 (large s, 1H) ; 8,00 (d, 1H) ; 7,60 (m, 1H) ; 7,50 (m, 2H) ; 7,40 (m, 1H) ; 7,30 (d, 1H) ; 6,75 (s, 1H) ; 5,20 (s, 2H) ; 3,70 (t, 2H) ; 3,60 (t, 2H) ; 3,10 (t, 2H) ; 2,80 (s, 3H) ; 2,40 (m, 1H) ; 1,90 (t, 2H) ; 1,60 (m, 2H) ; 1,50 (m, 4H). |
| **8** | 8,70 (large s, 1H) ; 8,40 (s, 1H) ; 7,90-7,70 (m, 2H); 7,65 (m, 2H) ; 7,25 (m, 2H) ; 6,90 (d, 1H) ; 6,80(s, 1H) ; 5,20 (s, 2H) ; 4,00 (m, 1H) ; 3,55 (t, 2H) ; 3,45 (t, 2H) ; 2,80 (s, 3H) ; 2,20 (m, 2H) ; 1,75 (m; 2H) ; 1,65-1,45 (m, 4H). |
| **9** | 8,70 (large s, 1H) ; 8,00 (d, 1H) ; 7,85 (d, 1H) ; 7,70 (m, 1H) ; 7,60 (m, 1H) ; 7,50 (m, 1H) ; 7,40 (m, 1H) ; 6,80 (s, 1H) ; 5,20 (s, 2H) ; 4,10 (m, 1H) ; 3,70-3,50 (m, 4H) ; 2,80(s, 3H) ; 2,20 (t, 2H) ; 1,70 (t, 2H) ; 1,70-1,50 (m, 4H). |
| **10** | 8,40 (s, 1H) ; 8,20 (large s, 1H) ; 7,80 (large s, 3H) ; 7,60 (s, 2H) ; 7,30 (s, 2H) ; 6,80 (s, 1H) ; 6,50 (t, 1H) ; 5,20 (s, 2H) ; 4,10 (m, 1H) ; 3,50 -3,30 (m, 4H) ; 2,30 (m, 2H) ; 2,00 ( m, 4H). |
| **11** | 8,70 (s, 1H) ; 8,40 (s, 1H) ; 7,80 (m, 2H) ; 7,60 (m, 2H) ; 7,30 (m, 2H) ; 6,80 (s, 1H) ; 6,50 (t, 1H) ; 5,20 (s, 2H) ; 4,10 (m, 1H) ; 3,50-3,30 (m, 4H) ; 2,80 (s, 3H) ; 2,30 (m, 2H) ; 2,00 ( m, 4H). |
| **12** | 8,70 (large s, 1H) ; 8,40 (s, 1H) ; 7,80 (m, 1H) ; 7,60 (m, 2H) ; 7,50 (m, 1H) ; 7,30 (m, 2H) ; 6,80 (s, 1H) ; 6,50 (d, 1H) ; 5,20 (s, 2H) ; 3,50 (m, 2H) ; 3,35 (m, 2H) ; 3,10 (t, 2H) 2,80 (s, 3H) ; 2,40 (m, 1H) ; 2,00 ( m, 4H) ; 1,80 (m, 2H). |
| **13** | 8,70 (large s, 1H) ; 8,40 (s, 1H) ; 7,80 (m, 1H) ; 7,60 (m, 2H) ; 7,50 (m, 1H) ; 7,30 (m, 2H) ; 6,80 (s, 1H) ; 6,50 (d, 1H) ; 5,20 (s, 2H) ; 3,50 (s, 2H) ; 3,40 (m, 2H) ; 3,10 (t, 2H) ; 2,80 (s, 3H) ; 2,40 (m, 1H) ; 2,10 ( t, 2H) ; 1,90 ( t, 2H) 1,80 (t, 2H). |
| **14** | 8,60 (s, 1H) ; 8,00 (d, 1H) ; 7,80 (d, 1H) ; 7,70 (large s, 1H) ; 7,60 (m, 1H) ; 7,50 (m, 2H) ; 7,40 (m, 1H) ;7,30 (d, 1H) ; 5,10 (s, 2H) ; 4,10 (m, 1H) ; 3,70-3,50 (m, 4H) ; 2,35 (m, 2H) ; 1,80 (t, 2H) ; 1,70-1,40 (m, 4H). |
| **15** | 8,70 (large s, 1H) ; 8,05 (d, 1H) ; 7,80 (d, 1H) ; 7,60 (m, 1H) ; 7,50 (m, 1H) ; 7,40 (m, 1H) ; 6,80 (s, 1H) ; 6,75 (s, 1H) ; 5,25 (s, 2H) ; 4,15 (s, 2H) ; 4,05 (s, 2H) ; 3,95 (m, 1H) ; 2,80(s, 3H) ; 2,50 (t, 2H) ; 2,20 (t, 2H). |
| **16** | 8,70 (large s, 1H) ; 8,00 (d, 1H) ; 7,55 (m, 3H) ; 7,40 (m, 1H) ; 6,90 (d, 1H) ; 6,80 (s, 1H) ; 5,20 (s, 2H) ; 3,55 (t, 2H) ; 3,45 (s, 2H) ; 3,10 (t, 2H) ; 2,75 (s, 3H) ; 2,45 (m, 1H) ; 2.02 (m, 4H) ; 1,80 (m, 2H). |
| **17** | 8,70 (large s, 1H), 8,40 (s, 1H) ; 7,81 (m, 2H) ; 7,62 (m, 2H) ; 7,25 (t, 2H) ; 6,78 (s, 1H) ; 6,50 (d, 1H) ; 5,20 (s, 2H) ; 4,08 (m, 1H) ; 3,50 (s, 2H), 3,40 (m, 2H) ; 2.78 (s, 3H), 2,30 (m, 2H) ; 1.99 (m, 4H). |
| **18** | 8,70 (large s, 1H), 8,38 (s, 1H) ; 7,80 (m, 2H) ; 7,60 (m, 2H) ; 7,23 (t, 2H) ; 6,78 (s, 1H) ; 6,48 (d, 1H) ; 5,18 (s, 2H) ; 4,08 (m, 1H) ; 3,45 (m, 2H), 3,40 (m, 2H) ; 2.78 (d, 3H), 2,25 (m, 2H) ; 2.00 (m, 4H). |
| **19** | 8,70 (large s, 1H) ; 8,00 (d, 1H) ; 7,80 (m, 2H) ; 7,50 (m, 2H); 7,30 (d, 1H) ; 6,80 (s, 1H); 5,20 (s, 2H) ; 4,10 (m, 1H) ; 3,75-3,55 (m, 4H) ; 2,80 (s, 3H) ; 2,20 (m, 2H) ; 1,70 (m, 2H) ; 1,70-1,50 (m, 4H). |
| **20** | 8,40 (s, 1H) ; 8,15 (**s large,** 1H) ; 7,80 (m, 3H) ; 7,65 (m, 2H) ; 7,25 (m, 2H); 6,80 (s, 1H) ; 6,40 (d, 1H) ; 5,20 (s, 2H) ; 4,00 (m, 2H) ; 3,90 (m, 3H) ; 2,50 (m, 2H) ; 2,15 (m, 2H) |
| **21** | 8,70 (**s large,** 1H) ; 8,10 (s, 1H) ; 7,80 (d1, 1H) ; 7,60 (d, 1H) ; 6,80 (s, 1H) ; 6,40 (d, 1H) ; 5,20 (s, 2H) ; 4,05 (m, 1H) ; 3,40 (m, 2H) ; 3,30 (m, 2H) ; 2,80 (s, 3H) ; 2,30 (m, 2H) ; 2,00 (m, 4H). |
| **22** | 8,70 (**s large,** 1H) ; 8,30 (m, 1H) ; 7,80 (dl, 1H) ; 6,80 (m, 2H) ; 6,70 (s, 1H) ; 5,20 (s, 2H) ; 4,10 (m, 1H) ; 3,50 (m, 2H) ; 3,40 (m, 2H) ; 2,80 (s, 3H) ; 2,30 (m, 2H) ; 2,00 (m, 4H). |
| **23** | 8,70 (m, 1H) ; 8,40 (s, 1H) ; 7,80 (m, 2H); 7,60 (m, 2H) ; 7,25 (m, 2H) ; 6,80 (s, 1H) ; 6,40 (d, 1H) ; 5,20 (s, 2H) ; 4,00 (s, 2H) ; 3,95 (m, 1H) ; 3,90 (s, 2H) ; 2,80 (s, 3H) ; 2,50 (m, 2H) ; 2,20 (m, 2H). |
| **24** | 8,70 (d, 1H) ; 8,15 (**s large**, 1H) ; 7, 85 (s large, 1H) ; 7, 75 (**s large**, 1H) ; 6,95 (d, 1H) ; 6,75 (s, 1H) ; 5,20 (s, 2H) ; 4,05 (m, 1H) ; 3,80 (m, 2H) ; 3,70 (m, 2H) ; 2,20 (m, 2H) ; 1,80 (m, 2H) ; 1,60 (m, 2H) ; 1,50 (m, 2H). |
| **25** | 8,40 (s, 1H) ; 8,30 (s, 1H) ; 8,10 (m, 3H) ; 7,85 (**s large,** 1H) ; 7,75 (dl, 1H) ; 7,30 (m, 2H) ; 6,80 (s, 1H) ; 5,20 (s, 2H) ; 4,05 (m, 1H) ; 3,65 (m, 2H) ; 3,55 (m, 2H) ; 2,10 (m, 2H) ; 1,80 (m, 2H) ; 1,65 (m, 2H) ; 1,60 (m, 2H). |
| **26** | 10,10 (**s large,** 1H) ; 9,00 (m, 1H) ; 8,60 (m, 1H) ; 7,70 (**s large,** 1H) ; 6,90 (m, 1H) ; 6,80 (m, 1H) ; 5,20 (s, 2H) ; 4,00 (m, 1H) ; 3,70 (m, 2H) ; 3,60 (m, 4H) ; 3,35 (m, 2H); 2,80 (s, 6H) ; 2,20 (m, 2H) ; 1,70 (m, 2H) ; 1,60 (m, 2H) ; 1,50 (m, 2H). |
| **27** | 8,15 (m, 2H) ; 7,85 (m, 3H) ; 7,75 (dl, 1H) ; 7,30 (m, 2H) ; 7,00 (s, 1H) ; 6,90 (d, 1H) ; 6,75 (s, 1H) ; 5,20 (s, 2H) ; 4,05 (m, 1H) ; 3,60 (m, 2H) ; 3,50 (m, 2H); 2,10 (m, 2H) ; 1,75 (m, 2H); 1,60 (m, 2H) ; 1,50 (m, 2H). |
| **28** | 8,15 (**s large,** 1H) ; 8,05 (d, 1H) ; 7,80 (**s large,** 1H) ; 7,75 (dl, 1H) ; 6,90 (s, 1H) ; 6,80 (s, 1H) ; 6,65 (d, 1H) ; 5,20 (s, 2H) ; 4,00 (m, 1H) ; 3,50 (m, 2H) ; 3,40 (m, 2H) ; 2,20 (m, 2H); 1,70 (m, 2H) ; 1,55 (m, 2H) ; 1,50 (m, 2H). |
| **29** | 8,30 (d, 1H) ; 8,15 (**s large,** 1H) ; 7,85 (**s large,** 1H) ; 7,75 (dl, 1H) ; 7,10 (s, 1H); 6,85 (d, 1H) ; 6,80 (s, 1H) ; 5,20 (s, 2H) ; 4,05 (m, 1H) ; 3,60 (m, 2H) ; 3,50 (m, 2H); 2,20 (m, 2H) ; 1,80 (m, 2H) ; 1,60 (m, 2H) ; 1,50 (m, 2H). |
| **30** | 8,50 (s, 1H) ; 8,15 (**s large,** 1H) ; 7,90 (m, 2H) ; 7,65 (dl, 1H) ; 7,50 (m, 3H) ; 7,10 (m, 1H) ; 6,90 (d, 1H) ; 6,80 (s, 1H) ; 5,20 (s, 2H) ; 4,05 (m, 1H) ; 3,60 (m, 2H) ; 3,50 (m, 2H) ; 2,20 (m, 2H) ; 1,70 (m, 2H) ; 1,60 (m, 2H) ; 1,50 (m, 2H). |
| **31** | 8,15 (m, 1H) ; 7,90 (m, 1H) ; 7.85 (m, 1H) ; 7,75 (m, 1H) ; 7,30 (m, 1H) ; 6,75 (m, 2H) ; 5,20 (s, 2H) ; 4,00 (m, 1H) ; 3,40 (m, 2H) ; 3,35 (m, 2H) ; 2,30 (m, 2H) ; 2,20 (m, 2H) ; 1,75 (m, 3H) ; 1,55 (m, 4H) ; 0,85 (d, 6H). |
| **32** | 8,15 (s, 1H) ; 8,00 (d, 1H) ; 7,85 (m, 1H) ; 7,80 (m, 2H) ; 7,50 (m, 2H) ; 7,30 (d, 1H); 6,80 (s, 1H) ; 5,20 (s, 2H) ; 4,05 (m, 1H); 3,70 (m, 2H) ; 3,60 (m, 2H) ; 2,20 (m, 2H) ; 1,75 (m, 2H) ; 1,65 (m, 2H) ; 1,55 (m, 2H). |
| **33** | 8,70 (m, 1H) ; 8,00 (d, 1H) ; 7,80 (s, 1H) ; 7,55 (m, 3H) ; 7,30 (d, 1H) ; 6,80 (s, 1H) ; 5,20 (s, 2H) ; 3,70 (m, 2H) ; 3,60 (m, 2H) ; 3,10 (m, 2H) ; 2,80 (d, 3H) ; 2,40 (m, 1H) ; 1,90 (m, 2H) ; 1,60 (m, 2H); 1,50 (m, 4H). |
| **34** | 8,70 (**s large,** 1H) ; 8,30 (d, 1H) ; 7,75 (dl, 1H) ; 7,05 (s, 1H) ; 6,80 (d, 1H) ; 6,75 (s, 1H) ; 5,20 (s, 2H); 4,05 (m, 1H) ; 3,60 (m, 2H) ; 3,50 (m, 2H) ; 2,80 (s, 3H) ; 2,20 (m, 2H) ; 1,80 (m, 2H) ; 1,60 (m, 2H) ; 1,50 (m, 2H). |
| **35** | 8,30 (d, 1H) ; 8,10 (m, 2H) ; 8,95 (dl, 1H) ; 7,45 (m, 2H) ; 7,05 (s, 1H) ; 6,80 (d, 1H) ; 5,40 (s, 2H) ; 4,05 (m, 1H) ; 3,55 (m, 4H) ; 2,20 (m, 2H) ; 1,80 (m, 2H) ; 1,60 (m, 4H). |
| **36** | 8,60 (s, 1H) ; 8,30 (d, 1H) ; 7,80 (d1, 1H) ; 7,60 (**s large,** 1H) ; 7,50 (**s large,** 1H) ; 7,05 (s, 1H) ; 6,80 (d, 1H) ; 5,10 (s, 2H) ; 4,00 (m, 1H) ; 3,50 (m, 4H) ; 2,20 (m, 2H) ; 1,70 (m, 2H) ; 1,50 (m, 4H). |
| **37** | 8,30 (d, 1H) ; 7,75 (m, 2H) ; 7,55 (m, 4H) ; 7,10 (s, 1H), 6,80 (d, 1H) ; 4,95 (s, 2H) ; 4,05 (m, 1H) ; 3,50 (m, 4H) ; 2,50 (s, 3H) ; 2,20 (m, 2H) ; 1,70 (m, 2H) ; 1,55 (m, 4H). |
| **38** | 8,30 (d, 1H) ; 7,90 (m, 1H) ; 7,10 (s, 1H), 6,80 (d, 1H) ; 5,30 (s, 2H) ; 4,00 (m, 1H) ; 3,55 (m, 4H) ; 2,75 (q, 2H) ; 2,10 (m, 2H) ; 1,80 (m, 2H) ; 1,55 (m, 4H) ; 1,25 (t, 3H). |
| **39** | 8,30 (d, 1H) ; 7,75 (m, 1H) ; 7,10 (s, 1H), 6,80 (d, 1H) ; 5,10 (s, 2H) ; 4,05 (m, 1H) ; 3,55 (m, 4H) ; 2,60 (s, 3H) ; 2,20 (m, 2H) ; 1,75 (m, 2H) ; 1,55 (m, 4H). |
| **40** | 8,70 (d, 1H) ; 8,15 (**s large,** 1H) ; 7,85 (**s large,** 1H) ; 7,70 (**s large,** 1H) ; 7,00 (d, 1H) ; 6,80 (s, 1H) ; 5,20 (s, 2H) ; 3,90 (m, 2H) ; 3,75 (m, 2H); 2,50 (m, 1H) ; 1,40 (m, 3H) ; 1,30 (m, 1H) ; 1,70 (m, 1H) ; 1,50 (m, 1H) |
| **41** | 8,70 (m, 2H) ; 7,70 (**s large,** 1H) ; 7,00 (d, 1H) ; 6,80 (s, 1H) ; 5,20 (s, 2H) ; 3,90 (m, 2H) ; 3,70 (m, 2H) ; 2,80 (d, 3H) ; 2,50 (m, 1H) ; 1,40 (m, 3H) ; 1,30 (m, 1H) ; 1,70 (m, 1H) ; 1,50 (m, 1H) |

Le tableau 3 qui suit donne les résultats des analyses LCMS pour les composés du tableau 1.
Méthode LC-MS (M+H) :
UPLC / TOF - Gradient 3 min - H₂O / ACN / TFA T0 : 98%A - T 1, 6 à T 2,1min : 100% B - T 2,5 à T 3 min : 98% A Voie A : H₂O + 0,05% TFA ; Voie B : ACN + 0,035% TFA Débit : 1.0 mL/min - T°= 40°C - Injection 2µL - Colonne Acquity BEH C18 (50*2,1mm ; 1,7µm)"; 220 nm

**Table 3**

| ***N*°** | ***MASSE LCUVMS*** | ***TEMPS DE RETENTION (min)*** | **ion observé** |
|---|---|---|---|
| 1 | 427 | 0,78 | MH+ |
| 2 | 454 | 0,74 | MH+ |
| 3 | 454 | 0,95 | MH+ |
| 4 | 464 | 0,82 | MH+ |
| 5 | 480 | 0,84 | MH+ |
| 6 | 468 | 0,78 | MH+ |
| 7 | 482 | 0,80 | MH+ |
| 8 | 494 | 0,87 | MH+ |
| 9 | 468 | 0,76 | MH+ |
| 10 | 466 | 0,80 | MH+ |
| 11 | 480 | 0,83 | MH+ |
| 12 | 494 | 0,88 | MH+ |
| 13 | 494 | 0,88 | MH+ |
| 14 | 454 | 0,71 | MH+ |
| 15 | 440 | 0,73 | MH+ |
| 16 | 468 | 0,77 | MH+ |
| 17 | 480 | 0,83 | MH+ |
| 18 | 480 | 0,83 | MH+ |
| 19 | 484 | 0,83 | MH+ |
| 20 | 452 | 0,86 | MH+ |
| 21 | 464 | 0,77 | MH+ |
| 22 | 454 | 0,83 | MH+ |
| 23 | 466 | 0,89 | MH+ |
| 24 | 455 | 1,24 | MH+ |
| 25 | 481 | 1,15 | MH+ |
| 26 | 526 | 1,11 | MH+ |
| 27 | 480 | 0,92 | MH+ |
| 28 | 420 | 0,76 | MH+ |
| 29 | 454 | 0,95 | MH+ |
| 30 | 480 | 0,92 | MH+ |
| 31 | 442 | 0,91 | MH+ |
| 32 | 470 | 0,87 | MH+ |
| 33 | 498 | 0,97 | MH+ |
| 34 | 468 | 1,16 | MH+ |
| 35 | 506 | 1,24 | MH+ |
| 36 | 454 | 0,85 | MH+ |
| 37 | 501 | 1,25 | MH+ |
| 38 | 440 | 1,06 | MH+ |
| 39 | 426 | 0,97 | MH+ |
| 40 | 441 | 1,11 | MH+ |
| 41 | 455 | 1,18 | MH+ |

Les composés de l'invention ont fait l'objet d'essais pharmacologiques permettant de déterminer leur effet inhibiteur de l'enzyme FAAH (Fatty Acid Amide Hydrolase).

L'activité inhibitrice a été mise en évidence dans un test radioenzymatique basé sur la mesure du produit d'hydrolyse de l'anandamide [éthanolamine 1-³H] par la FAAH (Life Sciences (1995), 56, 1999-2005 et Journal of Biochemical and Biophysical Methods (2004), 60(2), 171-177). Ainsi, les cerveaux de souris (moins le cervelet) sont prélevés et conservés à -80°C. Les homogénats membranaires sont préparés extemporanément par homogénéisation des tissus à l'aide d'un appareil Precellys® dans le tampon de réaction (Tris-HCl 10 mM pH=8, NaCl 150 mM et acide éthylène-diamine-tétraacétique (EDTA) 1 mM). La réaction enzymatique est conduite dans des plaques de filtration Multiscreen 96puits dans un volume final de 70µl. Du tampon de réaction supplémenté avec de l'albumine de sérum bovin sans acides gras (BSA, 1 mg/mL) est utilisé pour la réaction enzymatique, la dilution des composés et de l'anandamide [éthanolamine 1-³H]. Sont ajoutés successivement dans les puits, du tampon de réaction contenant la BSA (43µl/puit), les composés dilués testés à différentes concentrations (7µl/puit contenant 1% de DMSO) et la préparation membranaire (10µl/puit soit 200 µg de tissu par essai). Après 20 minutes de pré-incubation des composés avec l'enzyme à 25°C, la réaction est démarrée par l'addition d'anandamide [éthanolamine 1-³H] (activité spécifique de 15-20 Ci/mmol) diluée avec de l'anandamide froide (10µl/puit, concentration finale de 10 µM, 0,01µCi par essai). Après 20 minutes d'incubation à 25°C, la réaction enzymatique est arrêtée par addition d'une solution de charbon actif 5M préparée dans un tampon NaCl 1,5M et HCl 0,5M (50µl/puit). Le mélange est agité 10 minutes puis la phase aqueuse contenant l'éthanolamine [1-³H] est récupérée par filtration sous-vide et comptée par scintillation liquide.

Dans ces conditions, les composés les plus actifs de l'invention présentent des CI₅₀ (concentration inhibant de 50 % l'activité enzymatique contrôle de la FAAH) comprises entre 0,001 et 1 µM, par exemple les composés n°4, n°6, n°8, n°10, n°12, n°19, n°25, n°31, n°40 ont des CI₅₀ respectives de 0,0082, 0,00025, 0,00072, 0,0023, 0,00085, 0,0018, 0,0017, 0,0043, 0,0005 µM.

Il apparaît donc que les composés selon l'invention ont une activité inhibitrice sur l'enzyme FAAH.

L'activité *in vivo* des composés de l'invention a été évaluée dans un test d'analgésie.

Ainsi, l'administration intrapéritonéale (i.p.) de PBQ (phénylbenzoquinone, 2 mg/kg dans une solution de chlorure de sodium à 0,9 % contenant 5 % d'éthanol) chez des souris mâles OF1 de 25 à 30 g, provoque des étirements abdominaux, en moyenne 30 torsions ou contractions pendant la période de 5 à 15 minutes après injection. Les composés testés sont administrés par voie orale (p.o.) ou par voie intrapéritonéale (i.p.) en suspension dans du Tween 80 à 0,5 %, 60 minutes ou 120 minutes avant l'administration de PBQ. Dans ces conditions, les composés les plus puissants de l'invention réduisent de 35 à 80 % le nombre d'étirements induits par le PBQ, dans une gamme de doses comprise entre 1 et 30 mg/kg. Par exemple, le composé n°5 du tableau 1 réduit de 50% le nombre d'étirements induits par le PBQ, à la dose de 30 mg/kg p.o. à 120 minutes.

L'enzyme FAAH (Chemistry and Physics of Lipids, (2000), 108, 107-121) catalyse l'hydrolyse des dérivés endogènes d'amides et d'esters de différents acides gras tels que la N-arachidonoyléthanolamine (anandamide), la *N*-palmitoyl-éthanolamine, la N-oléoyléthanolamine, l'oléamide ou le 2-arachidonoylglycérol. Ces dérivés exercent différentes activités pharmacologiques en interagissant, entre autres, avec les récepteurs cannabinoïdes et vanilloïdes.

Les composés de l'invention, bloquent cette voie de dégradation et augmentent le taux tissulaire de ces substances endogènes. Ils peuvent être utilisés à ce titre dans la prévention et le traitement des pathologies dans lesquelles les cannabinoïdes endogènes et/ou tous autres substrats métabolisés par l'enzyme FAAH, sont impliqués. On peut par exemple citer les maladies et les affections suivantes :
la douleur notamment les douleurs aiguës ou chroniques de type neurogène : migraine, douleurs neuropathiques incluant les formes associées au virus de l'herpès et au diabète et à la chimiothérapie, les douleurs aiguës ou chroniques associées aux maladies inflammatoires : arthrite, arthrite rhumatoïde, ostéoarthrite, spondylite, goutte, vascularite, maladie de Crohn, syndrome du colon irritable, les douleurs aiguës ou chroniques périphériques, les vertiges, les vomissements, les nausées en particulier celles consécutives à une chimiothérapie, les troubles du comportement alimentaire en particulier les anorexies et cachexies de diverses natures,
les pathologies neurologiques et psychiatriques : tremblements, dyskinésies, dystonies, spasticité, comportements compulsifs et obsessionnels, syndrome de Tourette, toutes les formes de dépression et d'anxiété de toute nature et origine, troubles de l'humeur, psychoses, les maladies neuro-dégénératives aiguës et chroniques : maladie de Parkinson, maladie d'Alzheimer, démence sénile, chorée de Huntington, lésions liées à l'ischémie cérébrale et aux traumatismes crâniens et médullaires, l'épilepsie, les troubles du sommeil incluant les apnées du sommeil, les maladies cardiovasculaires en particulier hypertension, arythmies cardiaques, artériosclérose, crise cardiaque, ischémies cardiaques, l'ischémie rénale, les cancers : tumeurs bénignes de la peau, papillomes et tumeurs cérébrales, tumeurs de la prostate, tumeurs cérébrales (glioblastomes, médullo-épithéliomes, médulloblastomes, neuroblastomes, tumeurs d'origine embryonnaire, astrocytomes, astroblastomes, épendyomes, oligodendrogliomes, tumeur du plexus, neuroépithéliomes, tumeur de l'épiphyse, épendymoblastomes, méningiomes malins, sarcomatoses, mélanomes malins, schwénnomes), les désordres du système immunitaire, notamment les maladies auto-immunes : psoriasis, lupus érythémateux, maladies du tissu conjonctif ou connectivites, syndrome de Sjögrer's, spondylarthrite ankylosante, spondylarthrite indifférenciée, maladie de Behcet's, anémies auto-immunes hémolytiques, sclérose en plaques, sclérose latérale amyotrophique, amyloses, rejet de greffes, maladies affectant la lignée plasmocytaire, les maladies allergiques : l'hypersensibilité immédiate ou retardée, rhinites ou conjonctivites allergiques, dermatites de contact, les maladies infectieuses parasitaires, virales ou bactériennes : SIDA, méningites, les maladies inflammatoires, notamment les maladies articulaires : arthrite, arthrite rhumatoïde, ostéoarthrite, spondylite, goutte, vascularite, maladie de Crohn, syndrome du colon irritable, l'ostéoporose, les affections oculaires : hypertension oculaire, glaucome, les affections pulmonaires : maladies des voies respiratoires, bronchospasmes, toux, asthme, bronchite chronique, obstruction chronique des voies respiratoires, emphysème, les maladies gastro-intestinales: syndrome du colon irritable, désordres inflammatoires intestinaux, ulcères, diarrhées, l'incontinence urinaire et l'inflammation vésicale.

L'utilisation des composés selon l'invention, à l'état de base, de sel d'addition à un acide, d'hydrate ou de solvat pharmaceutiquement acceptable, pour la préparation d'un médicament destiné à traiter les pathologies ci-dessus mentionnées fait partie intégrante de l'invention.

L'invention a également pour objet des médicaments qui comprennent un composé de formule (I), ou un sel d'addition à un acide, ou encore un hydrate ou un solvat pharmaceutiquement acceptable du composé de formule (I). Ces médicaments trouvent leur emploi en thérapeutique, notamment dans le traitement des pathologies ci-dessus mentionnées.

Selon un autre de ses aspects, la présente invention concerne des compositions pharmaceutiques renfermant en tant que principe actif, au moins un composé selon l'invention. Ces compositions pharmaceutiques contiennent une dose efficace d'un composé selon l'invention, ou un sel d'addition à un acide, ou un hydrate, ou un solvat pharmaceutiquement acceptable dudit composé, et éventuellement un ou plusieurs excipients pharmaceutiquement acceptables.

Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intrathécale, intranasale, transdermique, pulmonaire, oculaire ou rectale, le principe actif de formule (I) ci-dessus, ou son sel d'addition à un acide, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules, les chewing-gums et les solutions ou suspensions orales, les formes d'administration sublinguale, buccale, intratrachéale, intraoculaire, intranasale, par inhalation, les formes d'administration sous-cutanée, intramusculaire ou intraveineuse et les formes d'administration rectale ou vaginale. Pour l'application topique, on peut utiliser les composés selon l'invention dans des crèmes, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |

| | |
|---|---|
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,01 à 20 mg de principe actif par kg de poids corporel, selon la forme galénique.

Il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, le poids et la réponse dudit patient.

## Revendications

1. Composé répondant à la formule (I) 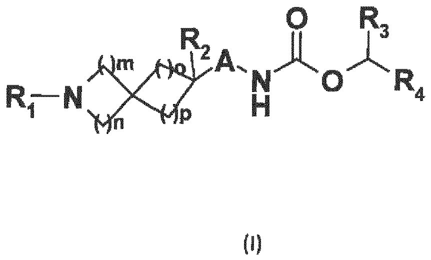 dans laquelle
R₂ représente un atome d'hydrogène, de fluor ou un groupe hydroxyle, cyano, trifluorométhyle, C₁₋₆-alkyle, C₁₋₆-alcoxy, NR₈R₉ ;
m, n, o et p représentent, indépendamment l'un de l'autre, un nombre entier égal à 0, 1, 2 ou 3 ;
A représente une liaison covalente ou un groupe C₁₋₈-alkylène ;
R₁ représente un groupe R₅ éventuellement substitué par un ou plusieurs groupes R₆ et/ou R₇ ;
R₅ représente un groupe choisi parmi un phényle, pyridinyle, pyridazinyle, pyrimidinyle, pyrazinyle, triazinyle, naphtalènyle, quinolinyle, isoquinolinyle, phtalazinyle, quinazolinyle, quinoxalinyle, cinnolinyle, naphthyridinyle ;
R₆ représente un atome d'halogène, un groupe cyano, -CH₂CN, nitro, hydroxyle, C₁₋₆-alkyle, C₁₋₆-alcoxy, C₁₋₆-thioalkyle, C₁₋₆-haloalkyle, C₁₋₆-haloalcoxy, C₁₋₆-halothioalkyle, C₃₋₇-cycloalkyle, C₃₋₇-cycloalkyle-C₁₋₃-alkylène, C₃₋₇-cycloalkyle-C₁₋₃-alkylène-o-, NR₈R₉, NR₈COR₉, NR₈CO₂R₉, NR₈SO₂R₉, NR₈SO₂NR₈R₉, COR₈, CO₂R₈, CONR₈R₉, SO₂R₈, SO₂NR₈R₉ ou -O-(C₁₋₃-alkylène)-O- ;
R₇ représente un groupe choisi parmi un furanyle, pyrrolyle, thiènyle, oxazolyle, isoxazolyle, thiazolyle, isothiazolyle, imidazolyle, pyrazolyle, oxadiazole, thiadiazole, phényle, pyridinyle, pyridazinyle, pyrimidinyle, pyrazinyle, triazine, naphtalènyle, quinolinyle, isoquinolinyle, phtalazinyle, quinazolinyle, quinoxalinyle, cinnolinyle, naphthyridinyle, imidazopyrimidinyle, thiènopyrimidinyle, benzofuranyle, benzothiènyle, benzimidazolyle, benzoxazolyle, benzisoxazolyle, benzothiazolyle, benzisothiazolyle, indolyle, isoindolyle, indazolyle, pyrrolopyridinyle, furopyridinyle, thiènopyridinyle, imidazopyridinyle, pyrazolopyridinyle, oxazolopyridinyle, isoxazolopyridinyle, thiazolopyridinyle, phényloxy, benzyloxy, pyrimidinoxy ;
le ou les groupes R₇ pouvant être substitués par un ou plusieurs groupes R₆ identiques ou différents l'un de l'autre ;
R₃ représente un atome d'hydrogène, de fluor, un groupe C₁₋₆-alkyle ou un groupe trifluorométhyle ;
R₄ représente un groupe choisi parmi un furanyle, pyrrolyle, thiènyle, thiazolyle, isothiazolyle, oxazolyle, isoxazolyle, pyrazolyle, oxadiazolyle, thiadiazolyle, imidazolyle, triazolyle, tétrazolyle;
ce groupe étant éventuellement substitué par un ou plusieurs substituants choisis parmi un atome d'halogène, un groupe C₁₋₆-alkyle, C₁₋₆-haloalkyle, C₃₋₇-cycloalkyle, C₃₋₇-cycloalkyle-C₁₋₃-alkylène, C₁₋₆-haloalcoxy, cyano, NR₈R₉, NR₈COR₉, NR₈CO₂R₉, NR₈SO₂R₉, NR₈SO₂NR₈R₉, COR₈, CO₂R₈, CONR₈R₉, CON(R₈) (C₁₋₃-alkylène-NR₁₀R₁₁), SO₂R₈, SO₂NR₈R₉, -O- (C₁₋₃-alkylène) -O-, phényle, phényloxy, benzyloxy, pyridinyle, pyrazinyle, pyridazinyle, triazinyle ou pyrimidinyle; les groupes phényle, phényloxy, benzyloxy, pyridinyle, pyrazinyle, pyridazinyle, triazinyle et pyrimidinyle pouvant être substitués par un ou plusieurs substituants choisis parmi un atome d'halogène, un groupe cyano, nitro, C₁₋₆-alkyle, C₁₋₆-alcoxy, C₁₋₆-thioalkyle, C₁₋₆-haloalkyle, C₁₋₆-haloalcoxy, C₁₋₆-halothioalkyle, C₃₋₇-cycloalkyle, C₃₋₇-cycloalkyle-C₁₋₃-alkylène ;
R₈ et R₉ représentent indépendamment l'un de l'autre un atome d'hydrogène ou un groupe C₁₋₆-alkyle,
ou forment avec le ou les atomes qui les portent,
dans le cas de NR₈R₉, un cycle choisi parmi les cycles azétidine, pyrrolidine, pipéridine, morpholine, thiomorpholine, azépine, oxazépine ou pipérazine, ce cycle étant éventuellement substitué par un groupe C₁₋₆-alkyle ou benzyle ;
dans le cas de NR₈COR₉, un cycle lactame ; dans le cas de NR₈CO₂R₉, un cycle oxazolidinone, oxazinone ou oxazépinone; dans le cas de NR₈SO₂R₉, un cycle sultame ; dans le cas de NR₈SO₂NR₈R₉, un cycle dioxyde de thiazolidine ou dioxyde de thiadiazinane ;
R₁₀ et R₁₁ représentent indépendamment l'un de l'autre un atome d'hydrogéne ou un groupe C₁₋₆-alkyle ;
à l'état de base ou de sel d'addition à un acide.

2. Composé de formule (I) selon la revendication 1 **caractérisé en ce que** R₂ représente un atome d'hydrogène ;
à l'état de base ou de sel d'addition à un acide.

3. Composé de formule (I) selon la revendication 1 ou 2 **caractérisé en ce que** le groupe 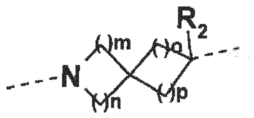 représente 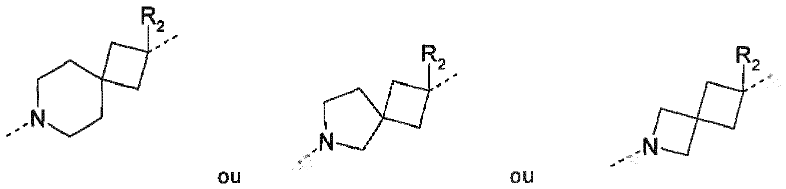 R₂ étant tel que défini dans la formule générale (I) selon la revendication 1 ;
à l'état de base ou de sel d'addition à un acide.

4. Composé de formule (I) selon l'une quelconque des revendications 1 à 3 **caractérisé en ce que** A représente une liaison covalente ou un groupe C₁₋₈-alkylène ; à l'état de base ou de sel d'addition à un acide.

5. Composé de formule (I) selon l'une quelconque des revendications 1 à 4 **caractérisé en ce que**
R₁ représente un groupe R₅ éventuellement substitué par un ou plusieurs groupes R₆ et/ou R₇ ;
R₅ représente un groupe pyrimidinyle, pyrazinyle, pyridinyle ou quinolinyle ;
R₆ représente un atome d'halogène, un groupe C₁₋₆-haloalkyle ou un groupe C₁₋₆-alkyle;
R₇ représente un phényle pouvant être substitué par un ou plusieurs groupes R₆ identiques ou différents l'un de l'autre.
à l'état de base ou de sel d'addition à un acide.

6. Composé de formule (I) selon l'une quelconque des revendications 1 à 5 **caractérisé en ce que** R₃ représente un atome d'hydrogène; à l'état de base ou de sel d'addition à un acide.

7. Composé de formule (I) selon l'une quelconque des revendications 1 à 6 **caractérisé en ce que**
R₄ représente un groupe choisi parmi un thiazolyle, un oxazolyle, un oxadiazolyle, un isoxazolyle;
ce groupe étant éventuellement substitué par un ou plusieurs substituants choisis parmi un groupe C₁₋₆-alkyle, CONR₈R₉, CON(R₈) (C₁₋₃-alkylène-NR₁₀R₁₁) ou un phényle ; le groupe phényle étant éventuellement substitué par un ou plusieurs substituants choisis parmi un atome d'halogène ;
R₈, R₉, R₁₀ et **R₁₁** représentent indépendamment l'un de l'autre un atome d'hydrogène ou un groupe C₁₋₆-alkyle ;
à l'état de base ou de sel d'addition à un acide.

8. Composé de formule (I) selon l'une quelconque des revendications 1 à 7 **caractérisé en ce qu'**il est choisi parmi :
[7-((6-Fluoro-quinolin-2-yl)-7-aza-spiro[3.5]non-2-yl]-carbamate de thiazol-4-ylméthyle ;
[7-((6-Fluoro-quinolin-2-yl)-7-aza-spiro[3.5]non-2-yl]-carbamate de 3-carbamoyl-isoxazol-5-ylméthyle ;
[7-((5-Trifluorométhyl-pyridin-2-yl)-7-aza-spiro[3.5]non-2-yl]-carbamate de 3-carbamoyl-isoxazol-5-ylméthyle ;
[7-((5-Bromo-pyridin-2-yl)-7-aza-spiro[3.5]non-2-yl]-carbamate de 3-carbamoyl-isoxazol-5-ylméthyle ;
{7-[5-(4-Fluoro-phényl)-pyridin-2-yl]-7-aza-spiro[3.5]non-2-yl}-carbamate de 3-carbamoyl-isoxazol-5-ylméthyle ;
[7-((6-Fluoro-quinolin-2-yl)-7-aza-spiro[3.5]non-2-yl]-méthylcarbamate de 3-carbamoyl-isoxazol-5-ylméthyle ;
[7-((6-Fluoro-quinolin-2-yl)-7-aza-spiro[3.5]non-2-yl]-méthylcarbamate de 3-(méthylcarbamoyl)-isoxazol-5-ylméthyle ;
{7-[5-(4-Fluoro-phényl)-pyridin-2-yl]-7-aza-spiro[3.5]non-2-yl}-carbamate de 3-(méthylcarbamoyl)-isoxazol-5-ylméthyle ;
[7-((6-Fluoro-quinolin-2-yl)-7-aza-spiro[3.5]non-2-yl]-carbamate de 3-(méthylcarbamoyl)-isoxazol-5-ylméthyle ;
{6-[5-(4-Fluoro-phényl)-pyridin-2-yl]-6-aza-spiro[3.4]oct-2-yl}-carbamate de 3-carbamoyl-isoxazol-5-ylméthyle ;
{6-[5-(4-Fluoro-phényl)-pyridin-2-yl]-6-aza-spiro[3.4]oct-2-yl}-carbamate de 3-(méthylcarbamoyl)-isoxazol-5-ylméthyle ;
{6-[5-(4-Fluoro-phényl)-pyridin-2-yl]-6-aza-spiro[3.4]oct-2-ylméthyl}carbamate de 3-(méthylcarbamoyl)-isoxazol-5-ylméthyle ;
{6-[5-(4-Fluoro-phényl)-pyridin-2-yl]-6-aza-spiro[3.4]oct-2-ylméthyl}carbamate de 3-(méthylcarbamoyl)-isoxazol-5-ylméthyle ;
[7-((6-Fluoro-quinolin-2-yl)-7-aza-spiro[3.5]non-2-yl]-carbamate de 4-carbamoyl-oxazol-2-ylméthyle ;
[2-((6-Fluoro-quinolin-2-yl)-2-aza-spiro[3.3]hept-6-yl]-carbamate de 3-(méthylcarbamoyl)-isoxazol-5-ylméthyle ;
[6-((6-Fluoro-quinolin-2-yl)-6-aza-spiro[3.4]oct-2-ylméthyl]-carbamate de 3-(méthylcarbamoyl)-isoxazol-5-ylméthyle ;
{6-[5-(4-Fluoro-phényl)-pyridin-2-yl]-6-aza-spiro[3.4]oct-2-yl}-carbamate de 3-(méthylcarbamoyl)-isoxazol-5-ylméthyle ;
{6-[5-(4-Fluoro-phényl)-pyridin-2-yl]-6-aza-apiro[3.4]oct-2-yl}-carbamate de 3-(méthylcarbamoyl)-isoxazol-5-ylméthyle ;
[7-((6-Chloro-quinolin-2-yl)-7-aza-spiro[3.5]non-2-yl]-carbamate de 3-(méthylcarbamoyl)-isoxazol-5-ylméthyle ;
{2-[5-(4-Fluoro-phényl)-pyridin-2-yl]-2-aza-spiro[3.3]hept-6-yl}-carbamate de 3-carbamoyl-isoxazol-5-ylméthyle ;
[6-((5-Bromo-pyridin-2-yl)-6-aza-spiro[3.4]oct-2-yl]-carbamate de 3-méthylcarbamoyl-isoxazol-5-ylméthyle ;
[6-((4-Trifluorométhyl-pyridin-2-yl)-6-aza-spiro[3.4]oct-2-yl]-carbamate de 3-méthylcarbamoyl-isoxazol-5-ylméthyle ;
{2-[5-(4-Fluoro-phényl)-pyridin-2-yl]-2-aza-spiro[3.3]hept-6-yl}-carbamate de 3-méthylcarbamoyl-isoxazol-5-ylméthyle ;
[7-((4-Trifluorométhyl-pyrimidin-2-yl)-7-aza-spiro[3.5]non-2-yl]-carbamate de 3-carbamoyl-isoxazol-5-ylméthyle ;
{7-[6-(4-Fluoro-phényl)-pyrazin-2-yl]-7-aza-spiro[3.5]non-2-yl}-carbamate de 3-carbamoyl-isoxazol-5-ylméthyle ;
[7-((4-trifluorométhyl-pyrimidin-2-yl)-7-aza-spiro[3.5]non-2-yl]-carbamate de 3-(2-diméthylamino-éthylcarbamoyl)-isoxazol-5-ylméthyle et son chlorhydrate ;
{7-[4-(4-Fluoro-phényl)-pyridin-2-yl]-7-aza-spiro[3.5]non-2-yl}-carbamate de 3-carbamoyl-isoxazol-5-ylméthyle ;
[7-((4-Chloro-pyridin-2-yl)-7-aza-spiro[3.5]non-2-yl]-carbamate de 3-carbamoyl-isoxazol-5-ylméthyle ;
[7-((4-Trifluorométhyl-pyridin-2-yl)-7-aza-spiro[3.5]non-2-yl]-carbamate de 3-carbamoyl-isoxazol-5-ylméthyle ;
{7-[5-(3-Fluoro-phényl)-pyridin-2-yl]-7-aza-apiro[3.5]non-2-yl}-carbamate de 3-carbamoyl-isoxazol-5-ylméthyle ;
[7-((5-Isobutyl-pyridin-2-yl)-7-aza-spiro[3.5]non-2-yl]-carbamate de 3-carbamoyl-isoxazol-5-ylméthyle ;
[7-((6-Chloro-quinolin-2-yl)-7-aza-spiro[3.5]non-2-yl]-carbamate de 3-carbamoyl-isoxazol-5-ylméthyle ;
[7-((6-Chloro-quinolin-2-yl)-7-aza-spiro[3.5]non-2-ylméthyl]-carbamate de 3-méthylcarbamoyl-isoxazol-5-ylméthyle ;
[7-((4-Trifluorométhyl-pyridin-2-yl)-7-aza-spiro[3.5]non-2-yl]-carbamate de 3-méthylcarbamoyl-isoxazol-5-ylméthyle ;
[7-((4-Trifluorométhyl-pyridin-2-yl)-7-aza-spiro[3.5]non-2-yl]-carbamate de 3-(4-fluoro-phényl)-[1,2,4]oxadiazol-5-ylméthyle ;
[7-((4-Trifluorométhyl-pyridin-2-yl)-7-aza-spiro[3.5]non-2-yl]-carbamate de 4-carbamoyl-oxazol-2-ylméthyle ;
[7-((4-Trifluorométhyl-pyridin-2-yl)-7-aza-spiro[3.5]non-2-yl]-carbamate de 5-méthyl-3-phényl-isoxazol-4-ylméthyle ;
[7-((4-Trifluorométhyl-pyridin-2-yl)-7-aza-spiro[3.5]non-2-yl]-carbamate de 3-éthyl-[1,2,4]oxadiazol-5-ylméthyle ;
[7-((4-Trifluoroméhyl-pyridin-2-yl)-7-aza-apiro[3.5]non-2-yl]-carbamate de 5-méthyl-[1,2,4]oxadiazol-3-ylméthyle ;
[6-((4-Trifluorométhyl-pyrimidin-2-yl)-6-aza-spiro[2.5]oct-1-yl]-carbamate de 3-carbamoyl-isoxazol-5-ylméthyle ;
[6-((4-Trifluorométhyl-pyrimidin-2-yl)-6-aza-spiro[2.5]oct-1-yl]-carbamate de 3-méthylcarbamoyl-isoxazol-5-ylméthyle.

9. Procédé de préparation d'un composé de formule (I) selon l'une quelconque des revendications 1 à 8, comprenant l'étape consistant à
faire réagir une amine de formule générale (II), 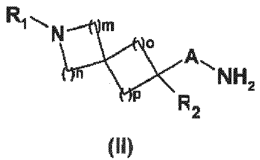 dans laquelle A, R₁, R2, m, n, o et p sont tels que définis dans la formule générale (I) selon la revendication 1,
soit avec un carbonate de formule générale (III) 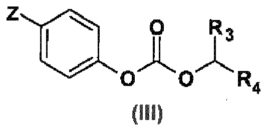 dans laquelle Z représente un atome d'hydrogène ou un groupe nitro, R₃ et R₄ sont tels que définis dans la formule générale (I) selon la revendication 1.
en présence d'une base, dans un solvant à une température comprise entre la température ambiante et la température de reflux du solvant ;
soit avec le chloroformiate de phényle ou de 4-nitro-phényle, en présence d'une base, dans un solvant à une température comprise entre 0°C et la température ambiante,
pour conduire au dérivé carbamate de formule générale (IV), 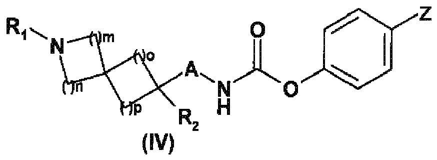 dans laquelle A, R₁, R₂, m, n, o et p sont tels que définis dans la formule générale (I) selon la revendication 1, et Z représente un atome d'hydrogène ou un groupe nitro,
puis à transformer le dérivé carbamate de formule générale (IV) ainsi obtenu en composé de formule générale (I), par action d'un alcool de formule générale HOCHR₃R₄ (IIIa), dans laquelle R₃ et R₄ sont tels que définis dans la formule générale (I) selon la revendication 1,
en présence d'une base, dans un solvant à une température comprise entre la température ambiante et la température de reflux du solvant.

10. Procédé de préparation d'un composé de formule (I) selon l'une quelconque des revendications 1 à 8, comprenant l'étape consistant à
faire réagir un composé de formule générale (Ia) 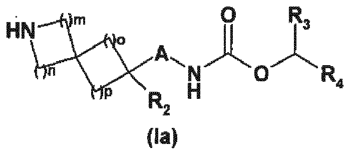 dans laquelle A, R₂, R₃, R₄, m, n, o et p sont tels que définis dans la formule générale (I) selon la revendication 1,
avec un dérivé de formule générale R₁-U (V), dans laquelle R₁ est tel que défini dans la formule générale (I) selon la revendication 1 et U₁ représente un atome d'halogène ou un groupe o-triflate,
en utilisant les conditions de réactions de substitution nucléophile aromatique ou hétéroaromatique ou en utilisant les conditions de *N*-arylation ou *N*-hétéroarylation de Buchwald.

11. Procédé de préparation d'un composé de formule (I) selon l'une quelconque des revendications 1 à 8, dans laquelle R₁ représente un groupe R₅ substitué notamment par un groupe R₆ de type C₁₋₆-alkyle, C₃₋₇-cycloalkyle ou C₃₋₇-cycloalkyle-C₁₋₃-alkylène, ou par un groupe R₇ tel que défini dans la formule générale (I) selon la revendication 1, comprenant l'étape consistant à
réaliser une réaction de couplage, 1 catalysée au moyen d'un métal de transition, sur le composé de formule générale (Ib). 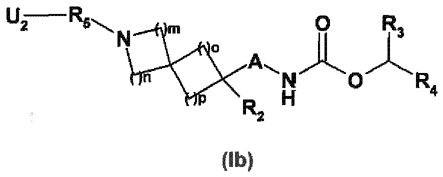 dans laquelle A, R₂, R₃, R₄, R₅, m, n, o et p sont tels que définis dans la formule générale (I) selon la revendication 1 et U2 représente un atome de chlore, de brome, d'iode ou un groupement triflate, U₂ étant dans la position où l'on souhaite introduire le groupe R₆ ou R₇:
- soit par une réaction de type Suzuki,
- soit selon une réaction de type Stille ;
- soit par une réaction de type Négishi.

12. Composé de formule (I) selon l'une quelconque des revendications 1 à 8, à l'état de base ou de sel d'addition à un acide pharmaceutiquement acceptable, pour son utilisation comme médicament.

13. Composition pharmaceutique contenant au moins un composé de formule (I) selon l'une quelconque des revendications 1 à 7, à l'état de base ou de sel d'addition à un acide pharmaceutiquement acceptable et éventuellement un ou plusieurs excipients pharmaceutiquement acceptables.

14. Utilisation d'un composé de formule (I) selon l'une quelconque des revendications 1 à 8, à l'état de base ou de sel d'addition à un acide pharmaceutiquement acceptable, pour la préparation d'un médicament destiné à prévenir ou à traiter les douleurs aiguës ou chroniques, les vertiges, les vomissements, les nausées, les troubles du comportement alimentaire, les pathologies neurologiques et psychiatriques, les maladies neuro-dégénératives aiguës ou chroniques, l'épilepsie, les troubles du sommeil, les maladies cardiovasculaires, l'ischémie rénale, les cancers, les désordres du système immunitaire, les maladies allergiques, les maladies infectieuses parasitaires virales ou bactériennes, les maladies inflammatoires, l'ostéoporose, les affections oculaires, les affections pulmonaires, les maladies gastro-intestinales ou l'incontinence urinaire,

## Patentansprüche

1. Verbindung der Formel (I), 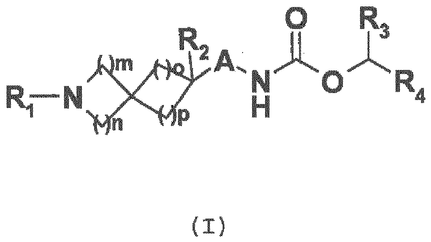 worin
R₂ für ein Wasserstoff- oder Fluoratom oder eine Hydroxyl-, Cyano-, Trifluormethyl-, C₁-C₆-Alkyl-, C₁-C₆-Alkoxy- oder NR₈R₉-Gruppe steht;
m, n, o und p unabhängig voneinander für eine ganze Zahl mit einem Wert von 0, 1, 2 oder 3 stehen;
A für eine kovalente Bindung oder eine C₁₋₈-Alkylengruppe steht;
R₁ für eine Gruppe R₅ steht, die gegebenenfalls durch eine oder mehrere Gruppen R₆ und/oder R₇ substituiert ist;
R₅ für eine Gruppe steht, die aus Phenyl, Pyridinyl, Pyridazinyl, Pyrimidinyl, Pyrazinyl, Triazinyl, Naphthalinyl, Chinolinyl, Isochinolinyl, Phthalazinyl, Chinazolinyl, Chinoxalinyl, Cinnolinyl Naphthiridinyl ausgewählt ist;
R₆ für ein Halogenatom oder eine Cyano-, -CH₂CN-, Nitro-, Hydroxyl-, C₁-C₆-Alkyl-, C₁-C₆-Alkoxy-, C₁-C₆-Thioalkyl-, C₁-C₆-Halogenalkyl-, C₁-C₆-Halogen-alkoxy-, C₁-C₆-Halogenthioalkyl-, C₃-C₇-Cycloalkyl-, C₃-C₇-Cycloalkyl-C₁-C₃-alkylen-, C₃-C₇-Cycloalkyl-C₁-C₃-alkylen-O-, NR₈R₉-, NR₈COR₉-, NR₉CO₂R₉-, NR₈SO₂R₉-, NR₈SO₂NR₈R₉-, COR₈-, CO₂R₈-, CONR₈R₉-, SO₂R₈-, SO₂NR₈R₉- oder -O-(C₁-C₃-Alkyen) -O-Gruppe steht;
R₇ für eine Gruppe steht, die aus Furyl, Pyrrolyl, Thienyl, Oxazolyl, Isoxazolyl, Thiazolyl, Isothiazolyl, Imidazolyl, Pyrazolyl, Oxadiazolyl, Thiadiazolyl, Phenyl, Pyridinyl, Pyridazinyl, Pyrimidinyl, Pyrazinyl, Triazin, Naphthalinyl, Chinolinyl, Isochinolinyl, Phthalazinyl, Chinazolinyl, Chinoxalinyl, Cinnolinyl, Naphthyridinyl, Imidazopyrimidinyl, Thienopyrimidinyl, Benzofuryl, Benzothienyl, Benzimidazolyl, Benzoxazolyl, Benzisoxazolyl, Benzothiazolyl, Benzisothiazolyl, Indolyl, Isoindolyl, Indazolyl, Pyrrolopyridinyl, Furopyridinyl, Thienopyridinyl, Imidazopyridinyl, Pyrazolopyridinyl, Oxazolopyridinyl, Isoxazolopyridinyl, Thiazolopyridinyl, Phenyloxy, Benzyloxy; Pyrimidinoxy ausgewählt ist;
wobei die Gruppe R₇ bzw. die Gruppen R₇ durch ein oder mehrere gleiche oder voneinander verschiedene Gruppen R₆ substituiert sein kann bzw. sein können;
R₃ für ein Wasserstoff- oder Fluoratom, eine C₁₋₆-Alkylgruppe oder eine Trifluormethylgruppe steht;
R₄ für eine Gruppe steht, die aus Furyl, Pyrrolyl, Thienyl, Thiazolyl, Isothiazolyl, Oxazolyl, Isoxazolyl, Pyrazolyl, Oxadiazolyl, Thiadiazolyl, Imidazolyl, Triazolyl und Tetrazolyl ausgewählt ist;
wobei diese Gruppe gegebenenfalls durch einen oder mehrere Substituenten substituiert ist, die aus einem Halogenatom oder einer C₁₋₆-Alkyl-, C₁₋₆-Halogenalkyl-, C₃₋₇-Cycloalkyl-, C₃₋₇-Cycloalkyl-C₁₋₃-alkylen-, C₁₋₆-Halogenalkoxy-, Cyano-, NR₈R₉-, NR₈COR₉-, NR₈CO₂R₉ NR₈SO₂R₉-, NR₈SO₂NR₉R₉-, COR₈-, CO₂R₈-, CONR₈R₉- CON(R₈) (C₁₋₃Alkylen-NR₁₀R₁₁) -, SO₂R₈-, SO₂NR₈R₉-, -O-(C₁₋₃-Alkylen)-O-, Phenyl-, Phenyloxy-, Benzyloxy-, Pyridinyl-, Pyrazinyl-, Pyridazinyl-, Triazinyl- oder Pyrimidinylgruppe ausgewählt sind; wobei die Phenyl-, Phenyloxy-, Benzyloxy-, Pyridinyl-, Pyrazinyl-, Pyridazinyl-, Triazinyl- und Pyrimidinylgruppen gegebenenfalls durch einen oder mehrere Substituenten substituiert sind, die aus einem Halogenatom und einer Cyano- , Nitro-, C₁₋₆-Alkyl-, C₁₋₆-Alkoxy-, C₁₋₆-Thioalkyl-, C₁₋₆-Halogenalkyl-, C₁₋₆-Halogen-alkoxy-, C₁₋₆-Halogenthioalkyl-, C₃₋₇-Cycloalkyl-, C₃₋₇-Cycloalkyl-C₁₋₃-alkylengruppe ausgewählt sind;
R₈ und R₉ unabhängig voneinander für ein Wasserstoffatom oder eine C₁-C₆-Alkylgruppe stehen oder mit dem Atom bzw. den Atomen, an das bzw. die sie gebunden sind,
im Fall von NR₈R₉ einen Ring, der aus Azetidin-, Pyrrolidin-, Piperidin-, Morpholin-, Thiomorpholin-, Azepin-, Oxazepin- oder Piperazinringen ausgewählt ist, wobei dieser Ring gegebenenfalls durch eine C₁₋₆-Alkyl- oder Benzylgruppe substituiert ist;
im Fall von NR₈COR₉ einen Lactamring; im Fall von NR₈CO₂R₉ einen Oxazolidinon-, Oxazinon- oder Oxazepinonring; im Fall von NR₈SO₂R₉ einen Sultamring; im Fall von NR₈SO₂NR₉R₉ einen Thiazolidindioxid- oder Thiadiazinandioxidring bilden;
R₁₀ und R₁₁ unabhängig voneinander für ein Wasserstoffatom oder eine C₁-C₆-Alkylgruppe stehen;
in Basen- oder Säureadditionssalzform.

2. Verbindung der Formel (I) nach Anspruch 1, **dadurch gekennzeichnet, dass** R₂ für ein Wasserstoffatom steht;
in Basen- oder Säureadditionssalzform.

3. Verbindung der Formel (I) nach Anspruch 1 oder 2, **dadurch gekennzeichnet, dass** die Gruppe 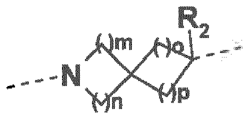 für 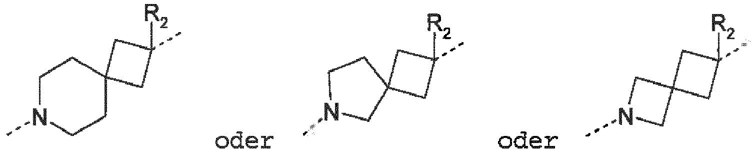 steht,
wobei R₂ wie in der allgemeinen Formel (I) nach Anspruch 1 definiert ist;
in Basen- oder Säureadditionssalzform.

4. Verbindung der Formel (I) nach einem der Ansprüche 1 bis 3, **dadurch gekennzeichnet, dass** A für eine kovalente Bindung oder eine C₁₋₈-Alkylengruppe steht;
in Basen- oder Säureadditionssalzform.

5. Verbindung der Formel (I) nach einem der Ansprüche 1 bis 4, **dadurch gekennzeichnet, dass**
R₁ für eine Gruppe R₅ steht, die gegebenenfalls durch eine oder mehrere Gruppen R₆ und/oder R₇ substituiert ist;
R₅ für eine Pyrimidinyl-, Pyrazinyl-, Pyridinyl- oder Chinolinylgruppe steht;
R₆ für ein Halogenatom, eine C₁-C₆-Halogenalkylgruppe oder eine C₁-C₆-Alkylgruppe steht;
R₇ für ein Phenyl steht, das durch eine oder mehrere gleiche oder voneinander verschiedene Gruppen R₆ substituiert sein kann;
in Basen- oder Säureadditionssalzform.

6. Verbindung der Formel (I) nach einem der Ansprüche 1 bis 5, **dadurch gekennzeichnet, dass** R₃ für ein Wasserstoffatom steht;
in Basen- oder Säureadditionssalzform.

7. Verbindung der Formel (I) nach einem der Ansprüche 1 bis 6, **dadurch gekennzeichnet, dass** R₄ für eine Gruppe steht, die aus Thiazolyl, Oxazolyl, Oxadiazolyl und Isoxazolyl ausgewählt ist;
wobei diese Gruppe gegebenenfalls durch einen oder mehrere Substituenten substituiert ist, die aus einer C₁₋₆-Alkyl-, CONR₈R₉- , CON(R₈) (C₁₋₃-Alkylen-NR₁₀R₁₁) - oder Phenylgruppe ausgewählt sind; wobei die Phenylgruppe gegebenenfalls durch einen oder mehrere Substituenten substituiert sein kann, die aus einem Halogenatom ausgewählt sind;
R₈, R₉, R₁₀ und R₁₁ unabhängig voneinander für ein Wasserstoffatom oder eine C₁-C₆-Alkylgruppe stehen;
in Basen- oder Säureadditionssalzform.

8. Verbindung der Formel (I) nach einem der Ansprüche 1 bis 7, **dadurch gekennzeichnet, dass** sie ausgewählt ist aus:
[7-((6-Fluorchinolin-2-yl)-7-azaspiro[3.5]non-2-yl]carbamidsäurethiazol-4-ylmethylester;
[7-((6-Fluorchinolin-2-yl)-7-azaspiro[3.5]non-2-yl]carbamidsäure-3-carbamoylisoxazol-5-ylmethylester;
[7-((5-Trifluormethylpyridin-2-yl)-7-azaspiro[3.5]non-2-yl]carbamidsäure-3-carbamoylisoxazol-5-ylmethylester;
[7-((5-Brompyridin-2-yl)-7-azaspiro[3.5]non-2-yl]-carbamidsäure-3-carbamoylisoxazol-5-ylmethylester;
{7-[5-(4-Fluorphenyl)pyridin-2-yl] -7-azaspiro[3.5]non-2-yl}carbamidsäure-3-carbamoylisoxazol-5-ylmethylester;
[7-((6-Fluorchinolin-2-yl)-7-azaspiro[3.5]non-2-yl]methylcarbamidsäure-3-carbamoylisoxazol-5-ylmethylester;
[7-((6-Fluorchinolin-2-yl)-7-azaspiro[3.5]non-2-yl]methylcarbamidsäure-3-(methylcarbamoyl)-isoxazol-5-ylmethylester;
{7-[5-(4-Fluorphenyl)pyridin-2-yl]-7-azaspiro[3.5]non-2-yl}carbamidsäure-3-(methylcarbamoyl)isoxazol-5-ylmethylester;
[7-((6-Fluorchinolin-2-yl)-7-azaspiro[3.5]non-2-yl]carbamidsäure-3-(methylcarbamoyl)isoxazol-5-ylmethylester;
{6-[5-(4-Fluorphenyl)pyridin-2-yl]-6-azaspiro[3.4]oct-2-yl}carbamidsäure-3-carbamoylisoxazol-5-ylmethylester;
{6-[5-(4-Fluorphenyl)pyridin-2-yl]-6-azaspiro[3.4]oct-2-yl}carbamidsäure-3-(methylcarbamoyl)isoxazol-5-ylmethylester;
{6-[5-(4-Fluorphenyl)pyridin-2-yl]-6-azaspiro[3.4]oct-2-ylmethyl}carbamidsäure-3-(methylcarbamoyl)isoxazol-5-ylmethylester;
{6-[5-(4-Fluorphenyl)pyridin-2-yl]-6-azaspiro[3.4]oct-2-ylmethyl}carbamidsäure-3-(methylcarbamoyl)isoxazol-5-ylmethylester;
[7-((5-Fluorchinolin-2-yl)-7-azaspiro[3.5]non-2-yl]carbamidsäure-4-carbamoyloxazol-2-ylmethylester;
[2-((5-Fluorchinolin-2-yl)-2-azaspiro[3.3]hept-6-yl]carbamidsäure-3-(methylcarbamoyl)isoxazol-5-ylmethylester;
[6-((6-Fluorchinolin-2-yl)-6-azaspiro[3.4]oct-2-ylmethyl]carbamidsäure-3-(methylcarbamoyl)isoxazol-5-ylmethylester;
{6-[5-(4-Fluorphenyl)pyridin-2-yl]-6-azaspiro[3.4]oct-2-yl}carbamidsäure-3-(methylcarbamoyl)isoxazol-5-ylmethylester;
{6-[5-(4-Fluorphenyl)pyridin-2-yl]-6-azaspiro[3.4]oct-2-yl}carbamidsäure-3-(methylcarbamoyl)isoxazol-5-ylmethylester;
[7-((6-Chlorchinolin-2-yl)-7-azaspiro[3.5]non-2-yl]carbamidsäure-3-(methylcarbamoyl)isoxazol-5-ylmethylester;
{2-[5-(4-Fluorphenyl)pyridin-2-yl]-2-azaspiro[3.3]hept-6-yl}carbamidsäure-3-carbamoylisoxazol-5-ylmethylester;
[6-((5-Brompyridin-2-yl)-6-azaspiro[3.4]oct-2-yl]-carbamidsäure-3-methylcarbamoylisoxazol-5-ylmethylester;
[6-((4-Trifluormethylpyridin-2-yl)-6-azaspiro[3.4]oct-2-yl]carbamidsäure-3-methylcarbamoylisoxazol-5-ylmethylester;
{2-[5-(4-Fluorphenyl)pyridin-2-yl]-2-azaspiro[3.3]hept-6-yl}carbamidsäure-3-methylcarbamoylisoxazol-5-ylmethylester;
[7-((4-Trifluormethylpyrimidin-2-yl)-7-azaspiro[3.5]non-2-yl]carbamidsäure-3-carbamoylisoxazol-5-ylmethylester;
{7-[6-(4-Fluorphenyl)pyrazin-2-yl]-7-azaspiro[3.5]non-2-yl}carbamidsäure-3-carbamoylisoxazol-5-ylmethylester;
[7-((4-Trifluormethylpyrimidin-2-yl)-7-azaspiro[3.5]non-2-yl]carbamidsäure-3-(2-dimethylaminoethylcarbamoyl)isoxazol-5-ylmethylester und das Hydrochlorid davon;
{7-[4-(4-Fluorphenyl)pyridin-2-yl]-7-azaspiro[3.5]non-2-yl}carbamidsäure-3-carbamoylisoxazol-5-ylmethylester;
[7-((4-Chlorpyridin-2-yl)-7-azaspiro[3.5]non-2-yl]-carbamidsäure-3-carbamoylisoxazol-5-ylmethylester;
[7-((4-Trifluormethylpyridin-2-yl)-7-azaspiro[3.5]non-2-yl]carbamidsäure-3-carbamoylisoxazol-5-ylmethylester;
{7-[5-(3-Fluorphenyl)pyridin-2-yl]-7-azaspiro[3.5]non-2-yl}carbamidsäure-3-carbamoylisoxazol-5-ylmethylester;
[7-((5-Isobutylpyridin-2-yl)-7-azaspiro[3.5]non-2-yl]carbamidsäure-3-carbamoylisoxazol-5-ylmethylester;
[7-((6-Chlorchinolin-2-yl)-7-azaspiro[3.5]non-2-yl]carbamidsäure-3-carbamoylisoxazol-5-ylmethylester;
[7-((6-Chlorchinolin-2-yl)-7-azaspiro[3.5]non-2-ylmethyl]carbamidsäure-3-methylcarbamoylisoxazol-5-ylmethylester;
[7-((4-Trifluormethylpyridin-2-yl)-7-azaspiro[3.5]non-2-yl]carbamidsäure-3-methylcarbamoylisoxazol-5-ylmethylester;
[7-((4-Trifluormethylpyridin-2-yl)-7-azaspiro[3.5]non-2-yl]carbamidsäure-3-(4-fluorphenyl)-[1,2,4]oxadiazol-5-ylmethylester;
[7-((4-Trifluormethylpyridin-2-yl)-7-azaspiro[3.5]non-2-yl]carbamidsäure-4-carbamoyl-oxazol-2-ylmethylester;
[7-((4-Trifluormethylpyridin-2-yl)-7-azaspiro[3.5]non-2-yl]carbamidsäure-5-methyl-3-phenylisoxazol-4-ylmethylester;
[7-((4-Trifluormethylpyridin-2-yl)-7-azaspiro[3.5]non-2-yl]carbamidsäure-3-ethyl[1,2,4]oxadiazol-5-ylmethylester;
[7-((4-Trifluormethylpyridin-2-yl)-7-azaspiro[3.5]non-2-yl]carbamidsäure-5-methyl[1,2,4]oxadiazol-3-ylmethylester;
[6-((4-Trifluormethylpyrimidin-2-yl)-6-azaspiro[2.5]oct-1-yl]carbamidsäure-3-carbamoylisoxazol-5-ylmethylester;
[6-((4-Trifluormethylpyrimidin-2-yl)-5-azaspiro[2.5]oct-1-yl]carbamidsäure-3-methylcarbamoylisoxazol-5-ylmethylester.

9. Verfahren zur Herstellung einer Verbindung der Formel (I) nach einem der Ansprüche 1 bis 8, bei dem man
ein Amin der allgemeinen Formel (II), 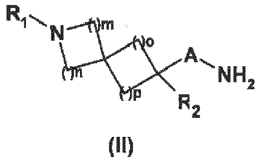 worin A, R₁, R₂, m, n, o und p wie in der allgemeinen Formel (I) nach Anspruch 1 definiert sind,
entweder in Gegenwart einer Base in einem Lösungsmittel bei einer Temperatur zwischen Umgebungstemperatur und der Rückflusstemperatur des Lösungsmittels mit einem Kohlensäureester der allgemeinen Formel (III), 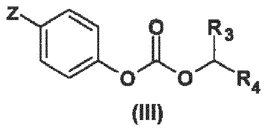 worin Z für ein Wasserstoffatom oder eine Nitrogruppe steht und R₃ und R₄ wie in der allgemeinen Formel (I) nach Anspruch 1 definiert sind;
oder in Gegenwart einer Base in einem Lösungsmittel bei einer Temperatur zwischen 0°C und Umgebungstemperatur mit Chlorameisensäurephenylester oder -4-nitrophenylester
zu einem Carbamidsäureesterderivat der allgemeinen Formel (IV), 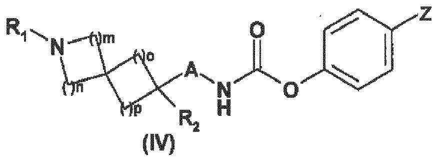 worin A, R₁, R₂, m, n, o und p wie in der allgemeinen Formel (I) nach Anspruch 1 definiert sind und Z für ein Wasserstoffatom oder eine Nitrogruppe steht,
umsetzt und dann das so erhaltene Carbamidsäureesterderivat der allgemeinen Formel (IV) durch Einwirkung eines Alkohols der allgemeinen Formel HOCHR₃R₄ (IIIa), worin R₃ und R₄ wie in der allgemeinen Formel (I) nach Anspruch 1 definiert sind,
in Gegenwart einer Base in einem Lösungsmittel bei einer Temperatur zwischen Umgebungstemperatur und der Rückflusstemperatur des Lösungsmittels in eine Verbindung der allgemeinen Formel (I) umwandelt.

10. Verfahren zur Herstellung einer Verbindung der Formel (I) nach einem der Ansprüche 1 bis 8, bei dem man
eine Verbindung der allgemeinen Formel (Ia), 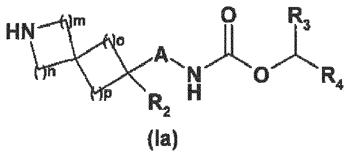 worin A, R₂, R₃, R₄, m, n, o und p wie in der allgemeinen Formel (I) nach Anspruch 1 definiert sind,
unter Verwendung von Bedingungen für aromatische oder heteroaromatische nucleophile Substitutionsreaktionen oder unter Verwendung von Bedingungen für die Buchwald-N-Arylierung oder -N-Heteroarylierung mit einem Derivat der allgemeinen Formel R₁-U (V), worin R₁ wie in der allgemeinen Formel (I) nach Anspruch 1 definiert ist und U₁ für ein Halogenatom oder eine O-Triflat-Gruppe steht,
umsetzt.

11. Verfahren zur Herstellung einer Verbindung der Formel (I) nach einem der Ansprüche 1 bis 8, worin R₁ für eine Gruppe R₅ steht, die insbesondere durch eine Gruppe R₆ vom C₁₋₆-Alkyl-, C₃₋₇-Cycloalkyl- oder C₃₋₇-Cycloalkyl-C₁₋₃-alkylen-Typ oder durch eine wie in der allgemeinen Formel (I) nach Anspruch 1 definierte Gruppe R₇ substituiert ist, bei dem man an einer Verbindung der allgemeinen Formel (Ib), 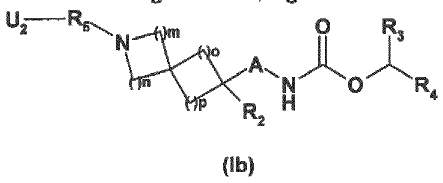 worin A, R₂, R₃, R₄, R₅, m, n, o und p wie in der allgemeinen Formel (I) nach Anspruch 1 definiert sind und U₂ für ein Chlor-; Brom-, Iodatom oder eine Triflatgruppe steht, wobei U₂ in der Position steht, in der die Gruppe R₆ oder R₇ eingeführt werden soll,
eine durch ein Übergangsmetall katalysierte Kupplungsreaktion durchführt; und zwar
- entweder durch eine Reaktion vom Suzuki-Typ
- oder gemäß einer Reaktion vom Stille-Typ
- oder durch eine Reaktion vom Negishi-Typ.

12. Verbindung der Formel (I) nach einem der Ansprüche 1 bis 8 in Basenform oder in Form eines Additions-salzes mit einer pharmazeutisch unbedenklichen Säure zur Verwendung als Arzneimittel.

13. Pharmazeutische Zusammensetzung, enthaltend mindestens eine Verbindung der Formel (I) nach einem der Ansprüche 1 bis 7 in Basenform oder in Form eines Additionssalzes mit einer pharmazeutisch unbedenklichen Säure und gegebenenfalls einen oder mehrere pharmazeutisch unbedenkliche Hilfsstoffe.

14. Verwendung einer Verbindung der Formel (I) nach einem der Ansprüche 1 bis 8 in Basenform oder in Form eines Additionssalzes mit einer pharmazeutisch unbedenklichen Säure zur Herstellung eines Arzneimittels zur Prävention oder Behandlung von akuten oder chronischen Schmerzen, Schwindel, Erbrechen, Übelkeit, Essstörungen, neurologischen und psychiatrischen Pathologien, akuten oder chronischen neurodegenerativen Erkrankungen, Epilepsie, Schlafstörungen, Herz-Kreislauf-Erkrankungen, Nierenischämie, Krebs, Störungen des Immunsystems, allergischen Erkrankungen, parasitären, viralen oder bakteriellen Infektionserkrankungen, entzündlichen Erkrankungen, Osteoporose, Augenleiden, Lungenleiden, Magen-DarmErkrankungen oder Harninkontinenz.

## Claims

1. Compound corresponding to formula (I) 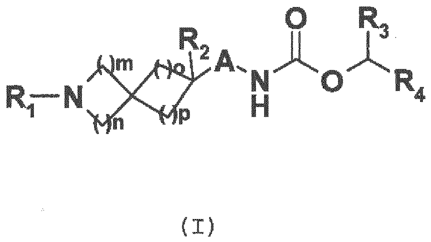 in which
R₂ represents a hydrogen or fluorine atom or a hydroxyl, cyano, trifluoromethyl, C₁₋₆-alkyl, C₁₋₆-alkoxy or NR₈R₉ group;
m, n, o and p represent, independently of each other, an integer equal to 0, 1, 2 or 3;
A represents a covalent bond or a group C₁₋₈-alkylene;
R₁ represents a group R₅ optionally substituted with one or more groups R₆ and/or R₇;
R₅ represents a group chosen from phenyl, pyridyl, pyridazinyl, pyrimidinyl, pyrazinyl, triazinyl, naphthyl, quinolinyl, isoquinolinyl, phthalazinyl, quinazolinyl, quinoxalinyl, cinnolinyl and naphthyridinyl;
R₆ represents a halogen atom or a cyano, -CH₂CN, nitro, hydroxy, C₁₋₆-alkyl, C₁₋₆-alkoxy, C₁₋₆-thioalkyl, C₁₋₆-haloalkyl, C₁₋₆-haloalkoxy, C₁₋₆-halothioalkyl, C₃₋₇-cycloalkyl, C₃₋₇-cycloalkyl-C₁₋₃-alkylene, C₃₋₇-cycloalkyl-C₁₋₃-alkylene-o-, NR₈R₉, NR₈COR₉, NR₈CO₂R₉, NR₈SO₂R₉, NR₈SO₂NR₈R₉, COR₈, CO₂R₈, CONR₈R₉, SO₂R₆, SO₂NR₈R₉ or -O-(C₁₋₃-alkylene)-O- group;
R₇ represents a group chosen from furyl, pyrrolyl, thienyl, oxazolyl, isoxazolyl, thiazolyl, isothiazolyl, imidazolyl, pyrazolyl, oxadiazolyl, thiadiazolyl, phenyl, pyridyl, pyridazinyl, pyrimidinyl, pyrazinyl, triazinyl, naphthyl, quinolinyl, isoquinolinyl, phthalazinyl, quinazolinyl, quinoxalinyl, cinnolinyl, naphthyridinyl, imidazopyrimidinyl, thienopyrimidinyl, benzofuryl, benzothienyl, benzimidazolyl, benzoxazolyl, benzisoxazolyl, benzothiazolyl, benzisothiazolyl, indolyl, isoindolyl, indazolyl, pyrrolopyridyl, furopyridyl, thienopyridyl, imidazopyridyl, pyrazolopyridyl, oxazolopyridyl, isoxazolopyridyl, thiazolopyridyl, phenyloxy, benzyloxy, pyrimidinoxy;
the group(s) R₇ possibly being substituted with one or more groups R₆ that may be identical to or different from each other;
R₃ represents a hydrogen or fluorine atom, a group C₁₋₆-alkyl or a trifluoromethyl group;
R₄ represents a group chosen from furyl, pyrrolyl, thienyl, thiazolyl, isothiazolyl, oxazolyl, isoxazolyl, pyrazolyl, oxadiazolyl, thiadiazolyl, imidazolyl, triazolyl and tetrazolyl;
this group being optionally substituted with one or more substituents chosen from a halogen atom, a group C₁₋₆-alkyl, C₁₋₆-haloalkyl, C₃₋₇-cycloalkyl, C₃₋₇-cycloalkyl-C₁₋₃-alkylene, C₁₋₆-haloalkoxy, cyano, NR₈R₉, NR₈COR₉, NR₈CO₂R₉, NR₈SO₂R₉, NR₈SO₂NR₈R₉, COR₈, CO₂R₈, CONR₈R₉, CON(R₈) (C₁₋₃-alkylene-NR₁₀R₁₁), SO₂R₈, SO₂NR₈R₉, -O-(C₁₋₃-alkylene)-O-, phenyl, phenyloxy, benzyloxy, pyridyl, pyrazinyl, pyridazinyl, triazinyl or pyrimidinyl; the phenyl, phenyloxy, benzyloxy, pyridyl, pyrazinyl, pyridazinyl, triazinyl and pyrimidinyl groups possibly being substituted with one or more substituents chosen from a halogen atom and a cyano, nitro, C₁₋₆-alkyl, C₁₋₆-alkoxy, C₁₋₆-thioalkyl, C₁₋₆-haloalkyl, C₁₋₆-haloalkoxy, C₁₋₆-halothioalkyl, C₃₋₇-cycloalkyl; C₃₋₇-cycloalkyl-C₁₋₃-alkylene group;
R₈ and R₉ represent, independently of each other, a hydrogen atom or a group C₁₋₆-alkyl,
or form, with the atom(s) that bear(s) them,
in the case of NR₈R₉, a ring chosen from azetidine, pyrrolidine, piperidine, morpholine, thiomorpholine, azepine, oxazepine or piperazine rings, this ring being optionally substituted with a group C₁₋₆-alkyl or benzyl;
in the case of NR₈COR₉, a lactam ring; in the case of NR₈CO₂R₉, a oxazolidinone, oxazinone or oxazepinone ring; in the case of NR₈SO₂R₉, a sultam ring; in the case of NR₈SO₂NR₈R₉, a thiazolidine dioxide or thiadiazinane dioxide ring;
R₁₀ and R₁₁ represent, independently of each other, a hydrogen atom or a group C₁₋₆-alkyl;
in the form of base or of acid-addition salt.

2. Compound of formula (I) according to Claim 1, **characterized in that** R₂ represents a hydrogen atom;
in the form of base or of acid-addition salt.

3. Compound of formula (I) according to Claim 1 or 2, **characterized in that** the group 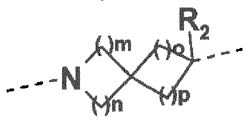 represents 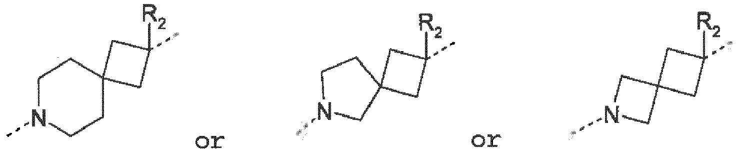 R₂ being as defined in the general formula (I) according to Claim 1;
in the form of base or of acid-addition salt.

4. Compound of formula (I) according to any one of Claims 1 to 3, **characterized in that** A represents a covalent bond or a group C₁₋₈-alkylene; in the form of base or of acid-addition salt.

5. Compound of formula (I) according to any one of Claims 1 to 4, **characterized in that**
R₁ represents a group R₅ optionally substituted with one or more groups R₆ and/or R₇;
R₅ represents a pyrimidinyl, pyrazinyl, pyridinyl or quinolinyl group;
R₆ represents a halogen atom, a group C₁₋₆-haloalkyl or a group C₁₋₆-alkyl;
R₇ represents a phenyl, which may be substituted with one or more groups R₆ that are identical to or different from each other.
in the form of base or of acid-addition salt.

6. Compound of formula (I) according to any one of Claims 1 to 5, **characterized in that** R₃ represents a hydrogen atom; in the form of base or of acid-addition salt.

7. Compound of formula (I) according to any one of Claims 1 to 6, **characterized in that**
R₄ represents a group chosen from a thiazolyl, an oxazolyl, an oxadiazolyl, and an isoxazolyl;
this group being optionally substituted with one or more substituents chosen from a group C₁₋₆-alkyl, CONR₈R₉, CON(R₈) (C₁₋₃-alkylene-NR₁₀R₁₁) or a phenyl; the phenyl group being optionally substituted with one or more substituents chosen from a halogen atom;
R₈, R₉, R₁₀ and R₁₁ represent, independently of each other, a hydrogen atom or a group C₁₋₆-alkyl;
in the form of base or of acid-addition salt.

8. Compound of formula (I) according to any one of Claims 1 to 7, **characterized in that** it is chosen from:
thiazol-4-ylmethyl [7-(6-fluoroquinolin-2-yl)-7-azaspiro[3.5]non-2-yl]carbamate;
3-carbamoylisoxazol-5-ylmethyl [7-(6-fluoroquinolin-2-yl)-7-azaspiro[3.5]non-2-yl]carbamate;
3-carbamoylisoxazol-5-ylmethyl [7-(5-trifluoromethyl-pyridin-2-yl)-7-azaspiro[3.5]non-2-yl]carbamate;
3-carbamoylisoxazol-5-ylmethyl [7-(5-bromopyridin-2-yl)-7-azaspiro[3.5]non-2-yl]carbamate;
3-carbamoylisoxazol-5-ylmethyl {7-[5-(4-fluorophenyl)-pyridin-2-yl]-7-azaspiro[3.5]non-2-yl}carbamate;
3-carbamoylisoxazol-5-ylmethyl [7-(6-fluoroquinolin-2-yl)-7-azaspiro[3.5]non-2-yl]methylcarbamate;
3-((methylcarbamoyl)isoxazol-5-ylmethyl [7-(6-fluoro-quinolin-2-yl)-7-azaspiro[3.5]non-2-yl]methylcarbamate;
3-((methylcarbamoyl)isoxazol-5-ylmethyl {7-[5-(4-fluoro-phenyl)pyridin-2-yl]-7-azaspiro[3.5]non-2-yl}carbamate;
3-((methylcarbamoyl)isoxazol-5-ylmethyl [7-(6-fluoro-quinolin-2-yl)-7-azaspiro[3.5]non-2-yl]carbamate;
3-carbamoylisoxazol-5-ylmethyl {6-[5-(4-fluorophenyl)-pyridin-2-yl]-6-azaspiro[3.4]oct-2-yl}carbamate;
3-((methylcarbamoyl)isoxazol-5-ylmethyl {6-[5-(4-fluoro-phenyl)pyridin-2-yl]-6-azaspiro[3.4]oct-2-yl}carbamate;
3-((methylcarbamoyl)isoxazol-5-ylmethyl {6-[5-(4-fluoro-phenyl)pyridin-2-yl]-6-azaspiro[3.4]oct-2-ylmethyl}carbamate;
3-((methylcarbamoyl)isoxazol-5-ylmethyl {6-[5-(4-fluoro-phenyl)pyridin-2-yl]-6-azaspiro[3.4]oct-2-ylmethyl}carbamate;
4-carbamoyloxazol-2-ylmethyl [7-(6-fluoroquinolin-2-yl)-7-azaspiro[3.5]non-2-yl]carbamate;
3-((methylcarbamoyl)isoxazol-5-ylmethyl [2-(6-fluoro-quinolin-2-yl)-2-azaspiro[3.3]hept-6-yl]carbamate;
3-((methylcarbamoyl)isoxazol-5-ylmethyl [6-(6-fluoro-quinolin-2-yl)-6-azaspiro[3.4]oct-2-ylmethyl]carbamate;
3-((methylcarbamoyl)isoxazol-5-ylmethyl {6-[5-(4-fluoro-phenyl)pyridin-2-yl]-6-azaspiro[3.4]oct-2-yl}carbamate;
3-((methylcarbamoyl)isoxazol-5-ylmethyl {6-[5-(4-fluoro-phenyl)pyridin-2-yl]-6-azaspiro[3.4]oct-2-yl}carbamate;
3-((methylcarbamoyl)isoxazol-5-ylmethyl [7-(6-chloro-quinolin-2-yl)-7-azaspiro[3.5]non-2-yl]carbamate;
3-carbamoylisoxazol-5-ylmethyl {2-[5-(4-fluorophenyl)-pyridin-2-yl]-2-azaspiro[3.3]hept-6-yl}carbamate;
3-methylcarbamoylisoxazol-5-ylmethyl [6-(5-bromopyridin-2-yl)-6-azaspiro[3.4]oct-2-yl]carbamate;
3-methylcarbamoylisoxazol-5-ylmethyl [6-(4-trifluoro-methylpyridin-2-yl)-6-azaspiro[3.4]oct-2-yl]carbamate;
3-methylcarbamoylisoxazol-5-ylmethyl {2-[5-(4-fluoro-phenyl)pyridin-2-yl]-2-azaspiro[3.3]hept-6-yl}carbamate;
3-carbamoylisoxazol-5-ylmethyl [7-(4-trifluoromethyl-pyrimidin-2-yl)-7-azaspiro[3.5]non-2-yl]carbamate;
3-carbamoylisoxazol-5-ylmethyl {7-[6-(4-fluorophenyl)-pyrazin-2-yl]-7-azaspiro[3.5]non-2-yl}carbamate;
3-((2-dimethylaminoethylcarbamoyl)isoxazol-5-ylmethyl [7-(4-trifluoromethylpyrimidin-2-yl)-7-azaspiro[3.5]non-2-yl]carbamate and the hydrochloride thereof;
3-carbamoylisoxazol-5-ylmethyl {7-[4-(4-fluorophenyl)-pyridin-2-yl]-7-azaspiro[3.5]non-2-yl}carbamate;
3-carbamoylisoxazol-5-ylmethyl [7-(4-chloropyridin-2-yl)-7-azaspiro[3.5]non-2-yl]carbamate;
3-carbamoylisoxazol-5-ylmethyl [7-(4-trifluoromethyl-pyridin-2-yl)-7-azaspiro[3.5]non-2-yl]carbamate;
3-carbamoylisoxazol-5-ylmethyl {7-[5-(3-fluorophenyl)-pyridin-2-yl]-7-azaspiro[3.5]non-2-yl}carbamate;
3-carbamoylisoxazol-5-ylmethyl [7-(5-isobutylpyridin-2-yl)-7-azaspiro[3.5]non-2-yl]carbamate;
3-carbamoylisoxazol-5-ylmethyl [7-(6-chloroquinolin-2-yl)-7-azaspiro[3.5]non-2-yl]carbamate;
3-methylcarbamoylisoxazol-5-ylmethyl [7-(6-chloro-quinolin-2-yl)-7-azaspiro[3.5]non-2-ylmethyl]carbamate;
3-methylcarbamoylisoxazol-5-ylmethyl [7-(4-trifluoro-methylpyridin-2-yl)-7-azaspiro[3.5]non-2-yl]carbamate;
3-((4-fluorophenyl)-[1,2,4]oxadiazol-5-ylmethyl [7-(4-trifluoromethylpyridin-2-yl)-7-azaspiro[3.5]non-2-yl]-carbamate;
4-carbamoyloxazol-2-ylmethyl [7-(4-trifluoromethyl-pyridin-2-yl)-7-azaspiro[3.5]non-2-yl]carbamate;
5-methyl-3-phenylisoxazol-4-ylmethyl [7-(4-trifluoro-methylpyridin-2-yl)-7-azaspiro[3.5]non-2-yl]carbamate;
3-ethyl[1,2,4]oxadiazol-5-ylmethyl [7-(4-trifluoro-methylpyridin-2-yl)-7-azaspiro[3.5]non-2-yl]carbamate;
5-methyl[1,2,4]oxadiazol-3-ylmethyl [7-(4-trifluoromethylpyridin-2-yl)-7-azaspiro[3.5]non-2-yl]-carbamate;
3-carbamoylisoxazol-5-ylmethyl [6-(4-trifluoromethyl-pyrimidin-2-yl)-6-azaspiro[2.5]oct-1-yl]carbamate;
3-methylcarbamoylisoxazol-5-ylmethyl [6-(4-trifluoro-methylpyrimidin-2-yl)-6-azaspiro[2.5]oct-1-yl]-carbamate.

9. Process for preparing a compound of formula (I) according to any one of Claims 1 to 8, comprising the step that consists in
reacting an amine of general formula (II), 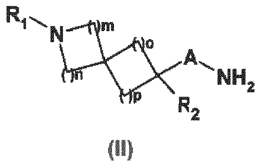 in which A, R₁, R₂, m, n, o and p are as defined in the general formula (I) according to Claim 1,
either with a carbonate of general formula (III) 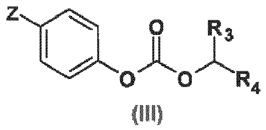 in which Z represents a hydrogen atom or a nitro group, and R₃ and R₄ are as defined in the general formula (I) according to Claim 1,
in the presence of a base, in a solvent at a temperature of between room temperature and the reflux temperature of the solvent;
or with phenyl or 4-nitrophenyl chloroformate,
in the presence of a base, in a solvent at a temperature of between 0°C and room temperature,
to give the carbamate derivative of general formula (IV), 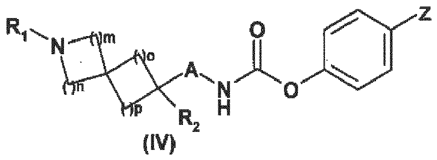 in which A, R₁, R₂, m, n, o and p are as defined in the general formula (I) according to Claim 1, and Z represents a hydrogen atom or a nitro group,
and then in converting the carbamate derivative of general formula (IV) thus obtained into a compound of general formula (I), via the action of an alcohol of general formula HOCHR₃R₄ (IIIa), in which R₃ and R₄ are as defined in the general formula (I) according to Claim 1, in the presence of a base, in a solvent at a temperature of between room temperature and the reflux temperature of the solvent.

10. Process for preparing a compound of formula (I) according to any one of Claims 1 to 8, comprising the step that consists in
reacting a compound of general formula (Ia) 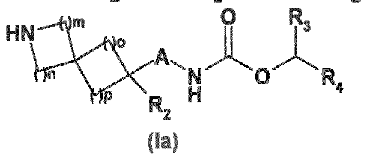 in which A, R₂, R₃, R₄, m, n, o and p are as defined in the general formula (I) according to Claim 1,
with a derivative of general formula R₁-U (V), in which R₁ is as defined in the general formula (I) according to Claim 1 and U₁ represents a halogen atom or an O-triflate group,
using aromatic or heteroaromatic nucleophilic substitution conditions or using Buchwald N-arylation or N-heteroarylation conditions.

11. Process for preparing a compound of formula (I) according to any one of Claims 1 to 8, in which R₁ represents a group R₅ substituted especially with a group R₆ of the type C₁₋₆-alkyl, C₃₋₇-cycloalkyl or C₃₋₇-cycloalkyl-C₁₋₃-alkylene, or with a group R₇ as defined in the general formula (I) according to Claim 1, comprising the step that consists in
performing a coupling reaction, catalysed by means of a transition metal, on the compound of general formula (Ib), 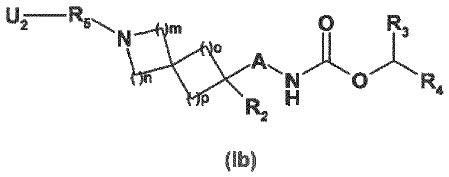 in which A, R₂, R₃, R₄, R₅, m, n, o and p are as defined in the general formula (I) according to Claim 1 and U₂ represents a chlorine, bromine, iodine atom or a triflate group, U₂ being in the position in which it is desired to introduce a group R₆ or R₇:
- either via a reaction of Suzuki type,
- or according to a reaction of Stille type;
- or via a reaction of Negishi type.

12. Compound of formula (I) according to any one of Claims 1 to 8, in the form of base or of a pharmaceutically acceptable acid-addition salt, for its use as a medicament.

13. Pharmaceutical composition containing at least one compound of formula (I) according to any one of Claims 1 to 7, in the form of base or of a pharmaceutically acceptable acid-addition salt and optionally one or more pharmaceutically acceptable excipients.

14. Use of a compound of formula (I) according to any one of Claims 1 to 8, in the form of base or of a pharmaceutically acceptable acid-addition salt, for the preparation of a medicament for preventing or treating acute or chronic pain, vertigo, vomiting, nausea, eating disorders, neurological and psychiatric pathologies, acute or chronic neurodegenerative diseases, epilepsy, sleeping disorders, cardiovascular diseases, renal ischaemia, cancers, immune system disorders, allergic diseases, parasitic, viral or bacterial infectious diseases, inflammatory diseases, osteoporosis, ocular complaints, pulmonary complaints, gastrointestinal diseases or urinary incontinence.
